(19) Europäisches Patentamt
European Patent Office
Office européen des brevets

(11) EP 0 471 609 B1

(12) **FASCICULE DE BREVET EUROPEEN**

(45) Date de publication et mention de la délivrance du brevet: **27.11.1996 Bulletin 1996/48**

(21) Numéro de dépôt: **91402201.7**

(22) Date de dépôt: **06.08.1991**

(51) Int Cl.[6]: **C07D 307/81**, C07D 209/14, C07D 333/58, C07D 471/04, A61K 31/34, A61K 31/38, A61K 31/40 // (C07D471/04, 221:00, 209:00)

(54) **Dérivés de Benzofuranne, Benzothiophène, Indole ou Indolizine, leur procédé de préparation ainsi que les compositions les contenant**

Benzofuranderivate, Benzothiophene, Indole oder Indolizine, Verfahren zu ihrer Herstellung und diese enthaltende Zusammensetzungen

Benzofuran Derivatives, Benzothiophenes, Indoles or Indolizines, Process for Production and Compositions containing them

(84) Etats contractants désignés:
**AT BE CH DE DK ES FR GB GR IT LI LU NL SE**

(30) Priorité: **06.08.1990 FR 9010036**

(43) Date de publication de la demande:
**19.02.1992 Bulletin 1992/08**

(73) Titulaires:
- **SANOFI**
  **75008 Paris (FR)**
  Etats contractants désignés:
  **CH DE DK ES FR GB GR IT LI LU NL SE AT**
- **S.A. SANOFI-PHARMA N.V.**
  **B-1120 Bruxelles (BE)**
  Etats contractants désignés:
  **BE**

(72) Inventeurs:
- **Gubin, Jean**
  **B-1020 Bruxelles (BE)**
- **Lucchetti, Jean**
  **B-5860 Chastre (BE)**
- **Inion, Henri**
  **B-1810 Wemmel (BE)**
- **Chatelain, Pierre**
  **B-1150 Bruxelles (BE)**
- **Rosseels, Gilbert**
  **B-1810 Wemmel (BE)**
- **Kilenyi, Steven**
  **B-1150 Bruxelles (BE)**

(74) Mandataire: **Moncheny, Michel et al**
**c/o Cabinet Lavoix**
**2 Place d'Estienne d'Orves**
**75441 Paris Cedex 09 (FR)**

(56) Documents cités:
**EP-A- 0 338 746**
**EP-A- 0 350 384**
**EP-A- 0 360 784**
**EP-A- 0 382 629**
**WO-A-89/02888**
**WO-A-89/02892**
**WO-A-89/02893**
**US-A- 4 024 273**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 471 609 B1

## Description

La présente invention se rapporte d'une manière générale à de nouveaux dérivés hétérocycliques ainsi qu'à leur procédé de préparation.

En particulier, l'invention concerne des dérivés de benzofuranne, benzothiophène, indole et indolizine, lesquels peuvent être représentés par la formule générale :

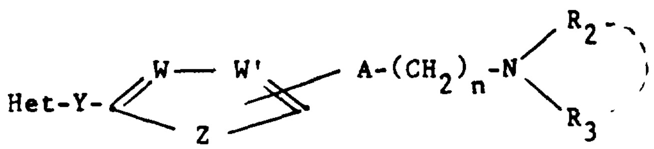

I

dans laquelle :

Het représente l'un des groupements :

α = 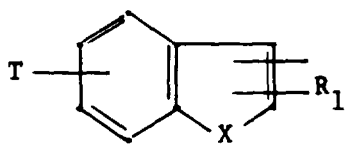 ou β = 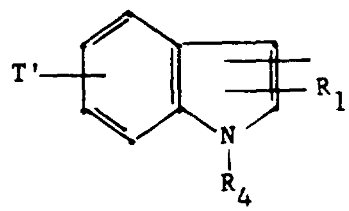

ou γ = 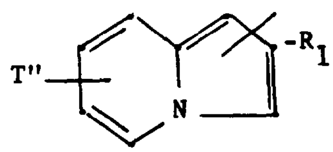

dans lesquels :

T représente un groupement :

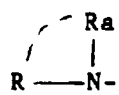

dans lequel :

R et Ra, identiques ou différents, représentent :

- l'hydrogène,
- un radical alkyle en $C_1$-$C_4$,
- un radical -$SO_2R'$ lequel lequel R' représente un radical alkyle, linéaire ou ramifié, en $C_1$-$C_6$, un radical trifluorométhyle, un radical phényle éventuellement substitué par un radical alkyle, en $C_1$-$C_4$, un radical benzyle éventuellement substitué par un radical alkyle en $C_1$-$C_4$ ou un radical benzoyle éventuellement substitué par par un radical alkyle en $C_1$-$C_4$, Ra et R' pouvant former avec l'atome d'azote du groupement sulfonamido, un cycle comportant de 3 à 6 atomes de carbone, notamment de 4 à 6 atomes de carbone,

T' représente :

- le groupement nitro,
- un groupement

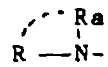

tel que défini précédemment,

T" représente :

- un groupement benzyloxycarbamoyle,
- un groupement :

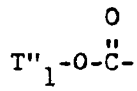

dans lequel $T"_1$ représente l'hydrogène ou un groupement alkyle en $C_1$-$C_4$
- un groupement :

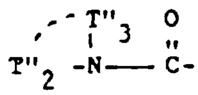

dans lequel $T"_2$ et $T"_3$, identiques ou différents, représentent l'hydrogène ou un groupement alkyle en $C_1$-$C_4$ ou $T"_2$ et $T"_3$ forment avec l'atome d'azote auquel ils sont attachés, un cycle ayant de 4 à 6 atomes de carbone,
- un groupement

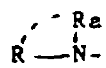

tel que défini précédemment,

X représente -O- ou -S-
Y représente un radical

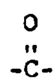 , 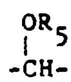

ou $-CH_2-$ dans lequel $R_5$ représente l'hydrogène, un radical alkyle en $C_1$-$C_4$ ou un radical acyle de formule

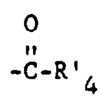

dans lequel $R'_4$ représente un radical alkyle en $C_1$-$C_4$
$R_1$ représente un radical alkyle en $C_1$-$C_6$, phényle éventuellement substitué par un radical alkyle en $C_1$-$C_4$ ou halogénophényle
$R_2$ représente :

- l'hydrogène,
- un radical alkyle linéaire ou ramifié en $C_1$-$C_6$

$R_3$ représente :

- un radical alkyle, linéaire ou ramifié en $C_1$-$C_6$
- un radical de formule :

$$-Alk-R_6$$

dans lequel Alk représente une liaison simple ou un radical alkylène, linéaire ou ramifié en $C_1$-$C_5$ et $R_6$ représente un radical pyridyle, phényle, phénoxy; méthylènedioxy-3,4 phényle ou un groupement phényle ou phénoxy substitué par un ou plusieurs substituants, identiques ou différents, sélectionnés parmi des halogènes, des groupements alkyles en $C_1$-$C_4$ ou alkoxy en $C_1$-$C_4$,

$R_2$ et $R_3$, lorsqu'ils sont pris ensemble, représentent un radical alkylène ou alkénylène en $C_3$-$C_6$ éventuellement substitué par un radical phényle ; éventuellement interrompu par -O-, -NH-, -N= ou $>N-R_7$, $R_7$ représentant un radical alkyle en $C_1$-$C_4$ ou phényle
$R_4$ représente :

- l'hydrogène,
- un radical alkyle en $C_1$-$C_4$
- un radical $-SO_2R'_1$ dans lequel $R'_1$ représente un radical alkyle en $C_1$-$C_4$, un radical phényle éventuellement substitué par un radical alkyle en $C_1$-$C_4$ ou un radical benzyle éventuellement substitué par un radical alkyle en $C_1$-$C_4$
- un radical

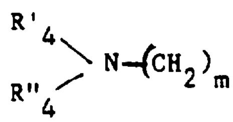

dans lequel $R'_4$ et $R''_4$, identiques ou différents, représentent un radical alkyle en $C_1$-$C_4$ et m représente un nombre de 1 à 3

A représente -O-, -S- ou

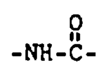

W, W′ et Z sont tels que :

- lorsque W et W′, identiques représentent

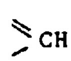

ou N, Z représente -O-
- lorsque W représente

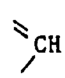

et W' représente

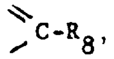

Z représente

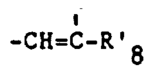

$R_8$ et $R'_8$ étant identiques ou différents et représentant l'hydrogène, un atome d'halogène par exemple fluor, chlore ou brome, un radical alkyle en $C_1$-$C_4$ tel que méthyle ou alkoxy en $C_1$-$C_4$ tel que méthoxy

n représente un nombre de 1 à 5, à condition que lorsque $R_4$ représente un radical $-SO_2R'_1$, T' représente un groupement nitro ou un groupement

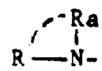

cyclisé.

Dans la formule I ci-dessus :

Het peut représenter notamment un radical T-4 benzofuryl-2 ou -3, T-5 benzofuryl-2 ou -3, T-7 benzofuryl-2 ou -3, T-4 benzothiényl-2 ou -3, T-5 benzothiényl-2 ou -3, T-7 benzothiényl-2 ou -3, T'-4 indolyl-2 ou -3, T'-5 indolyl-2 ou -3, T'-7 indolyl-2 ou -3, T"-7 indolizinyl-1 ou -3
R et $R_a$ peuvent représenter notamment un radical méthyle, éthyle, n-propyle, isopropyle, n-butyle, isobutyle ou tertiobutyle
$R_1$ peut représenter un radical méthyle, éthyle, n-propyle, isopropyle, n-butyle, isobutyle, tertiobutyle, méthyl-1 propyle, n-pentyle, néopentyle, n-hexyle, phényle, mono-méthylphényle, mono-fluoro-, mono-chloro- ou mono-bromo-phényle,
$R_2$ peut représenter notamment un radical méthyle, éthyle, n-propyle, isopropyle, n-butyle, isobutyle, tertiobutyle, n-pentyle, néopentyle ou n-hexyle
$R_3$ peut représenter notamment un radical méthyle, éthyle, n-isopropyle, isopropyle, n-butyle, isobutyle, tertiobutyle, méthyl-1 propyle, n-pentyle, n-hexyle, phényle, benzyle, phénéthyle, méthoxy-phényle, diméthoxyphénéthyle par exemple diméthoxy-3,4 phénéthyle ou diméthoxy-3,5 phénéthyle, triméthoxyphénéthyle, diméthylphénéthyle, diméthoxybenzyle, pyridyléthyle ou un radical phénéthyle substitué dans la partie aromatique par des radicaux méthyle et méthoxy,
$R_2$ et $R_3$ pris ensemble peuvent représenter notamment un radical tétraméthylène-1,4; pentaméthylène-1,5; oxo-3 pentaméthylène-1,5; aza-3 pentaméthylène-1,5; méthylaza-3 pentaméthylène-1,5; phénylaza-3 pentaméthylène-1,5 ou -CH-CH-N=CH de sorte que $R_2$ et $R_3$, pris avec l'atome d'azote auquel ils sont attachés, peuvent représenter notamment un radical pyrrolidinyle, pipéridinyle, morpholinyle, pipérazinyle, méthyl-4 pipérazinyle, phényl-4 pipérazinyle ou 1H-imidazolyle.
W′, W' et Z, pris avec les atomes de carbone auquel ils sont attachés, peuvent former notamment un radical phényle, furyle.

Une classe de composés préférés de l'invention peut être représentée par les composés de formule I dans laquelle R représente un groupement $-SO_2R'$ et $R_a$ représente l'hydrogène. De même, une classe particulière de composés de formule I est celle dans laquelle Y représente un radical

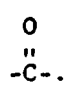

Une autre classe de composés préférés est celle dans laquelle l'entité

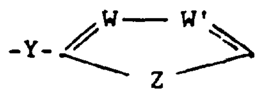

représente un radical benzoyle.

De même, une classe particulière de composés de formule I est celle dans laquelle

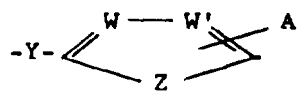

représente un radical benzoyl oxy-4.

De plus, les composés de formule I dans laquelle X représente -O- sont des composés préférés de même que ceux dans lesquels la chaine

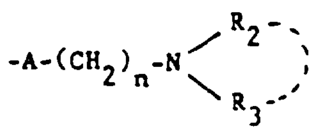

se trouve en position 4.

Enfin, les composés de formule I dans laquelle $R_1$, $R_2$ et $R_3$ représentent un radical n-butyle et n représente 3 peuvent être considérés également comme préférés.

L'invention se rapporte également aux sels pharmaceutiquement acceptables des composés de formule I formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophilline acétate ainsi que les sels formés à partir d'un acide aminé tel que le sel de lysine ou l'histidine.

Comme sels inorganiques de ce genre, on peut citer les chlorhydrate, bromhydrate, sulfate, sulfamate, phosphate et nitrate.

On a trouvé que les composés de l'invention possèdent de remarquables propiétés pharmacologiques puisqu'ils se sont révélés capables d'allonger de manière uniforme le potentiel d'action et la période réfractaire des cellules du myocarde. De plus, la plupart des composés de l'invention ont également révélé des propriétés bradycardisantes, antihypertensives et antiadrénergiques.

Ces propriétés sont capables de rendre les composés en question très utiles dans le traitement de certains syndromes pathologiques du système cardiovasculaire en particulier dans le traitement de l'angine de poitrine, de l'hypertension, de l'arythmie, de l'insuffisance circulatoire cérébrale.

Dans le domaine antitumoral, les composés de l'invention pourront être utiles comme potentialisateurs d'anticancéreux.

En conséquence, l'invention se rapporte également à des compositions pharmaceutiques ou vétérinaires contenant comme principe actif, au moins un composé de l'invention, en association avec un véhicule pharmaceutique ou excipient approprié.

Selon la voie d'administration choisie, la posologie journalière pour un être humain pesant 60 kg se situera entre 2 et 500 mg de principe actif.

Les composés de formule I peuvent être préparés selon les méthodes suivantes :

I- <u>Les composés de formule I dans laquelle Y représente un groupement</u>

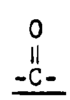

peuvent être obtenus :

A- Dans le cas où T ou T' représente un groupement amino, en hydrogénant un dérivé nitré de formule générale II

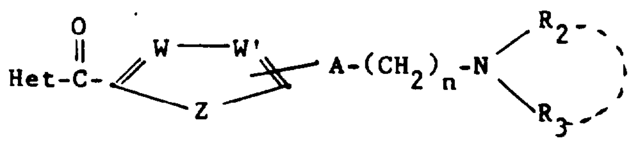

II

dans laquelle Het représente un groupement :

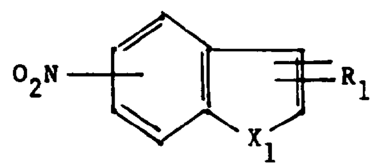

dans lequel $X_1$ représente -0-, -S- ou un radical , $\rangle N-R_4$ et $R_1$, $R_2$, $R_3$, A, W, W', X, Y, Z et n ont la même signification que précédemment, la réaction ayant lieu en présence d'un catalyseur approprié tel que l'oxyde de platine ou de palladium, le zinc en milieu acide chlorhydrique ou l'étain en milieu acide chlorhydrique et dans un solvant polaire tel qu'un alcool par exemple l'éthanol, ce qui fournit, sous forme de base libre, les composés désirés de formule I.

B- Dans le cas où T, T' ou T" représente un groupement

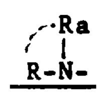

dans lequel :

a) R représente un groupement $-SO_2R'$ et $R_a$ représente l'hydrogène ou un groupement $-SO_2R'$ en faisant réagir un composé de formule I dans laquelle R et $R_a$ représentent chacun l'hydrogène avec un ou deux équivalents d'un halogénure de formule générale :

$$Hal-SO_2-R' \qquad III$$

ou d'un anhydride de formule générale :

$$(R'SO_2)_2O \qquad IIIa$$

dans laquelle R' a la même signification que précédemment et Hal représente un atome d'halogène, par exemple chlore ou brome, la réaction ayant lieu en présence d'un accepteur d'acide et dans un solvant organique approprié, ce qui fournit, sous forme de base libre, les composés désirés de formule I.
Comme accepteur d'acide, on utilise généralement un composé à caractère basique telle qu'une amine par exemple la triéthylamine tandis qui le solvant peut être un solvant aprotique tel que le dichloréthane ou le tétrachlorure de carbone

b) R a la valeur indiquée et $R_a$ représente un radical alkyle en faisant réagir, en milieu alcalin, un dérivé de formule I dans laquelle R a la valeur indiquée et $R_a$ représente l'hydrogène avec un ou deux équivalents

d'un halogénure de formule générale :

$$R'_a\text{-Hal} \qquad IV$$

dans laquelle Hal a la même signification que précédemment et $R'_a$ représente un radical alkyle en $C_1$-$C_4$, ce qui fournit, sous forme de base libre les composés désirés de formule I.

C. Dans le cas où T′ représente un groupement nitro ou T, T′ ou T″ représente un groupement

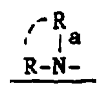

cyclisé, en faisant réagir, dans un solvant approprié généralement un solvant polaire tel que le N,N-diméthylformamide, l'acétonitrile ou la méthyl éthyl cétone ou un solvant apolaire tel que le benzène ou le toluène, un composé de formule générale :

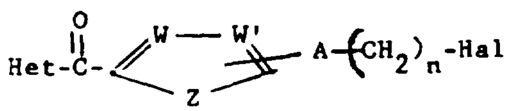 V

dans laquelle A, W, W′, Z, Hal et n ont la même valeur que précédemment et HET représente un groupement de formule générale :

α = 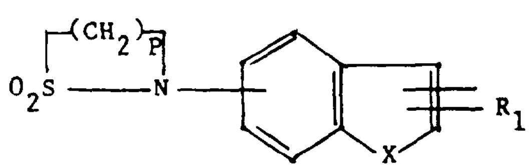 ou β = 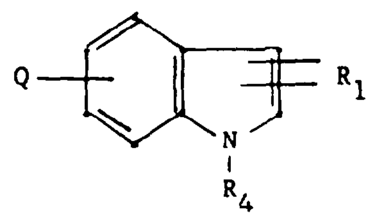

ou

γ = 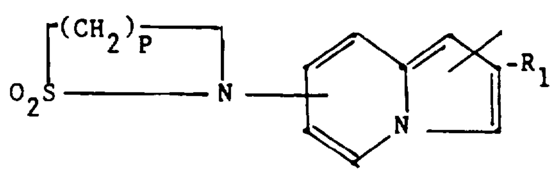

dans laquelle $R_1$, $R_4$ et X ont la même valeur que précedemment, Q représente un groupement nitro ou

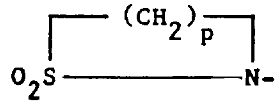

et p représente un nombre de 1 à 4, avec un composé azoté de formule générale :

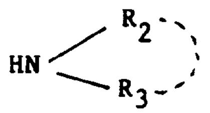

VI

dans laquelle $R_2$ et $R_3$ ont la même signification que précédemment, la réaction ayant lieu en présence d'un agent basique tel qu'un carbonate ou hydroxyde de métal alcalin ou un excès d'amine de formule VI ce qui fournit, sous forme de base libre, les composés désirés de formule I.

D- <u>Dans le cas où T" représente un groupement carbalkoxy</u>, en traitant, à une température de 80 à 110°C, un dérivé d'indolizine de formule générale :

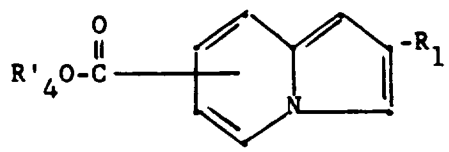

VII

dans laquelle $R_1$ et $R'_4$ ont la même signification que précédemment, avec un halogénure de formule générale :

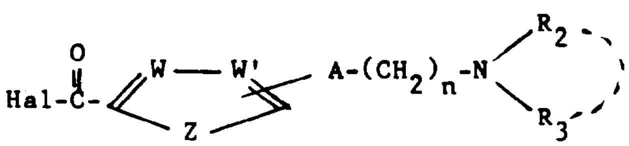

VIII

dans laquelle Hal, A, $R_2$, $R_3$, W, W', Z et n ont la même signification que précédemment, ce qui fournit, sous forme de base libre, les composés désirés de formule I.

E- <u>Dans le cas où T" représente un groupement carboxy,</u> en saponifiant un dérivé de carbalkoxy-indolizine de formule I dans laquelle Het représente un groupement de formule générale :

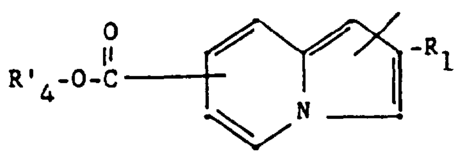

IX

dans laquelle $R_1$et $R'_4$ ont la même signification que précédemment, au moyen d'un hydroxyde de métal alcalin, la saponification ayant lieu de préférence à la température de reflux du milieu et dans un solvant approprié tel qu'un alcool par exemple l'éthanol, pour obtenir, sous forme de base libre, les composés désirés de formule I.

F- <u>Dans le cas où T" représente un groupement benzyloxycarbonylamino,</u> en faisant réagir, dans un solvant approprié tel que l'acétone et à une température de 0 à + 10°C, un dérivé de carboxy-indolizine de formule I dans laquelle et représente un groupement :

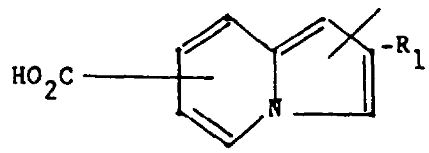 X

dans laquelle $R_1$ a la même signification que précédemment avec le chloroformiate d'éthyle en présence d'un accepteur d'acide tel qu'une amine par exemple la triéthylamine, ensuite avec un azide de métal alcalin et finalement avec l'alcool benzylique, par exemple à une température de 90 à 110°C, pour obtenir, sous forme de base libre, les composés désirés de formule I.

G- Dans le cas où T" représente un groupement amino, en hydrogénant dans un solvant approprié tel qu'un alcool par exemple l'éthanol et en présence d'un catalyseur approprié tel que le platine ou le palladium, un dérivé benzyloxycarbonylamino-indolizine de formule I dans laquelle Het représente un groupement de formule :

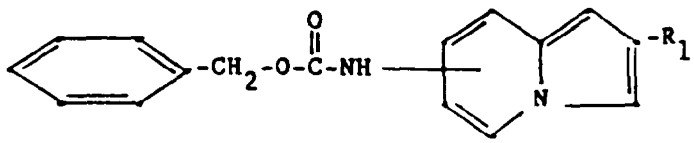 XI

dans laquelle $R_1$ a la même signification que précédemment, pour obtenir sous forme de base libre, les composés désirés de formule I.

H- Dans le cas où T" représente un groupement carboxamido substitué ou non, en chauffant à une température de 90 à 110°C et dans un solvant approprié tel qu'un alcool, un composé de formule I dans laquelle Het représente un groupement de formule X, avec un composé de formule générale :

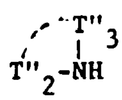 XII

dans laquelle $T"_2$ et $T"_3$ ont la même signification que précédemment, ce qui fournit, sous forme de base libre, les composés désirés de formule I.

II - Les composés de formule I dans laquelle Y représente un groupement

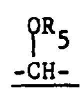

peuvent être obtenus :

a) Lorsque $R_5$ représente l'hydrogène, en réduisant, au moyen d'un borohydrure de métal alcalin tel que le borohydrure de sodium et dans un solvant approprié tel qu'un alcool ou un éther, un composé de formule I dans laquelle Y représente un groupement

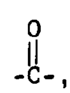

ce qui fournit sous forme de base libre, les composés désirés de formule I.

b) Lorsque $R_5$ représente un radical $R'_4$ ou un radical acyle de formule

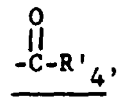

en faisant réagir l'alcool secondaire ainsi formé, c'est-à-dire un composé de formule I dans laquelle Y représente un groupement

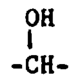

soit avec un alcoolate de métal alcalin, puis avec un halogénure de formule générale :

$$R'_4\text{-Hal} \qquad \text{XIII}$$

dans laquelle Hal et $R'_4$ ont la même signification que précédemment
soit avec un halogénure d'acyle de formule générale:

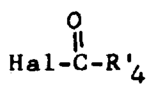

XIV

dans laquelle Hal et $R'_4$ ont la même signification que précédemment, la réaction ayant lieu en présence d'un accepteur d'acide telle que la pyridine, de manière à obtenir, sous forme de base libre, les composés désirés de formule I.

Selon la structure du composé de départ, des mélanges de composés peuvent être obtenus lors de la réduction. Ces produits peuvent être séparés de leur mélange selon des techniques classiques par exemple par chromatographie d'élution.

III - Les composés de formule I dans laquelle Y représente un groupement $-CH_2-$ peuvent être préparés, préférentiellement, en réduisant au moyen d'un borohydrure de métal alcalin tel que le borohydrure de sodium, en présence d'acide trifluoroacétique et dans un solvant approprié tel qu'un alcool, un éther ou un hydrocarbure halogéné, un composé de formule I dans laquelle Y représente un groupement

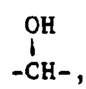

ce qui fournit, sous forme de base libre, les composés désirés de formule I.

Généralement, la réduction des composés de formule I dans laquelle Y représente un groupement

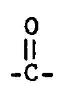

ou

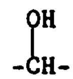

s'effectue à une température de l'ordre de -10° à + 10°C, de préférence à 0°C.

Des variantes aux procédés décrits précédemment peuvent être utilisés pour la préparation de certains composés de formule I essentiellement des composés de formule I dans laquelle Y représente un groupement

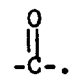

A titre d'exemple, on a décrit de tels procédés ci-après :

I- <u>Lorsque T' représente un groupement nitro et Y représente un groupement</u>

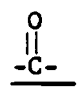

a) on condense, en présence d'un acide de Lewis tel que le chlorure d'aluminium ou le chlorure stannique, un halogénure d'acyle de formule générale :

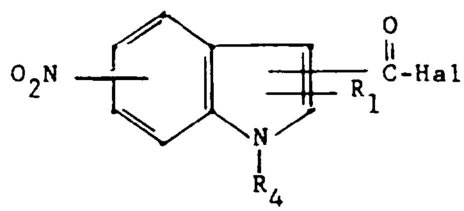 XV

dans laquelle $R_1$, $R_4$ et Hal ont la même signification que précédemment, avec une amine de formule générale :

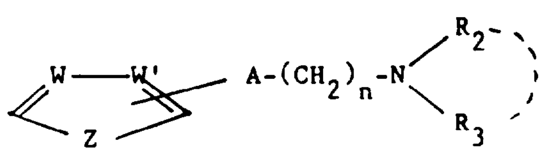 XVI

dans laquelle A, $R_2$, $R_3$, W, W′, Z et n ont la même signification que précédemment, ce qui fournit, sous forme de base libre, les composés désirés de formule I.

II- <u>Lorsque T′ représente un groupement nitro ou lorsque T, T′ ou T″</u> <u>représente un groupement</u>

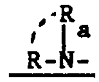

<u>cyclisé, Y représente un groupement</u>

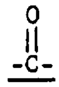

et A représente -O-, on condense à une température de l'ordre de 90 à 110°C et dans un solvant approprié, par exemple un solvant polaire tel que le N,N-diméthylformamide, une cétone de formule générale :

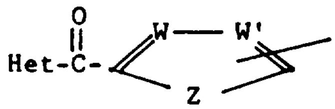

XVII

dans laquelle W, W′ et Z ont la même signification que précédemment et Het représente un groupement de formule générale :

$\alpha_1$ = 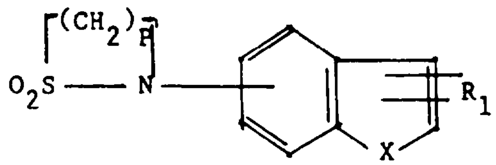 ou $\beta_1$ = 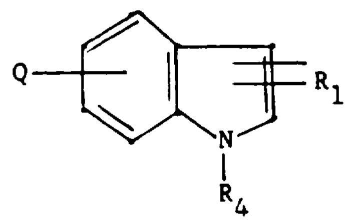

ou $\gamma_1$ = 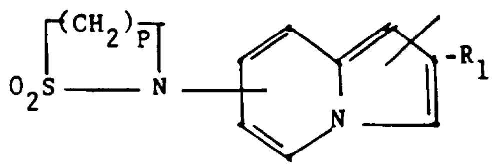

dans laquelle Q, $R_1$, $R_4$, X et p ont la même signification que précédemment, avec un composé de formule générale :

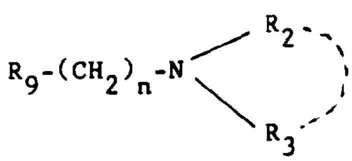

XVIII

dans laquelle $R_2$, $R_3$ et n ont la même signification que précédemment et $R_9$ représente un atome d'halogène, un radical alkylsulfonyloxy en $C_1$-$C_4$ ou arylsulfonyloxy en $C_6$-$C_{10}$, ce qui fournit, sous forme de base libre, les composés désirés de formule I.

III- Lorsque T' représente l'hydrogène, un groupement nitro ou un groupement

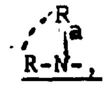

$R_4$ représente un groupement $-SO_2R'$ et Y représente un groupement

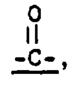

on traite dans un solvant approprié tel qu'un solvant polaire par exemple le N,N-diméthylformamide et en présence d'un agent basique tel qu'un hydrure de métal alcalin ou un alcoolate de métal alcalin, un dérivé d'indole de formule générale :

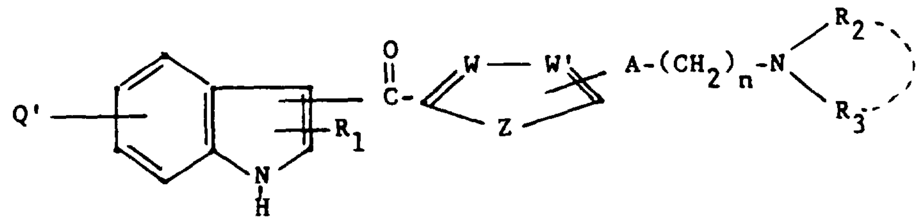

XIX

dans laquelle A, $R_1$, $R_2$, $R_3$, A, W', Z et n ont la même signification que précédemment et Q' représente l'hydrogène, un groupement nitro ou un groupement

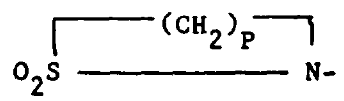

tel que défini précédemment, avec un halogénure de formule III ou IIIa, ce qui fournit, sous forme de base libre, les composés désirés de formule I.

IV- Lorsque T' représente un groupement nitro, Y représente un groupement

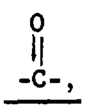

A représente -O-, $R_2$, $R_3$, $R_4$ et $R'_4$ sont identiques et m et n sont identiques, on fait réagir, en présence d'un agent basique tel qu'un carbonate ou hydroxyde de métal alcalin, un dérivé cétonique de formule générale :

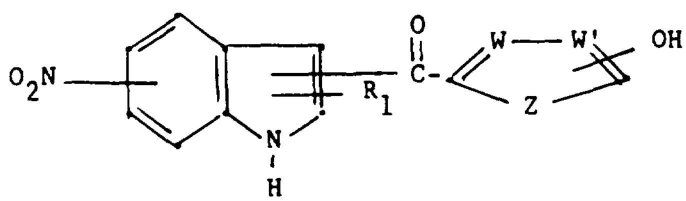

XX

dans laquelle, $R_1$, W, W′ et Z ont la même signification que précédemment avec un composé de formule XVIII, ce qui fournit, sous forme de base libre, les composés désirés de formule I.

V- Lorsque T, T′ ou T″ représente un groupement -NH-$SO_2R'$, on traite dans un solvant approprié tel qu'un alcool, par exemple l'éthanol, un composé de formule I dans laquelle T, T′ ou T″ représente un groupement -N($SO_2R'$)$_2$ avec un hydroxyde de métal alcalin, ce qui fournit, sous forme de base libre, les composés désirés de formule I.

VI- Lorsque T ou T′ représente un groupement

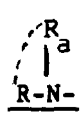

dans lequel R et Ra sont identiques et représentent chacun un groupement -$SO_2R'$, Y représente un groupement

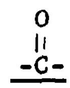

et A représente un groupement -O-, on fait réagir en présence d'un agent basique tel qu'un carbonate ou hydroxyde de métal alcalin et à la température de reflux du milieu, un composé de formule générale :

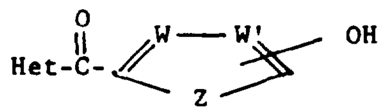

XXI

dans laquelle W, W′ et Z ont la même signification que précédemment et Het représente un groupement :

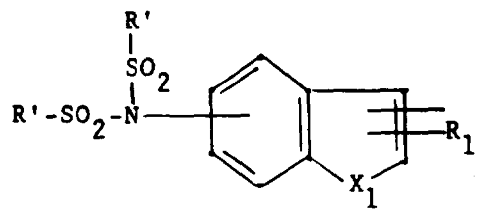

dans lequel R′, $R_1$ et $X_1$ ont la même signification que précédemment, avec un composé de formule XVIII, ce qui fournit, sous forme de base libre, les composés désirés de formule I.

Les composés de formule I obtenus sous forme de base libre selon l'une ou l'autre des méthodes décrites ci-dessus, peuvent ensuite être transformés en sels pharmaceutiquement acceptables par réaction avec un acide organique ou inorganique approprié par exemple l'acide oxalique, maléique, fumarique, méthanesulfonique, benzoique, ascorbique, pamoïque, succinique, hexamique, bisméthylènealicyclique, éthanedisulfonique, acétique, propionique, tartrique, salicylique, citrique, gluconique, lactique,malique, cinnamique, mandélique, citraconique, aspartique, palmitique, stéarique, itaconique, glycolique, p-aminobenzoïque, glutamique, benzènesulfonique, p-toluènesulfonique, théophylline acétique ou avec la lysine ou l'histidine.

Les composés de formule II ainsi que les composés de formule V et XVII dans lesquelles Het représente un groupement $\alpha_1$ ou $\beta_1$ peuvent être préparés au départ d'un composé de formule générale :

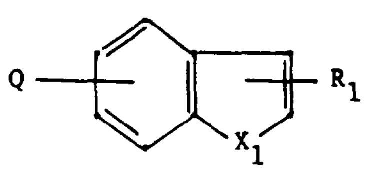

XXII

dans laquelle Q, $R_1$ et $X_1$ ont la même signification que précédemment, par réaction, éventuellement en présence d'un acide de Lewis, avec un halogénure de formule générale :

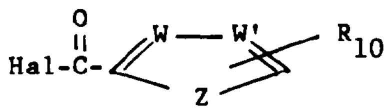

XXIII

dans laquelle W, W′, Z et Hal ont la même signification que précédemment et $R_{10}$ représente un radical $-OCH_3$, $-A-(CH_2)_n-Hal$ ou

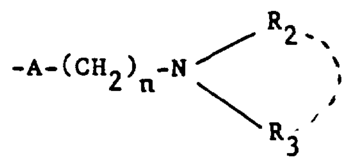

dans lequel A, $R_2$, $R_3$, Hal et n ont la même signification que précédemment, de manière à obtenir une cétone de formule générale :

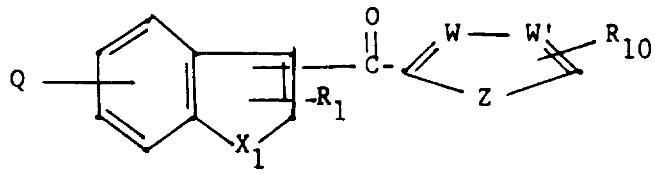

XXIV

dans laquelle Q, $R_1$, $R_{10}$, $X_1$, W, W′, et Z ont la même signification que précédemment.

Généralement, la réaction s'effectue en présence d'un acide de Lewis tel que le chlorure d'aluminium, le chlorure stannique ou le trifluorométhanesulfonate d'argent. Dans certains cas, cette réaction peut être accomplie sans l'aide de catalyseur notamment lors de la mise en oeuvre d'un composé de formule XXIII dans laquelle $R_{10}$ représente un radical

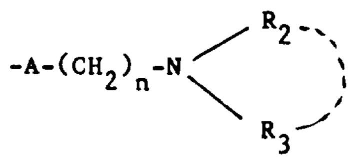

Les cétones de formule XXIV dans laquelle Q représente le groupement nitro et dans laquelle $R_{10}$ représente un groupement

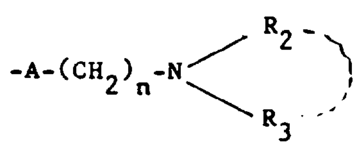

sont des composés de formule II tandis que celles dans laquelle $R_{10}$ représente un groupement $-A-(CH_2)_n-Hal$ sont en fait des composés de formule V.

Les cétones de formule XXIV dans laquelle $R_{10}$ représente un radical $-OCH_3$ ou $-A-(CH_2)_n-Hal$ peuvent être mises en oeuvre de la manière suivante:

a) lorsque $R_{10}$ représente un groupement $-OCH_3$, on provoque une O-déméthylation en présence d'un agent approprié tel que le chlorhydrate de pyridine, le tribromure de bore ou le chlorure d'aluminium pour obtenir un dérivé cétonique correspondant notamment à la formule XVII dans laquelle Het représente un groupement de formule $\alpha_1$ ou $\beta_1$ ou de formule XX, que l'on condense ensuite en présence d'un agent basique tel qu'un carbonate ou hydroxyde de métal alcalin :

- soit, avec une amine de formule XVIII, pour obtenir les composés de formule II dans laquelle A représente -O-
- soit, avec un dihalogénoalkane de formule générale :

$$Hal-(CH_2)_n-Hal \qquad XXV$$

dans laquelle Hal et n ont la même signification que précédemment pour obtenir un composé de formule V dans laquelle A représente -O-, puis avec une amine de formule VI, la réaction ayant lieu en présence d'un agent basique tel qu'un carbonate ou hydroxyde de métal alcalln ou un excès d'amine de formule VI, ce qui fournit les composés de formule II dans laquelle A représente -O-.

b) lorsque $R_{10}$ représente un groupement $-A-(CH_2)_n-Hal$, on condense une amine de formule VI, ce qui fournit les composés de formule II dans laquelle A représente -O-, -S- ou

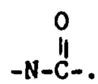

Selon une variante, on peut obtenir les composés de formule XVII ou XX par réaction directe entre un composé de formule XXII et un halogénure de formule XXIII dans laquelle $R_{10}$ représente un groupement $-OCH_3$ et ce, en présence d'un excès de chlorure d'aluminium.

D'une autre manière, les composés de formule XXIV dans laquelle $R_{10}$ représente un groupement $-OCH_3$ peuvent être obtenus au départ de composés de formule XXII par mise en oeuvre des différentes étapes suivantes :

a) acétylation au moyen d'anhydride acétique en présence de chlorure d'aluminium, puis bromation en présence d'un hydroxyde de métal alcalin de manière à former un dérivé d'acide carboxylique selon la méthode décrite dans Monatschefte für chemie 101, pp 1806-1816 (1970), dérivé d'acide carboxylique que l'on fait ensuite réagir avec le chlorure de thionyle, ce qui fournit les chlorures d'acyle de formule générale :

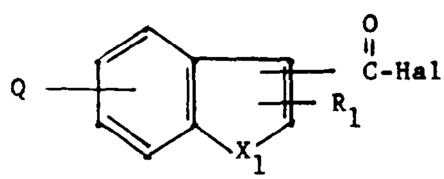

XXVI

dans laquelle Q, $R_1$, $X_1$ et Hal ont la même signification que précédemment,

b) condensation du chlorure d'acyle ainsi formé, en présence d'un acide de Lewis tel que le chlorure d'aluminium ou le chlorure stannique, avec une amine de formule générale :

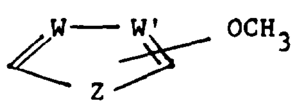

XXVII

dans laquelle W, W′ et Z ont la même signification que précédemment, ce qui fournit les composés désirés de formule XXIV.

Toutefois, lorsqu'on utilise un excès de chlorure d'aluminium dans l'étape b) précédente, on provoque également une O-déméthylation donnant naissance aux composés de formule XVII ou XX.

Les composés de formule XXVI ci-dessus englobent notamment des composés de formule XV précédemment citée.

Quant aux composés de formule XXII dans laquelle Q représente un groupement nitro, ceux-ci ont été décrits par exemple dans les brevets US N° 3.452.033 ou 4.024.273, dans J. Org. chem. 28 p 2262 (1963), chem. Abst. 99, 212380, 87, 152014 ou 83, 9985, dans Ann. chem. (Rome), 55, p. 1028 (1965) ou dans J.A.C.S. 75 - 1877 (1953).

Généralement, ces composés peuvent être obtenus à partir d'un bromobenzyle de formule générale :

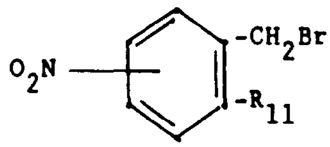

XXVIII

dans laquelle $R_{11}$ représente un radical -OH, -SH ou $-NH_2$, que l'on fait réagir avec la triphénylphosphine puis cyclise au moyen d'un chlorure d'acyle de formule générale :

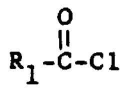

XXIX

dans laquelle $R_1$ a la même signification que précédemment.

Ce procédé est notamment appliqué dans le cas où $R_{11}$ représente un radical -OH.

D'une autre manière, on peut également obtenir les composés de formule XXII dans laquelle $X_1$ représente -S-, en faisant réagir un chloro-2 nitro-benzaldéhyde avec le sulfure de sodium puis avec une chlorocétone puis en réduisant le groupement cétone du composé cétonique ainsi formé, de manière à obtenir un alkyl-2 nitro-benzothiophène.

Les composés de formule XXII dans laquelle Q représente un groupement

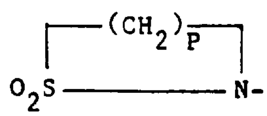

peuvent être préparés par hydrogénation catalytique par exemple en présence d'oxyde de platine, d'un composé de formule XXII dans laquelle Q représente le groupement nitro, puis réaction du dérivé amino ainsi formé, avec une alkane sultone, ce qui fournit un dérivé aminoalkylsulfonate que l'on cyclise avec du pentachlorure de phosphore de manière à obtenir les composés désirés.

Les composés de formule V dans laquelle Het représente un groupement $\gamma_1$ peuvent être préparés au départ d'un dérivé d'indolizine de formule générale :

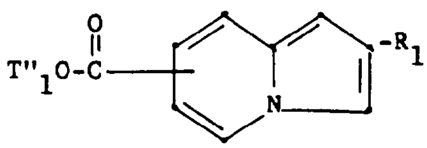

XXX

dans laquelle $R_1$ et T″ ont la même signification que précédemment, par mise en oeuvre des étapes suivantes :

a) saponification au moyen d'un hydroxyde de métal alcalin pour obtenir un dérivé de carboxy-indolizine

b) réaction du dérivé de carboxy-indolizine avec le chloroformiate d'éthyle en présence d'une amine, ensuite avec un azide de métal alcalin et finalement avec l'alcool benzylique pour obtenir un dérivé de benzyloxycarbamoyl-indolizine

c) hydrogénation de ce dérivé benzyle en présence d'un catalyseur tel que le platine, ce qui fournit un dérivé d'amino-indolizine

d) réaction du dérivé amino avec une alkane sultone et cyclisation avec du pentachlorure de phosphore

e) condensation en présence d'un acide de Lewis tel que le chlorure d'aluminium, avec un halogénure de formule XXIII dans laquelle :

1) $R_{10}$ représente un radical $-A-(CH_2)_n-Hal$, pour obtenir les composés requis de formule V

2) $R_{10}$ représente un radical $OCH_3$, pour obtenir un composé que l'on O-déméthyle au moyen de chlorhydrate de pyridine, de tribromure de bore ou de chloruer d'aluminium, pour obtenir les composés requis de formule

XVII.

Quant aux composés de formule XXX et VII, ceux-ci peuvent être obtenus par réaction d'un ester de méthyl-2 pyridine avec une bromoalkanone pour obtenir un dérivé de pyridinium que l'on cyclise en présence de triéthylamine.

Les amines de formules XVI et XXVII sont des produits connus pour la plupart puisque décrits dans le brevet US No. 4.831.054. Généralement, ces composés de formules XVI et XXVII peuvent être préparés en faisant réagir en présence d'un agent basique tel qu'un carbonate ou un hydroxyde de métal alcalin, un composé de formule générale :

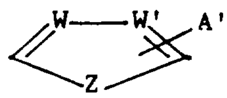

XXXI

dans laquelle W, W′ et Z ont la même signification que précédemment et A′ représente -OH, -SH ou $-NH_2$, avec un composé de formule générale :

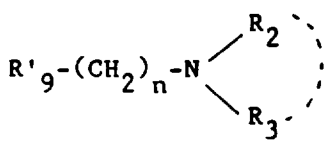

XXXII

dans !aquelle $R_2$, $R_3$ et n ont la même signification que précédemment et $R'_9$ est tel que :

1) lorsque A′ représente -OH ou -SH, $R'_9$ représente un atome d'halogène ou un radical alkylsulfonyloxy en $C_1$-$C_4$ ou arylsulfonyloxy en $C_6$-$C_{10}$
2) lorsque A′ représente $-NH_2$, $R'_9$ représente un radical

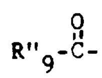

dans lequel $R''_9$ représente un atome d'halogène.

Les composés de formule XIX sont soit des composés de formule I ci-dessus, soit des composés ayant été décrits dans Eur. J. Med. Chem. - Chimica Therapeutica, 12, No. 5 pp. 483-487 (1977).

Ceux-ci peuvent être préparés par des méthodes connues notamment par application de méthodes analogues à celles décrites précédemment.

Les composés de formule XXI peuvent être obtenus à partir d'un composé amino de formule générale :

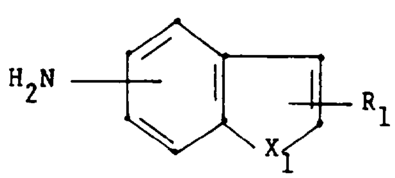

XXXIII

dans laquelle $R_1$et $X_1$ ont la même signification que précédemment, par mise en oeuvre du procédé suivant :

a) réaction avec deux équivalents d'un composé de formule III ou IIIa en présence d'un accepteur d'acide telle qu'une amine par exemple la triéthylamine,
b) condensation du dérivé bisulfonamido obtenu, avec un composé de formule XXIV dans laquelle $R_{10}$ représente un groupement $-OCH_3$ et ce, en présence d'un acide de Lewis tel que le tétrachlorure d'étain,
c) déméthylation au moyen d'un agent approprié tel que le tribromure de bore, pour obtenir les composés désirés de formule XXI.

Les composés de formule XXXIII peuvent être préparés par hydrogénation, en présence d'oxyde de platine ou de palladium, d'un dérivé de formule XXII dans laquelle Q représente un groupement nitro.

Quant aux composés de formules VI, VIII, XII, XXIII, XXV, XXVIII, XXXI ou XXXII il s'agit de composés connus. La plupart d'entre eux ont été publiés notamment dans les brevets US No. 4.024.273 ou No. 4.831.054, les demandes de brevet EP 0235.111 ou WO 90/02743 ou dans Eur. J. Med. Chem. - Chimica therapeutica 12, No. 5 pp. 483-487 (1977). Ces composés peuvent être tous préparés par des méthodes connues. A l'heure actuelle, il existe un intérêt incontestable à utiliser, comme moyens de prévention des arythmies cardiaques, les potentialités offertes par la conduction différée de l'influx électrique au niveau de la cellule cardiaque ou la prolongation de la période réfractaire.

Bien que de nombreux états physio-pathologiques prolongent la repolarisation de la cellule cardiaque et soient associés à une incidence réduite de fibrillation cardiaque, le concept du contrôle pharmacologique de troubles du rythme par l'augmentation du potentiel d'action est relativement nouveau.

Le potentiel d'action de la cellule myocardique représente en fait une modification du potentiel de repos de cette cellule qui, après avoir atteint de manière suffisamment rapide le potentiel seuil (-70 millivolts), initie une séquence d'altérations du potentiel de membrane.

Après passage de l'influx, le myocarde demeure transitoirement insensible à une nouvelle stimulation; pendant la période réfractaire absolue le myocarde est totalement inexcitable alors que pendant la période réfractaire relative, un stimulus suffisamment puissant peut entraîner une réponse lentement propagée. L'existence de ces périodes réfractaires conditionne l'unidirectionalité de la propagation de l'influx.

Les caractéristiques du potentiel d'action déterminent celles de la conduction et des périodes réfractaires. Aussi, tout raccourcissement de la repolarisation est arythmogène par l'abréviation concommittante de la période réfractaire. Inversément, toute intervention allongeant uniformément le potentiel d'action produit un allongement de la période réfractaire absolue, ce qui diminue l'arythmogénicité.

En d'autres termes, si on diffère l'atteinte d'un niveau seuil du potentiel membranaire nécessaire pour générer un second potentiel d'action, en réponse à un stimulus, en interférant dans des processus qui normalement gouvernent la vitesse de repolarisation, les périodes réfractaires (période absolue et période efficace) du muscle cardiaque devraient être prolongées de manière correspondante ce qui devrait engendrer un mécanisme antiarythmique.

L'amiodarone ou n-butyl-2 [(diéthylamino-2 éthoxy)-4 diiodo-3,5 benzoyl]-3 benzofuranne est, à l'heure actuelle, l'un des rares agents anti-arythmiques commercialisés qui possède les propriétés explicitées précédemment.

Ce composé prolonge, en effet, le plateau de repolarisation sans modifier la vitesse de dépolarisation rapide. Son effet antiarythmique découle de l'allongement uniforme des potentiels d'action et des périodes réfractaires des cellules myocardiques.

En outre, l'amiodarone présente des propriétés antiadrénergiques de type $\alpha$ et $\beta$ incomplètes. Ce composé peut donc être considéré, non comme un $\beta$-bloquant mais comme un adrénofreinateur, c'est-à-dire comme un antagoniste partiel des réactions adrénergiques $\alpha$ et $\beta$. Ces propriétés sont d'un bénéfice incontestable puisqu'il parait souhaitable de ne pas rechercher des propriétés antagonistes $\alpha$ ou $\beta$ complètes étant donné les effets secondaires qu'elles peuvent entraîner en clinique ("Bruxelles Médical", N° 9, septembre 1969, pages 543-560).

On connaît déjà des dérivés de benzofuranne, benzothiophène, indole ou indolizine analogues à la structure chimique de l'amiodarone c'est-à-dire comportant en position 3 une chaîne dialkylamino- ou monoalkylamino-alkoxybenzoyle : ces composés ont révélé, à des degrés divers, des propriétés actives sur le système cardiovasculaire.

A cet effet, on peut citer, par exemple, les brevets US Nos. 3.920.707 et 3.947.470, les demandes de brevet EP Nos. 338.746 et 360.784, les demandes de brevet PCT Nos. WO 89/02888, 89/02892 et 89/02893 ainsi que Eur. J. Med. Chem. - Chimica Therapeutica 12, N° 5 p. 483-487 (1977).

Or, il n'existe, apparemment, à l'heure actuelle, aucun dérivé de benzofuranne, benzothiophène ou indole connu comportant la chaîne dialkylamino- ou monoalkylamino-alkoxybenzoyle et substitué sur l'homocycle par un groupement amino lui-même substitué ou non.

Si le brevet US N° 3.947.470 couvre bien des dérivés azotés sur l'homocycle c'est-à-dire des dérivés comportant le groupement nitro, aucun produit de ce type ne peut être considéré comme ayant été réellement préparé et encore moins testé d'un point de vue pharmacologique.

Or, on a maintenant découvert de manière surprenante, dans le cadre de l'invention, que des dérivés de benzofuranne, benzothiophène, indole ou indolizine comportant une chaîne monoalkylamino- ou dialkylamino-alkoxy-benzoyle ainsi que d'autres groupements fixés sur l'hétérocycle et notamment sur l'homocycle benzofuranne, benzothiophène ou indole ou sur l'entité pypyridinique de l'indolizine, présentent de remarquables propriétés pharmacologiques se traduisant notamment par une augmentation de la durée du potentiel d'action et des périodes réfractaires de la cellule cardiaque.

Ces propriétés se sont même révélées supérieures à celles enregistrées avec des dérivés connus ou avec des dérivés analogues comportant un substituant nitro sur l'homocycle.

En outre, on a pu mettre en évidence que des composés de l'invention présentent moins de possibilités d'effets secondaires que des composés connus analogues.

On sait notamment, que l'amiodarone provoque des phospholipidoses dans le poumon, avec pour conséquence la destruction de macrophages dans les alvéoles. Cette destruction se traduira, chez le patient soumis à un traitement à l'amiodarone, par l'apparition de complications pulmonaires, telle qu'insuffisance respiratoire, qui nécessitera l'arrêt du traitement.

D'autres composés connus tel que le n-butyl-2[(di-n-butylamino-3 propoxy-4 benzoyl]-3 benzofuranne, présentent également cet effet secondaire. Par exemple, on a pu mettre en évidence que ce composé à la dose de 139 mg/kg par voie orale pendant 14 jours provoque chez le rat une augmentation de 11,6% des phospholipides pulmonaires. Dans les mêmes conditions, à la dose de 100 mg/kg, l'amiodarone provoque une augmentation de 26,7% du taux de phospholipides également dans le poumon.

Par contre, les composés suivants de l'invention :

- n-butyl-2[(di-n-butylamino-3 propoxy)-4 benzoyl]-3 méthylsulfonamido-5 benzofuranne (Ex. 2)
- amino-5[(di-n-butylamino-3 propoxy)-4 benzoyl]-3 n-butyl-2 benzofuranne (Ex. 3)

ne modifient pas les taux de phospholipides pulmonaires respectivement aux doses de 116 mg/kg et 135 mg/kg.

Compte tenu de cette absence de lipidose pulmonaire et suite à d'autres tests pharmacologiques réalisés, le composé de l'invention qui a montré les meilleures potentialités comme agent antiadrénergique et antiarythmique est le n-butyl-2[(di-n-butylamino-3 propoxy)-4 benzoyl]-3 méthylsulfonamido-5 benzofuranne et ses sels pharmaceutiquement acceptables.

Les résultats de tests pharmacologiques effectués en vue de déterminer les propriétés des composés de l'invention sur le système cardiovasculaire sont répertoriés ci-dessous.

I - **Effet sur la durée du potentiel d'action**

L'effet des composés de l'invention sur la durée du potentiel d'action a été mis en évidence par mesure de l'effet d'une administration intraveineuse chez le rat "in vivo".

La technique suivante est utilisée.

On anesthésie des rats mâles Sprague-Dowley de 250 à 450g et on les fixe en position dorsale sur un portoir. Après avoir placé des électrodes bipolaires aux 4 membres pour l'enregistrement de l'électrocardiogramme on place le rat sous respiration artificielle. Après thoracotomie et rupture du péricarde , on applique une électrode à succion sur le coeur (en évitant les artères coronaires). celle-ci adhère instantanément par la force d'aspiration d'une trompe à vide à laquelle elle est reliée.

On enregistre alors simultanément le potentiel d'action ainsi que l'électrocardiogramme à la vitesse de 200 mm/sec avant l'administration d'un composé à tester ainsi qu'à différents temps (1, 3, 5 et 10 minutes) après l'administration en 30 secondes d'une dose d'un composé à tester.

Les résultats obtenus sont exprimés en % d'augmentation de la durée du potentiel d'action (D.P.A.) par rapport à la durée enregistrée avant l'administration du composé à étudier, cette durée étant mesurée à 90% de l'amplitude du potentiel d'action.

A titre d'exemple, les résultata suivants ont été obtenus, les composés de formule I étant sous forme de base ou de sel.

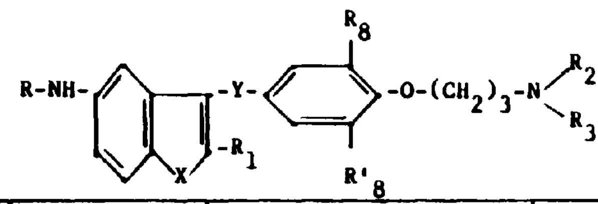

| Y | X | R | $R_1$ | $R_2$ | $R_3$ | $R_8$ et $R'_8$ | Dose(mg/kg) | ↑ D.P.A. |
|---|---|---|---|---|---|---|---|---|
| $\overset{O}{\parallel}{C}$ | O | $CH_3SO_2$- | n-$C_4H_9$ | n-$C_4H_9$ | n-$C_4H_9$ | H | 10 | 60 |
| $\overset{O}{\parallel}{C}$ | O | $CH_3SO_2$- | n-$C_4H_9$ | H | -$C(CH_3)_3$ | H | 10 | 32 |
| $\overset{O}{\parallel}{C}$ | O | H | n-$C_4$-$H_9$ | $CH_3$ | 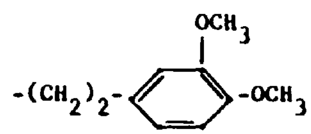 | H | 10 | 51 |
| $\overset{O}{\parallel}{C}$ | O | $CH_3SO_2$- | n-$C_4H_9$ | $CH_3$ | 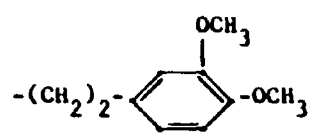 | H | 10 | 70 |
| $\overset{O}{\parallel}{C}$ | O | H | n-$C_4H_9$ | n-$C_4H_9$ | n-$C_4H_9$ | $CH_3$ | 5 | 57 |
| $\overset{O}{\parallel}{C}$ | O | $CF_3SO_2$- | n-$C_4H_9$ | n-$C_4H_9$ | n-$C_4H_9$ | H | 0,1 | 63 |

| | | | | | | | | | |
|---|---|---|---|---|---|---|---|---|---|
| $\overset{O}{\overset{\|}{C}}$ | O | $CH_3SO_2-$ | 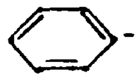 - | $n-C_4H_9$ | $n-C_4H_9$ | H | 10 | 65 | |
| $\overset{O}{\overset{\|}{C}}$ | O | $CH_3SO_2-$ | iso-$C_3H_7$ | $n-C_4H_9$ | $n-C_4H_9$ | H | 10 | 60 | |
| $\overset{O}{\overset{\|}{C}}$ | O | $CH_3-$ 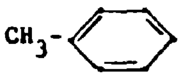 $-SO_2-$ | $n-C_4H_9$ | $n-C_4H_9$ | $n-C_4H_9$ | H | 10 | 37 | |
| $-\overset{OH}{\overset{\|}{CH}}-$ | O | $CH_3SO_2-$ | $n-C_4H_9$ | $n-C_4H_9$ | $n-C_4H_9$ | H | 10 | 24 | |
| $\overset{O}{\overset{\|}{C}}$ | NH | $CH_3SO_2-$ | $n-C_4H_9$ | $n-C_4H_9$ | $n-C_4H_9$ | H | 10 | 42 | |
| $\overset{O}{\overset{\|}{C}}$ | $NCH_3$ | H | $n-C_4H_9$ | $n-C_4H_9$ | $n-C_4H_9$ | H | 10 | 83 | |
| $\overset{O}{\overset{\|}{C}}$ | NH | H | $n-C_4H_9$ | $n-C_4H_9$ | $n-C_4H_9$ | H | 10 | 42 | |
| $-CH_2-$ | O | $CH_3SO_2$ | $n-C_4H_9$ | $n-C_4H_9$ | $n-C_4H_9$ | H | 10 | 38 | |

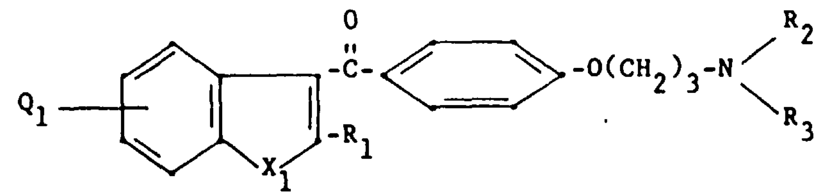

| $X_1$ | $Q_1$ | $R_1$ | $R_2$ | $R_3$ | Dose (mg/kg) | ↑ D.P.A. |
|---|---|---|---|---|---|---|
| O | $(CH_3SO_2)_2N$-7 | $CH_3$ | n-$C_4H_9$ | n-$C_4H_9$ | 5 | 80 |
| O | $NH_2$-7 | $CH_3$ | n-$C_4H_9$ | n-$C_4H_9$ | 10 | 73 |
| O | $CH_3SO_2N$-7 | $CH_3$ | n-$C_4H_9$ | n-$C_4H_9$ | 10 | 140 |
| O | 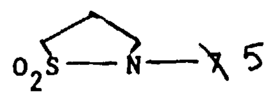 | n-$C_4H_9$ | n-$C_4H_9$ | n-$C_4H_9$ | 10 | 70 |
| $>N-(CH_2)_3-N(C_4H_9)_2$ | $NO_2$-5 | n-$C_4H_9$ | n-$C_4H_9$ | n-$C_4H_9$ | 10 | 42 |
| $>N-SO_2CH_3$ | H | n-$C_4H_9$ | n-$C_4H_9$ | n-$C_4H_9$ | 10 | 77 |

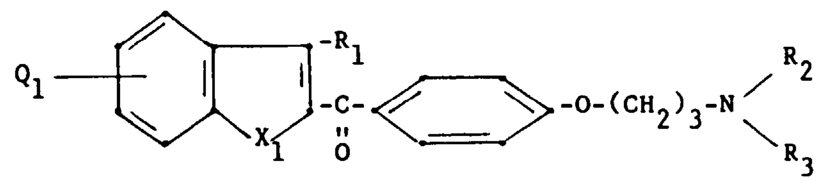

| $X_1$ | $Q_1$ | $R_1$ | $R_2$ | $R_3$ | Dose (mg/kg) | ↑ D.P.A. |
|---|---|---|---|---|---|---|
| O | $NH_2$-5 | $CH_3$ | n-$C_4H_9$ | n-$C_4H_9$ | 10 | 77 |
| S | $NH_2$-5 | n-$C_4H_9$ | $CH_3$ | 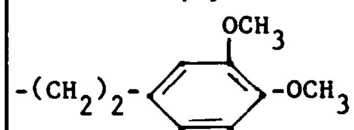 | 10 | 35 |
| S | $CH_3SO_2$-5 | n-$C_4H_9$ | $CH_3$ | 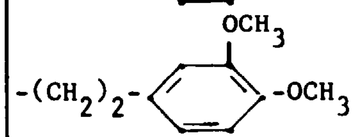 | 10 | 33 |
| NH | $NO_2$-5 | n-$C_4H_9$ | n-$C_4H_9$ | n-$C_4H_9$ | 10 | 64 |

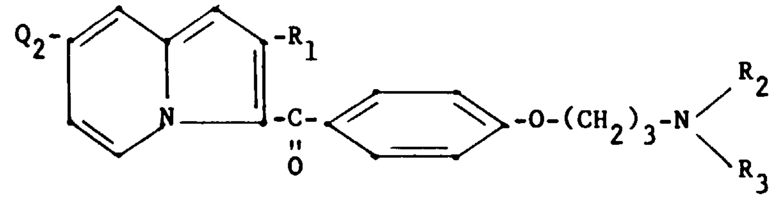

| $Q_2$ | $R_1$ | $R_2$ | $R_3$ | Dose (mg/kg) | ↗ D.P.A. |
|---|---|---|---|---|---|
| $H_5C_2O-C(=O)-$ | 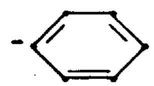 | $n-C_4H_9$ | $n-C_4H_9$ | 10 | 52 |
| $HOC(=O)-$ | $n-C_4H_9$ | $n-C_4H_9$ | $n-C_4H_9$ | 1 | 13 |
| 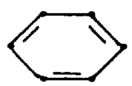 $-CH_2-O-C(=O)-NH-$ | $n-C_4H_9$ | $n-C_4H_9$ | $n-C_4H_9$ | 10 | 22 |
| $NH_2$ | $n-C_4H_9$ | $n-C_4H_9$ | $n-C_4H_9$ | 10 | 53 |
| $CH_3SO_2-$ | $n-C_4H_9$ | $n-C_4H_9$ | $n-C_4H_9$ | 10 | 66 |
| $CH_3-NH-C(=O)-$ | $n-C_4H_9$ | $n-C_4H_9$ | $n-C_4H_9$ | 10 | 23 |
| 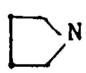 $N-C(=O)-$ | $n-C_4H_9$ | $n-C_4H_9$ | $n-C_4H_9$ | 10 | 52 |

A titre de comparaison, on a obtenu les résultats suivants avec un composé connu :

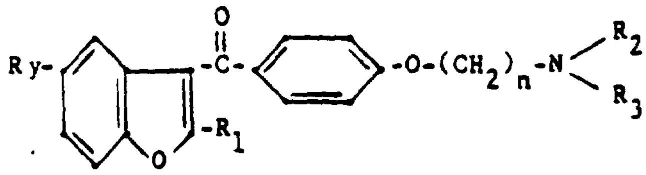

| $R_y$ | $R_1$ | $R_2$ | $R_3$ | n | Dose(mg/kg) | ↑ D.P.A.(%) |
|---|---|---|---|---|---|---|
| H | $n-C_4H_9$ | $n-C_4H_9$ | $n-C_4H_9$ | 3 | 10 | 31 |

Ces résultats montrent la nette supériorité des composés de l'invention sur le composé connu et ce, en tant qu'agents capables d'augmenter la durée du potentiel d'action de la cellule myocardique.

**II - Propriétés antiadrénergiques**

Le but de ce test est de déterminer la capacité des composés l'invention de réduire l'augmentation de la pression sanguine induite par l'épinéphrine (effet anti-α) et l'accélération de la fréquence cardiaque induite par l'isoprénaline (effet anti-β) chez le chien préalablement anesthésié au pentobarbital et atropiné.

On détermine d'abord pour chaque chien la dose d'épinéphrine (entre 3 et 10μg/kg) qui provoque une augmentation reproductible de la pression artérielle d'environ $133,10^2$Pa et la dose d'isoprénaline (1 à 2 μg/kg) qui provoque une augmentation reproductible de la fréquence cardiaque d'environ 70 battements/minute. On injecte alternativement toutes les dix minutes la dose d'épinéphrine et d'isoprénaline ainsi déterminée et après obtention de deux réponses de références successives, on administre une quantité du composé à étudier par voie intraveineuse.

- Effet anti-α

On enregistre le pourcentage de réduction de l'hypertension provoquée par le composé à étudier comparativement à l'hypertension de référence obtenue précédemment (environ 100 mm Hg).

- Effet anti-β

On enregistre le pourcentage de réduction de l'accélération de la fréquence cardiaque provoquée par le composé à étudier comparativement à la tachycardie de référence mesurée précédemment (environ 70 battements).

Dans les deux cas, on a exprimé les résultats de la réduction de la pression artérielle ou de la fréquence cardiaque comme suit :

+ pour une réduction < 50%

++ pour une réduction ≧ 50%

+++ pour une réduction sub-totale (réduction presque complète)

On a enregistré les résultats suivants :

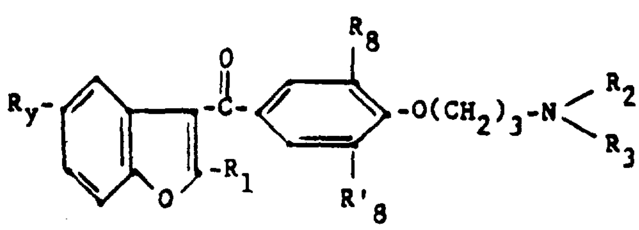

| $R_y$ | $R_1$ | $R_2$ | $R_3$ | $R_8$ et $R'_8$ | Dose (mg/kg) | Effet | |
|---|---|---|---|---|---|---|---|
| | | | | | | anti-α | anti-β |
| $CH_3SO_2NH-$ | n-$C_4H_9$ | n-$C_4H_9$ | n-$C_4H_9$ | H | 10 | +++ | +++ |
| $CH_3SO_2NH-$ | n-$C_4H_9$ | n-$C_4H_9$ | n-$C_4H_9$ | $CH_3$ | { 6,5<br>{ 5 | +++ | +++ |
| $NH_2$ | n-$C_4H_9$ | n-$C_4H_9$ | n-$C_4H_9$ | $CH_3$ | 5,7 | +++ | +++ |
| $(CH_3SO_2)_2N-$ | n-$C_4H_9$ | n-$C_4H_9$ | n-$C_4H_9$ | H | 5 | +++ | ++ |

A titre de comparaison, on a consigné ci-dessous des résultats obtenus avec composés connus :

| | | | | | | | |
|---|---|---|---|---|---|---|---|
| H | n-$C_4H_9$ | H | n-$C_4H_9$ | H | 10 | (+) | + |
| H | n-$C_4H_9$ | n-$C_4H_9$ | n-$C_4H_9$ | $CH_3$ | 10 | ++ | ++ |

**III - <u>Toxicité</u>**

La toxicité des composés de l'invention s'est révélée compatible avec leur utilisation en thérapeutique.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 50 à 500 mg en poids d'ingrédient actif pour l'administration orale, de 50 à 200 mg d'ingrédient actif pour l'administration rectale et de 50 à 150 mg d'ingrédient actif pour l'administration parentérale.

Suivant la voie d'administration choisie, les compositions thérapeutiques ou vétérinaires de l'invention seront préparées en associant au moine un des composés de formule I ou un sel d'addition non toxique de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.

Les Exemples, non limitatifs suivants, illustrent la préparation des composés et compositions de l'invention :

**<u>EXEMPLE 1</u>**

<u>Préparation de l'amino-5 [(di-n-butylamino-3 propoxy)-4 benzoyl]-3 n-butyl-2 benzofuranne</u> **(SR 33580)**

a) <u>Bromure d'hydroxy-2 nitro-5 benzyltriphénylphosphonium</u>

On chauffe à reflux pendant 0,5 heure, 100g (0,43 mole) d'hydroxy-2 nitro-5 bromobenzyle et 113g (0,43 mole) de triphénylphosphine dans 1600ml de chloroforme. On laisse refroidir et on essore le précipité blanc formé. On évapore le filtrat à sec, sous vide, et on reprend au moyen de 500ml de toluène. On essore, on lave avec du toluène, on réunit les solides formés et on sèche sous vide à 50°C.

De cette manière, on obtient 210,5g de bromure d'hydroxy-2 nitro-5 benzyltriphénylphosphonium.
Rendement : 99,01%

b) <u>n-Butyl-2 nitro-5 benzofuranne</u>

On ajoute lentement, sous agitation, 113,5g (0,94 mole) de chlorure de pentanoyle à un mélange de 370g (0,75 mole) de bromure d'hydroxy-2 nitro-5 benzyltriphénylphosphonium et 120,2g (1,52 mole) de pyridine dans 700ml de chloroforme. On chauffe à reflux durant 2 heures. On ajoute 2800ml de toluène et on distille 1400ml de solvants. On ajoute alors 228g (2,28 moles) de triéthylamine et on chauffe durant 3 heures à reflux. On laisse refroidir, on essore

la triphénylphosphine-oxyde formée, on lave avec de l'acétate d'éthyle et on concentre le filtrat sous vide. On dissout, dans de l'acétonitrile, le résidu visqueux obtenu et on extrait, dans un appareil à extraction liquide-liquide, avec du pentane. On sèche sur sulfate de sodium, on filtre et on évapore à sec.

De cette manière, on obtient le n-butyl-2 nitro-5 benzofuranne sous forme brute.
Pureté [chromatographie liquide haute pression (CLHP)] : 97,9%
P.E. 120-123°C (0,02 mm Hg ou 2,66 Pa)

De la même manière que décrit précédemment, on a préparé les composés suivants :
Méthyl-2 nitro-5 benzofuranne
P.F. : 96°C (éther isopropylique)
Pureté (CLHP) : 100%
Ethyl-2 nitro-5 benzofuranne
P.F. : 86°C (isopropanol)
Pureté (CLHP) : 99,8%
n-Propyl-2 nitro-5 benzofuranne
P.F. : 38°C (isopropanol)
Pureté (CLHP) : 99%
Isopropyl-2 nitro-5 benzofuranne
P.F. : 73°C (éther isopropylique)
Pureté (CLHP) : 99,45%
Phényl-2 nitro-5 benzofuranne
P.F. : 159°C (éther isopropylique)
Pureté (CLHP) : 99,5%

c) n-Butyl-2 (méthoxy-4 benzoyl)-3 nitro-5 benzofuranne

A une solution de 44,5g (0,2 mole) de n-butyl-2 nitro-5 benzofuranne et de 44,3g (0,26 mole) de chlorure d'anisoyle dans 308ml de dichloréthane on ajoute, peu à peu, 59,8ml (0,50 mole) de tétrachlorure d'étain. On maintient la température à 23°C et on prolonge l'agitation pendant 24 heures. On verse sur 770ml d'eau glacée et on extrait avec 3 fois 150ml de dichloréthane. On lave à l'eau avec une solution d'hydrogénocarbonate de sodium à 5% et à nouveau à l'eau. On évapore à sec et on obtient ainsi un produit qui cristallise rapidement [pureté en chromatographie liquide haute pression (CLHP) : 91,69%]. On cristallise dans 250ml d'isopropanol et on obtient ainsi 59g de n-butyl-2 (méthoxy-4 benzoyl)-3nitro-5 benzofuranne.
Rendement : 83,5%
Pureté (CLHP) : 96,39%
P.F. : 95°C

En suivant un procédé analogue au procédé précédent, on a préparé les composés suivants :
(Méthoxy-4 benzoyl)-3 méthyl-2 nitro-5 benzofuranne
P.F. : 167°C (méthyl éthyl cétone)
Pureté (CLHP) : 99,9%
Isopropyl-2 (méthoxy-4 benzoyl)-3 nitro-5 benzofuranne
Huileux
pureté (CLHP) : 99,6%
(Méthoxy-4 benzoyl)-3 nitro-5 phényl-2 benzofuranne
P.F. : 153°C (méthyl éthyl cétone )
Pureté (CLHP) : 99,8%
(Méthoxy-4 benzoyl)-3 éthyl-2 nitro-5 benzofuranne
P.F. : 130°C (méthanol)
Pureté (CLHP) : 99,5%
(Méthoxy-4 benzoyl)-3 nitro-5 propyl-2 benzofuranne
P.F. : 73°C (méthanol)
Pureté (CLHP) : 99,1%
(Méthoxy-4 benzoyl)-2 méthyl-3 nitro-5 benzofuranne
P.F. : 180°C
Pureté (CLHP) : 99,02%
(Méthoxy-4 benzoyl)-3 méthyl-2 nitro-7 benzofuranne
P.F. : 130-132°C
Pureté (CLHP) : 98,32%
(Méthoxy-4 benzoyl)-2 n-butyl-3 nitro-5 indole

P.F. : 142°C (heptane/acétate d'éthyle)
(Méthoxy-4 benzoyl)-2 n-butyl-3 méthyl-1 nitro-5 indole
P.F. : 102°C (éthanol)
(Méthoxy-4 benzoyl)-3 éthyl-2 méthyl-1 nitro-4 indole
P.F. : 136°C (heptane/isopropanol 6/4)

d) n-Butyl-2 (hydroxy-4 benzoyl)-3 nitro-5 benzofuranne

On chauffe à reflux pendant 20 heures, 69,7g (0,20 mole) de n-butyl-2 (méthoxy-4 benzoyl)-3 nitro-5 benzofuranne dans 510ml de dichloréthane en présence de 60g (0,45 mole) de chlorure d'aluminium. Après réaction, on laisse refroidir, on verse sur 510ml d'eau glacée, on essore, on lave la phase organique jusqu'à neutralité et on sèche le produit final sous vide à 50°C.

De cette manière, on obtient 61,2g de n-butyl-2 (hydroxy-4 benzoyl)-3 nitro-5 benzofuranne.
Rendement : 90,1%
Pureté (CLHP) : 99,19%
P.F. : 121°C

En suivant le même procédé que décrit ci-dessus, on a préparé les composés suivants :
(Hydroxy-4 benzoyl)-3 méthyl-2 nitro-5 benzofuranne
P.F. : 182°C (isopropanol)
Pureté (CLHP) : 99,7%
(Hydroxy-4 benzoyl)-3 isopropyl-2 nitro-5 benzofuranne
P.F. : 132°C
Pureté (CLHP) : 99,9%
(Hydroxy-4 benzoyl)-3 nitro-5 phényl-2 benzofuranne
P.F. : 207°C (chloroforme)
Pureté (CLHP) : 100%
Ethyl-2 (hydroxy-4 benzoyl)-3 nitro-5 benzofuranne
P.F. : 152°C (dichloréthane)
Pureté (CLHP) : 99,4%
(Hydroxy-4 benzoyl)-3 nitro-5 n-propyl-2 benzofuranne
P.F. : 119°C (toluène). Pureté (CLHP) : 98,8%
(Hydroxy-4 benzoyl)-2 méthyl-3 nitro-5 benzofuranne
P.F. : 235°C (méthanol)
Pureté (CLHP) : 96,19%
(Hydroxy-4 benzoyl)-3 méthyl-2 nitro-7 benzofuranne
P.F. : 201°C (méthyl éthyl cétone)
Pureté (CLHP) : 98,51%

e) n-Butyl-2[(di-n-butylamino-3 propoxy)-4 benzoyl-3 nitro-5 benzofuranne

On agite pendant 0,5 heure un mélange constitué de 11,9g (0,035 mole) de n-butyl-2 (hydroxy-4 benzoyl)-3 nitro-5 benzofuranne et de 4,8g (0,035 mole) de carbonate de potassium dans 60ml de méthyl éthyl cétone. On ajoute alors 7,2g (0,035 mole) de chloro-1 di-n-butylamino-3 propane et on chauffe à reflux durant 20 heures. On laisse refroidir, on essore les sels formés, lave avec de la méthyl éthyl cétone et évapore à sec. On dissout le résidu dans 250ml d'éther éthylique et on lave 2 fois avec 500ml d'hydroxyde de sodium à 5%. On lave à l'eau, sèche sur sulfate de sodium, essore et décolore avec du charbon actif. On filtre puis on évapore.

De cette manière, on obtient 15,8g de n-butyl-2 [(di-n-butyl-amino-3 propoxy)-4 benzoyl]-3 nitro-5 benzofuranne.
Rendement : 88,76%
Pureté (CLHP) : 98,25%
P.F. (oxalate) : 84°C (éther/isopropanol)

En suivant un procédé analogue à celui décrit ci-dessus, on a obtenu les composés suivants :
[(Di-n-butylamino-3 propoxy)-4 benzoyl]-3 nitro-5 méthyl-2 benzofuranne.
P.F. : 63°C (éther isopropylique)
Pureté (CLHP) : 98,9%
[(Tertiobutylamino-3 propoxy)-4 benzoyl]-3 n-butyl-2 nitro-5 benzofuranne
P.F. (oxalate acide) : 244°C (acétone/méthanol)
{[N-Méthyl N-(diméthoxy-3,4 β-phénéthyl)amino-3 propoxy]-4 benzoyl}-3 nitro-5 n-butyl-2 benzofuranne
P.F. (oxalate acide) : 114°C (acétone)

[(Di-n-butylamino-3 propoxy)-4 benzoyl]-3 éthyl-2 nitro-5 benzofuranne
P.F. (chlorhydrate) : 139°C (acétate d'éthyle)
Pureté (CLHP) : 99,6%
[(Di-n-butylamino-3 propoxy)-4 benzoyl]-3 nitro-5 n-propyl -2 benzofuranne
P.F. : 126°C (acétate d'éthyle). Pureté : 97,9%
n-Butyl-2 [(diéthylamino-3 propoxy)-4 benzoyl]-3 nitro-5 benzofuranne
P.F. (chlorhydrate) : 131°C (acétate d'éthyle)
Pureté (CLHP) : 98,4%
[(Di-n-butylamino-3 propoxy-4 benzoyl]-3 méthyl-2 nitro-7 benzofuranne
P.F. (toluènesulfonate) : 118°C (acétate d'éthyle)
Pureté (CLHP) : 99,44%
[(Di-n-butylamino-3 propoxy)-4 benzoyl]-2 méthyl-3 nitro-5 benzofuranne
P.F. (fumarate acide) : 148°C (acétate d'éthyle)
Pureté (CLHP) : 99,8%
[(Di-n-butylamino-3 propoxy)-4 benzoyl]-3 nitro-5 isopropyl-2 benzofuranne
Huile
Pureté (CLHP) : 99%
[(Di-n-butylamino-3 propoxy)-4 benzoyl]-3 nitro-5 phényl-2 benzofuranne
P.F. : 95°C (éther isopropylique)
Pureté (CLHP) : 98,5%

f) Amino-5 [(di-n-butylamino-3 propoxy)-4 benzoyl]-3 n-butyl-2 benzofuranne

Dans un appareil à hydrogéner, on agite, sous pression de 3,4 atmosphères (3,44x$10^5$ Pa) d'hydrogène, 20,4g (0,04 mole) de n-butyl-2 [(di-n-butylamino-3 propoxy)-4 benzoyl]-3 nitro-5 benzofuranne dans 200ml d'éthanol en présence de 0,6g d'oxyde de platine.
Lorsque la pression atteint 2,7 atm (2,73x$10^5$ Pa), la réaction est terminée, ce qui nécessite environ 20 minutes.

De cette manière, on obtient l'amino-5 [(di-n-butylamino-3 propoxy)-4 benzoyl]-3 n-butyl-2 benzofuranne avec un rendement de 98,4%.
Pureté (CLHP) : 95,28%

**EXEMPLE 2**

Préparation du dioxalate d'amino-5 [(di-n-butylamino-3 propoxy)-4 benzoyl]-3 méthyl-2 benzofuranne

A une solution d'amino-5 [(di-n-butylamino-3 propoxy)-4 benzoyl]-3 méthyl-2 benzofuranne dans l'éther diéthylique, on ajoute une solution d'acide oxalique dans l'éther diéthylique. On filtre et on recristallise dans le méthanol.

De cette manière, on obtient le dioxalate d'amino-5 [(di-n-butylamino-3 propoxy)-4 benzoyl]-3 méthyl-2 benzofuranne.
P.F. : 136°C
Pureté (CLHP) : 99,3%

**EXEMPLE 3**

Préparation du chlorhydrate de n-butyl-2 [(di-n-butylamino-3 propoxy)-4 benzoyl]-3 méthylsulfonamido-5 benzofuranne **(SR 33589B)**

a) n-Butyl-2 [(di-n-butylamino-3 propoxy)-4 benzoyl]-3 méthylsulfonamido-5 benzofuranne

A une solution de 68,3g (0,15 mole) d'amino-5 [(di-n-butylamino-3 propoxy)-4 benzoyl]-3 n-butyl-2 benzofuranne et de 23,6g (0,23 mole) de triéthylamine dans 750ml de dichloréthane, on ajoute, goutte à goutte, une solution de 17,6g (0,154 mole) de chlorure de méthanesulfonyle dans 375ml de dichloréthane. On agite durant 20h et on verse dans 500ml d'eau. On décante, on lave à l'eau et on évapore à sec. On purifie alors par chromatographie d'élution sur colonne de silice (éluant : acétate d'éthyle), le produit brut ainsi obtenu (79,5g; rendement brut : 100%).

De cette manière, on recueille 48g de n-butyl-2 [(di-n-butylamino-3 propoxy)-4 benzoyl]-3 méthylsulfonamido-5 benzofuranne purifié.
Rendement : 61,6%

Un traitement par l'hexane du produit ainsi obtenu a fourni une fraction de 44g à l'état cristallin (pureté par CLHP :

96,1%) et une fraction de 4g à l'état cristallin (pureté par CLHP : 99%)
P.F. : 65,3%

b) Chlorhydrate de n-butyl-2 [(di-n-butylamino-3 propoxy)-4 benzoyl]-3 méthylsulfonamido-5 benzofuranne

On dissout 2g de n-butyl-2 [(di-n-butylamino-3 propoxy)-4 benzoyl]-3 méthylsulfonamido-5 benzofuranne dans 40ml d'acétate d'éthyle anhydre.

Tout en agitant, on ajoute de l'éther chlorhydrique jusqu'à pH =3. Après quelques minutes, le chlorhydrate commence à précipiter. On le filtre après 0,75 heure , ce qui fournit 2,03g d'un produit blanc.

De cette manière, on recueille le chlorhydrate de n-butyl-2 [(di-n-butylamino-3 propoxy)-4 benzoyl]-3 méthylsulfonamido-5 benzofuranne.
P.F. : 143°C (acétone)

**EXEMPLE 4**

Préparation du dioxalate de l'amino-5 n-butyl-2 [(di-n-butylamino-2 éthoxy)-4 benzoyl]-3 benzofuranne

a) [(Bromo-2 éthoxy)-4 benzoyl]-3 n-butyl-2 nitro-5 benzofuranne

On agite pendant 0,5h un mélange constitué de 23,22g (0,06 mole) de n-butyl-2 (hydroxy-4 benzoyl)-3 nitro-5 benzofuranne et 10g (0,07 mole) de carbonate de potassium anhydre finement broyé dans 400ml de méthyl éthyl cétone. On ajoute alors 45g (0,24 mole) de dibromo-1,2 éthane et on chauffe à reflux pendant 6h. On laisse refroidir, filtre les sels minéraux, lave à l'acétone et évapore à sec sous vide. On purifie le produit brut obtenu, par chromatographie d'élution sur colonne de silice (éluant : toluène).

De cette manière, on obtient 15g de [(bromo-2 éthoxy)-4 benzoyl]-3 n-butyl-2 nitro-5 benzofuranne après recristallisation dans le pentane.
Rendement : 56%
P.F. : 81°C
Pureté (CLHP) : 95,2%

En suivant le même procédé que celui décrit ci-dessus, on a préparé les composés suivants :
[(Bromo-5 pentoxy)-4 benzoyl]-3 n-butyl-2 nitro-5 benzofuranne
P.F. : 55°C (pentane)
Pureté (CLHP) : 98%
[(Bromo-3 propoxy)-4 benzoyl]-3 n-butyl-2 nitro-5 benzofuranne
Pureté (CLHP) : 91,28%

b) Chlorhydrate de n-butyl-2 [(di-n-butylamino-2 éthoxy)-4 benzoyl]-3 nitro-5 benzofuranne

On chauffe à reflux pendant 3 jours un mélange constitué de 15g (0,0336 mole) de [(bromo-2 éthoxy)-4 benzoyl]-3 n-butyl-2 nitro-5 benzofuranne, 17,3g (0,134 mole) de di-n-butylamine et 18,5g (0,134 mole) de carbonate de potassium anhydre dans 200ml de toluène. On laisse refroidir, verse dans l'eau et décante la phase organique. On extrait la phase aqueuse avec 3 fois 50ml de toluène; on réunit les phases organiques et on lave à l'eau. On sèche, sur sulfate de sodium, filtre et évapore à sec sous vide. On purifie alors par chromatographie d'élution sur colonne de silice (éluants : hexane/acétate d'éthyle 8/2) ce qui fournit 12,7g (rendement : 76,4%) de composé désiré sous forme basique. On forme le chlorhydrate dans l'éther éthylique et on recristallise dans l'acétate d'éthyle.

De cette manière, on obtient le chlorhydrate de n-butyl-2 [(di-n-butylamino-2 éthoxy)-4 benzoyl]-3 nitro-5 benzofuranne
P.F. : 119,5°C
Pureté (CLHP) : 100%

En suivant un procédé analogue à celui décrit ci-dessus, on a obtenu les composés ci-dessous :
Oxalate acide de n-butyl-2 [(di-n-butylamino-5 pentoxy)-4 benzoyl]-3 nitro-5 benzofuranne
P.F. : 106,7°C (isopropanol)
Pureté (CLHP) : 99,6%
n-Butyl-2 [(n-butylamino-3 propoxy)-4 benzoyl]-3 nitro-5 benzofuranne
Huile
Pureté (CLHP) : 97,8%

c) Dioxalate d'amino-5 n-butyl-2 [(di-n-butylamino-2 éthoxy)-4 benzoyl]-3 benzofuranne

Ce composé a été obtenu selon la méthode décrite à l'Exemple 1f.
P.F. : 155°C (isopropanol)
Pureté (CLHP) : 96%

**EXEMPLE 5**

Préparation du [(di-n-butylamino-3 propoxy)-4benzoyl]-3 bisméthylsulfonamido -5 éthyl-2 benzofuranne

A une solution de 10g (0,022 mole) d'amino-5 [(di-n-butylamino-3 propoxy)-4 benzoyl]-3 éthyl-2 benzofuranne et de 22,45g (0,22 mole) de triéthylamine dans 200ml de tétrachlorure de carbone, on ajoute, goutte à goutte, une solution de 7,56g (0,066 mole) de chlorure de méthanesulfonyle dans 40ml de tétrachlorure de carbone. On chauffe à reflux pendant 20h, verse sur un mélange de glace et d'eau puis décante la phase organique. On lave à l'eau, sèche sur sulfate de sodium, filtre et évapore à sec sous vide. On purifie alors par chromatographie d'élution sur colonne de silice (éluant : acétate d'éthyle) puis on recristallise le produit brut obtenu dans l'heptane.

De cette manière, on obtient 8,4g de [(di-n-butylamino-3 propoxy)-4 benzoyl]-3 bisméthylsulfonamido -5 éthyl-2 benzofuranne
Rendement : 63%
P.F. : 70°C
Pureté (CLHP) : 98,2%

**EXEMPLE 6**

Préparation du [(di-n-butylamino-2 éthoxy)-4 benzoyl]-3 n-butyl-2 méthylsulfonamido-5 benzofuranne **(SR 34488)**

On agite pendant 4h un mélange constitué de 6,2g (0,01 mole) de [(di-n-butylamino-2 éthoxy)-4 benzoyl]-3 n-butyl-2 bisméthylsulfonamido-5 benzofuranne et 8g (0,2 mole) d'hydroxyde de sodium dans 190ml d'éthanol. On verse alors dans un grand volume d'eau et on extrait avec 3 fois 50ml d'acétate d'éthyle. On lave à l'eau, sèche sur sulfate de sodium, filtre et évapore à sec sous vide. On purifie ensuite par chromatographie d'élution sur colonne de silice (éluant : acétate d'éthyle). On agite alors le produit obtenu, dans de l'heptane jusqu'à obtention de cristaux. De cette manière, on recueille 4g de [(di-n-butylamino-2 éthoxy)-4 benzoyl]-3 n-butyl-2 méthylsulfonamido-5 benzofuranne après recristallisation dans l'heptane.
Rendement : 74%
P.F. : 86°C
Pureté (CLHP) : 98,08%

**EXEMPLE 7**

préparation du dioxalate de n-butyl-2 [(di-n-butyiamino-3 propionamido)-4 benzoyl]-3 amino-5 benzofuranne **(SR 34512A)**

a) Acide (chloro-3 propionamido)-4 benzoïque

Dans un ballon de 250 mL, on dissout 13,7g (0,1 mole) d'acide p-aminobenzoïque dans 100ml d'acide acétique à 10°C. Sous agitaation et à 10°C, on additionne alors 9,5 ml (12,7g; 0,1 mole) de chlorure de chloro-3 propionyle. Après 1h d'agitation à 10°C, on introduit une solution aqueuse d'acétate de sodium (25g dans 100ml d'eau) et on filtre le mélange réactionnel. On lave à l'eau le solide blanc ainsi obtenu et on sèche.

De cette manière, on obtient 13,2g d'acide (chloro-3 propionamido)-4 benzoïque avec un rendement de 58%.
P.F. : 225°C

En suivant le même procédé que celui décrit ci-dessus, on a préparé le composé suivant :
Acide (chloro-4 butyramido)-4 benzoïque
Rendement : 46%
P.F. : 220°C (décomposition)

b) Chlorure de (chloro-3 propionamido)-4 benzoyle

Dans un ballon de 250ml, muni d'un tube réfrigérant et d'une garde à chlorure de calcium, on place, sous agitation

et à reflux (70°C), un mélange de 13,2g (0,058 mole) d'acide (chloro-3 propionamido)-4 benzoïque dans 100ml de chlorure de thionyle. Après 0,5h, on évapore le chlorure de thionyle et on recristallise le produit brut réactionnel dans 200ml de toluène chaud. Après évaporation, on obtient 11,91g de chlorure de (chloro-3 priopionamido)-4 benzoyle
Rendement : 83%
P.F. : 130°C

En suivant le même procédé que celui décrit ci-dessus, on a préparé le composé suivant :
Chlorure de (chloro-4 butyramido)-4 benzoyle
Rendement : 69%
P.F. : 100°C (décomposition)

c) [(Chloro-3 propionamido)-4 benzoyl]-3 n-butyl-2 nitro-5 benzofuranne

Dans un ballon de 500ml surmonté d'une garde à chlorure de calcium, on dissout 11,91g (0,048 mole) de chlorure de (chloro-3 propionamido)-4 benzoyle et 10,61g (0,048 mole) de nitro-5 butyl-2 benzofuranne dans 280ml de dichloro-1,2 éthane. Sous bonne agitation et à 0°C, on introduit alors 19,84g (0,149 mole) de chlorure d'aluminium. On place le mélange réactionnel sous agitation et à température ambiante pendant 5h puis on verse sur 1kg de glace pilée contenant 50ml d'acide chlorhydrique concentré. Après agitation, on extrait le produit formé avec 2 fois 250ml d'acétate d'éthyle. On réunit les phases organiques, sèche sur sulfate de sodium, filtre et évapore les solvants sous vide (1330 Pa). On récupère ainsi un solide brut pesant 19,7g que l'on recristallise dans 250ml de toluène chaud.

De cette manière, on obtient 10,9g de [(chloro-3 propionamido)-4 benzoyl]-3 n-butyl-2 nitro-5 benzofuranne
Rendement : 53%

En suivant le même procédé que celui décrit précédemmennt, on a préparé le composé suivant :
[(Chloro-4 butyramido)-4 benzoyl]-3 n-butyl-2 nitro-5 benzofuranne
Rendement : 77%
P.F. : environ 100°C (décomposition)

d) Oxalate de n-butyl-2 [(di-n-butylamino-3 propionamido)-4 benzoyl]-3 nitro-5 benzofuranne

Dans un ballon de 100ml, surmonté d'un réfrigérant et d'une garde à chlorure de calcium, on dissout 10,9g (0,025 mole) de [(chloro-3 propionamido)-4 benzoyl]-3 n-butyl-2 nitro-5 benzofuranne dans 50ml de benzène sec à reflux. On ajoute alors au mélange réactionnel 13ml soit 9,9g (0,076 mole) de di-n-butylamine. Après 5h de réaction, le mélange est refroidi et hydrolysé avec 200ml d'eau. On extrait avec 2 fois 100ml d'acétate d'éthyle puis on sèche la phase organique sur sulfate de sodium.
On filtre et évapore les solvants sous vide (1330 P a). On chromatographie le produit brut réactionnel sur 800g de silice (éluant : dichloréthane/méthanol 95/5) pour récupérer 13,31g de composé désiré (rendement : 100%) sous forme basique. On forme alors le sel oxalique dans 250ml d'éthanol absolu et on le récupère par filtration.

De cette manière, on obtient l'oxalate de n-butyl-2 [(di-n-butylamino-3 propionamido)-4 benzoyl]-3 nitro-5 benzofuranne
P.F. : 147°C (éthanol)

De la même manière que celle décrite précédemment, on a préparé le composé suivant :
Oxalate de n-butyl-2[(di-n-butylamino-4 butyramido)-4 benzoyl]-3 nitro-5 benzofuranne
P.F. : 105°C

e) Dioxalate de n-butyl-2[(di-n-butylamino-3 propionamido)-4 benzoyl]-3 amino-5 benzofuranne

Dans un ballon de 250ml, on dissout 9,58g (0,018 mole) de n-butyl-2 [(di-n-butylamino-3 propionamido)-4 benzoyl]-3 nitro-5 benzofuranne sous forme basique, dans 100ml d'éthanol absolu. On y ajoute 0,958g de palladium à 5% sur charbon actif et on place le mélange réactionnel sous atmosphère d'hydrogène. On agite fortement pendant 5h et on filtre sur verre fritté. On traite alors la solution éthanolique ainsi récupérée, avec 3,24g (0,036 mole) d'acide oxalique de façon à former le dioxalate qui précipite. On filtre et on recristallise le sel dans 100ml d'éthanol chaud.

De cette manière, on obtient 8,43g de dioxalate de n-butyl-2 [(di-n-butylamino-3 propionamido)-4 benzoyl]-3 amino-5 benzofuranne
Rendement : 70%
P.F. : 145°C

**EXEMPLE 8**

Préparation de l'oxalate acide de N-{n-butyl-2[(di-n-butylamino-3 propoxy)-4 benzoyl]-3 benzofuryl-5}-propane-1,3 sultame

a) Amino-5 n-butyl-2 benzofuranne

Dans un appareil d'hydrogénation, on agite, sous pression de 3,4 atm. (50 p.s.i.) d'hydrogène, 8,8g (0,04 mole) de n-butyl-2 nitro-5 benzofuranne dans 100ml d'éthanol en présence de 0,6g d'oxyde de platine. Lorsque la pression atteint 2,7 atm. (40 p.s.i.), la réaction est terminée, ce qui dure environ 20 min. On filtre le catalyseur et on évapore à sec sous vide pour obtenir 7,3g (rendement : 96,56%) d'un composé huileux brut.

De cette manière, on obtient l'amino-5 n-butyl-2 benzofuranne

b) (n-Butyl-2 benzofuryl)-5 ammonium-3 propane-1 sulfonate

On chauffe à reflux pendant 1h, 2,85g (0,015 mole) d'amino-5 n-butyl-2 benzofuranne et 1,85g (0,015 mole) de propane-1,3 sultone dans 500ml d'acétonitrile . Le produit attendu précipite en cours de réaction.

On laisse refroidir, essore, lave à l'éther éthylique et sèche sous vide pour fournir 2,9g de produit que l'on recristallise dans 450ml d'éthanol.

De cette manière, on obtient 1,6g de (n-butyl-2 benzofuryl-5 ammonium-3 propane-1 sulfonate.
Rendement : 34 %
P.F. : 250 - 253°C

c) (n-Butyl-2 benzofuryl-5) propane-1,3 sultame

On triture dans un mortier pendant 20 min, un mélange de 0,78 g (0,0025 mole) de (n-butyl-2 benzofuryl)-5 ammonium-3 propane-1 sulfonate avec 1,05 g (0,005 mole) de pentachlorure de phosphore. On dilue ensuite avec de l'eau glacée, triture, essore, lave à l'eau et sèche sous vide. De cette manière, on obtient 0,4 g de (n-butyl-2 benzofuryl-5) propane-1,3 sultame après recristallisation dans le cyclohexane.
Rendement : 54,5 %
P.F. : 84,5 - 86°C

d) N-[n-butyl-2(méthoxy-4 benzoyl)-3 benzofuryl-5] propane-1,3 sultame

A une solution de 7,35 g (0,025 mole) de (n-butyl-2 benzofuryl-5) propane-1,3 sultame et de 5,55 g (0,0325 mole) de chlorure d'anisoyle dans 38 ml de dichloréthane, on ajoute, en 45 min., 16,25 g (0,0625 mole) de tétrachlorure d'étain. On continue l'agitation pendant 3h et on verse sur un mélange de glace et d'eau. On agite pendant 30 min., lave la phase organique avec 3 fois 50 ml d'eau, 1 fois avec 100 ml d'une solution d'hydrogénocarbonate de sodium saturée puis à nouveau avec 100 ml d'eau. Après évaporation, on obtient 9 g de produit que l'on purifie par chromatographie d'élution sur silice avec du chloroforme comme éluant. De cette manière, on obtient 6 g de N-[n-butyl-2 (méthoxy-4 benzoy)-3 benzofuryl-5] propane-1,3 sultame après recristallisation dans l'éthanol.
Rendement : 56,1 %
P.F : 104 - 107°C

e) N-[n-Butyl-2 (hydroxy-4 benzoyl)-3 benzofuryl-5]-propane-1,3 sultame

On chauffe à reflux pendant 4 h, 2,15 g (0,005 mole) de N-(n-butyl-2 méthoxy-4 benzofuryl-5) propane-1,3 sultame dans 33 ml de dichloréthane en présence de 2,66 g (0,02 mole) de chlorure d'aluminium. On laisse refroidir et on verse sur un mélange de glace et d'eau. On extrait la phase organique avec 3 fois 20 ml d'hydroxyde de sodium à 5 % puis on extrait cette solution avec 2 fois 25 ml de dichlorométhane. On acidifie avec de l'acide chlorhydrique concentré et on laisse la solution acide sous agitation jusqu'à cristallisation. On essore, lave à l'eau et sèche sous vide. De cette manière, on obtient 1,8 g de N-[n-butyl-2(hydroxy-4 benzoyl)-3 benzofuryl-5] propane-1,3 sultame.
Rendement : 86,9 %
P.F. : 79 - 84 ° C

f) Oxalate acide de N-{n-butyl-2 [(di-n-butylamino-3 propoxy)-4 benzoyl]-3 benzofuryl-5]-propane-1,3 sultame

Ce composé a été obtenu selon la méthode décrite à l'Exemple 1 e.

P.F. : 75 °C (isopropanol)

**EXEMPLE 9**

Fumarate acide de n-butyl-2 {[(di-n-butylamino-3 propoxy)-4 phényl]hydroxyméthyl}-3 méthylsulfonamido-5 benzofuranne (SR 34173 A)

Dans un ballon de 5 l refroidi à 0° C, on introduit 53,1 g (0,0954 mole de n-butyl-2 [(di-n-butylamino-3 propoxy)-4 benzoyl]-3 méthylsulfonamido-5 benzofuranne, 2,5 l de tétrahydrofuranne et 285 ml de méthanol. On ajoute alors, par portions, 5,7 g (0,152 mole) de borohydrure de sodium. Lorsque l'addition est terminée, on agite encore durant 2 heures. On contrôle par chromatographie sur couche mince (CCM) la disparition du produit carbonylé de départ et on ajoute au milieu réactionnel 930 ml d'eau et 930 ml de dichlorométhane. On décante la phase organique et on extrait la phase aqueuse avec 2 fois 400 ml de dichlorométhane. On réunit les phases organiques, on les lave à l'eau et on sèche sur sulfate de sodium. On filtre et on évapore à sec sous vide. On ajoute de l'éther isopropylique au résidu obtenu et on chauffe. On filtre à chaud un produit insoluble peu important et on laisse cristalliser pour obtenir le produit attendu sous forme basique. On reprend dans l'éther et on forme le fumarate par addition d'acide fumarique dans l'éther.

De cette manière, on recueille le fumarate acide de n-butyl-2 {[(di-n-butylamino-3 propoxy)-4 phényl] hydroxyméthyl}-3 méthylsulfonamido-5 benzofuranne.
Rendement : 52,5 %.
P.F. : 94 ° C.

**EXEMPLE 10**

n-Butyl-2 [(di-n-butylamino-3 propoxy)-4 benzyl]-3 méthylsulfonamido-5 benzofuranne (SR 34162)

Dans un ballon de 500 ml, refroidi à 0°C, on introduit, sous courant d'azote, 100 ml d'acide trifluoroacétique. On ajoute alors, par portions, 0,6 g (0,016 mole) de borohydrure de sodium.
on agite pendant 0,5 h à 0°C et on ajoute, goutte à goutte, une solution de 5,58 g (0,01 mole) de n-butyl-2 [(di-n-butylamino-3 propoxy)-4 phényl] hydroxyméthyl}-3 méthylsulfonamido-5 benzofuranne dissout dans 500 ml de dichlorométhane. Lorsque l'addition est terminée, on agite encore durant 40 min. à 0°C puis on détruit l'excès de borohydrure de sodium au moyen de 5 ml d'eau. On évapore à sec sous vide et on ajoute au résidu 250 ml d'eau et 250 ml de dichlorométhane. On sépare la phase organique. On lave avec une solution à 2,5 % de NaOH puis avec de l'eau et on sèche sur sulfate de sodium. On filtre et on évapore à sec sous vide. On purifie par chromatographie d'élution sur silice avec un mélange dichlorométhane/méthanol 85/15. On agite alors avec de l'hexane le produit cristallin obtenu et on filtre.

De cette manière, on obtient le n-butyl-2 [(di-n-butylamino-3 propoxy)-4 benzyl] -3 méthylsufonamido-5 benzofurame avec un rendement de 66,7 %.
Pureté (CLHP) : 97, 1 %.

**EXEMPLE 11**

Préparation du dioxalate d'amino-5[(di-n-butylamino-3 propoxy)-4 benzoyl]-3 éthyl-2 benzothiophène (SR 34407 A)

a) Acétyl-2 nitro-5 benzothiophène

On porte à 50°C, une solution de 18,4 g (0,1 mole) de chloro-2 nitro-5 benzaldéhyde dans 80 ml d'éthanol. Sous bonne agitation, on ajoute un mélange de 12 g de sulfure de sodium ($Na_2S$, 9 $H_20$) et 1,6 g de soufre. On poursuit le chauffage à 50°C pendant 0,5 h et l'on enregistre un précipité jaune. On refroidit à 20°C et on ajoute une solution de 6 g d'hydroxyde de sodium et 6 g de sulfure de sodium dans 40 ml d'eau. On poursuit l'agitation à cette température pendant 0,5 h pour obtenir une solution rouge de nitro-5 thio-2 benzaldéhyde. On refroidit à 10°C au moyen d'un bain de glace et ajoute 9,2 g (0,1 mole) de chloracétone. On poursuit l'agitation à la température ambiante pendant 2 h puis on verse dans 200 ml d'eau. On neutralise avec de l'acide acétique et filtre le précipité brun ainsi formé. On sèche sous vide à la température de 50°C, ce qui fournit 21 g d'acétyl-2 nitro-5 benzothiophène que l'on purifie par chromatographie sur colonne de silice (éluant : dichloréthane/heptane 9/1).
P.F. : 103°C
Rendement : 85 %.

b) Ethyl-2 nitro-5 benzothiophène

On refroidit, au moyen d'un bain de glace/chlorure de sodium à 0°C, une solution de 22,6 g (0,195 mole) de triéthylamine dans 50 ml de dichlorométhane. On introduit ensuite du trifluorure de bore jusqu'à saturation du milieu (environ 25 g). En maintenant la température à 0°C, on ajoute une solution de 14,4 g (0,065 mole) d'acétyl-2 nitro-5 benzothiophène dans 50 ml de dichlorométhane. On poursuit l'agitation à la température de 0°C pendant 0,5 h en maintenant un très léger courant de trifluorure de bore. On décompose le complexe formé avec une solution saturée de chlorure de sodium, lave trois fois la fraction organique avec une solution de chlorure de sodium et sèche sur du sulfate de sodium anhydre. On filtre et évapore le solvant à l'évaporateur rotatif. De cette manière, on obtient 16,6 g d'éthyl-2 nitro-5 benzothiophène sous forme d'un solide jaune, que l'on purifie par chromatographie sur colonne de silice (éluant : heptane/acétate d'éthyle 9/1).
P.F. : 55°C
Rendement : 61,9 %.

c) (Méthoxy-4 benzoyl)-3 éthyl-2 nitro-5 benzothiophène

On dissout 9 g (0,0434 mole) d'éthyl-2 nitro-5 benzothiophène dans 300 ml de dichloréthane. On refroidit la solution à 0 °C au moyen d'un bain de glace/chlorure de sodium. On ajoute alors, par portions 28,9 g (0,217 mole) de chlorure d'aluminium puis, goutte à goutte, 7,5 g (0,0434 mole) de chlorure d'anisoyle dans 100 ml de dichloréthane. On agite à la température ambiante pendant 3 h, verse sur de la glace et lave deux fois la fraction organique avec de l'eau. On sèche sur sulfate de sodium anhydre, filtre et évapore le solvant à l'évaporateur rotatif.

De cette manière on obtient 17 g de (méthoxy-4 benzoyl)-3 éthyl-2 nitro-5 benzothiophène que l'on purifie par chromatographie sur colonne de silice (éluant : heptane/acétate d'éthyle 7/3).
P.F. : 103°C.
Rendement : 60,1 %.

d) (Hydroxy-4 benzoyl)-3 éthyl-2 nitro-5 benzothiophène

On chauffe à 185°C pendant 2,5 h un mélange de 7,7 g (0,0225 mole) de (méthoxy-4 benzoyl)-3 éthyl-2 nitro-5 benzothiophène et 35 g de chlorhydrate de pyridine. On refroidit et reprend avec 100 ml d'eau. On filtre, lave à l'eau le produit sur filtre et sèche sous vide à la température de 50°C. Après purification par chromatographie sur colonne de silice (éluant : heptane/ acétate d'éthyle 6/4), on obtient 5,6 g d' (hydroxy-4 benzoyl)-3 éthyl-2 nitro-5 benzothiophène.
P.F. : 210°C
Rendement : 76 %

e) Oxalate acide de [(di-n-butylamino-3 propoxy)-4 benzoyl] -3 éthyl-2 nitro-5 benzothiophène

Ce composé a été obtenu selon la méthode décrite à l'Exemple 1e.
P.F. : environ 86°C (éther diéthylique)

De manière analogue, on a préparé le composé suivant : Chlorhydrate de {[N-méthyl N- (diméthoxy-3,4β phénéthyl) amino-3 propoxy-4 benzoyl} -3 éthyl-2 nitro-5 benzothiophène.
P.F. : 168°C (acétate d'éthyle/isopropanol 8/1)

f) Dioxalate d'amino-5 [(di-n-butylamino-3 propoxy)-4 benzoyl]-3 éthyl-2 benzothiophène

ce composé a été obtenu selon la méthode décrite aux Exemples 1f et 2.
P.F. : 136°C (acétate d'éthyle/éthanol 8/2)

**EXEMPLE 12**

Préparation du dichlorhydrate d'amino-5 [(di-n-butylamino-3 propoxy)-4 benzoyl]-3 n-butyl-2 benzothiophène (SR 34479 A)

a) Butyroyl-2 nitro-5 benzothiophène

Ce composé a été obtenu de manière analogue à celle décrite à l'Exemple 11a.
Rendement : 52,2 %

P.F. : 158°C

b) n-Butyl-2 nitro-5 benzothiophène

Ce composé a été obtenu selon une méthode analogue à celle décrite à l'Exemple 11b.
Huile consistante .
Rendement : 72,3 %.

c) (Hydroxy-4 benzoyl)-3 n-butyl-2 nitro-5 benzothiophène

On dissout 7,8 g (0,033 mole) de n-butyl-2 nitro-5 benzothiophène dans 250 ml de dichloréthane. On refroidit la solution à 0°C au moyen d'un bain de glace/chlorure de sodium puis on ajoute, par portions, 22 g (0,165 mole) de chlorure d'aluminium. On additionne ensuite, goutte à goutte, 6 g (0,35 mole) de chlorure d'anisoyle dans 50 ml de dichloréthane. On agite à la température ambiante durant environ 12 h puis on verse sur de la glace. On lave deux fois la fraction organise avec de l'eau, on sèche sur du sulfate de sodium anhydre, filtre et évapore le solvant à l'évaporateur rotatif. On purifie ensuite le résidu huileux par chromatographie sur colonne de silice (éluant : dichloréthane/acétate d'éthyle 98/2).
De cette manière, on obtient 7,5 g d' (hydroxy-4 benzoyl)-3 n- butyl-2 nitro-5 benzothioph'
Rendement : 64,1 %
P.F. : 147°C

d) Oxalate acide de [(di-n-butylamino-3 propoxy)-4 benzoyl]-3 n-butyl-2 nitro-5 benzothiophène

Ce composé a été obtenu selon le procédé de l'Exemple 7e.
P.F. : environ 88°C (éther diéthylique)
e) Dichlorhydrate d'amino-5 [(di-n-butylamino-3 propoxy-4 benzoyl] -3 n-butyl-2 benzothiophène
Ce composé a été obtenu selon le procédé décrit aux Exemples 1 f et 2.
P.F. : 116°C (éther diéthylique).

**EXEMPLE 13**

Préparation de l'oxalate d'amino-5 [(di-n-butylamino-3 propoxy)-4 benzoyl]-2 n-butyl-3 benzothiophène (SR 34224 A)

a) Chlorure de l'acide carboxy-2 n-butyl-3 nitro-5 benzothiophène

On réalise une suspension de 15,2 g (0,054 mole) de carboxy-2 n-butyl-3 nitro-5 benzothiophène (brevet RFA 2.854.014) dans 50 ml de chlorure de thionyle et 1 ml de N,N-diméthylformamide. On agite et chauffe jusqu'à dissolution complète de l'acide puis on distille l'excès de chlorure de thionyle à l'évaporateur rotatif. On reprend le résidu solide dans de l'heptane, filtre, lave le produit sur filtre avec de l'heptane et sèche sous vide à la température de 60°C.
De cette manière, on obtient 16,4 g de chlorure de l'acide carboxy-2 n-butyl-3 nitro-5 benzothiophène.
P.F. : 96°C

b) (Hydroxy-4 benzoyl)-2 n-dutyl-3 nitro-5 benzothiophène

On dissout 7,3 g (0,0245 mole) de chlorure de l'acide carboxy-2 n-butyl-3 nitro-5 benzothiophène dans 240 ml de dichloréthane, on refroidit à 0°C et on ajoute 26,3 g (0,122 mole) de chlorure d'aluminium. Après 0,5 h d'agitation à 0°C, on ajoute 7,94 g (0,0735 mole) d'anisole. On laisse revenir à la température ambiante puis on chauffe à 60°C pendant 6 heures. On verse dans de l'eau glacée, sépare la fraction organique et lave la fraction aqueuse au dichloréthane. On réunit les fractions organiques et on lave à l'eau, sèche sur sulfate de sodium, filtre et chasse le solvant avec un évaporateur rotatif. On purifie alors par chromatographie sur colonne de silice (éluant : dichloréthane/acétate d'éthyle 98/2).
De cette manière, on obtient 2,7 g d' (hydroxy-4 benzoyl)-2 n-butyl-3 nitro-5 benzothiophène.
Rendement : 31 %
P.F. : 182°C (acide acétique/eau 9/1).

c) [(Di-n-butylamino-3 propoxy)-4 benzoyl]-2 n-butyl-3 nitro-5 benzothio-phène

On dissout 3,4 g (0,0095 mole) d' (hydroxy-4 benzoyl)-2 n-butyl-3 nitro-5 benzothiophène dans 100 ml de N,N-diméthylformamide. On agite puis on additionne 7 g de carbonate de potassium anhydre broyé et 1,95 g (0,0095 mole) de chlorure de di-n-butylaminopropyle. On chauffe à 100°C pendant 0,5 heure, refroidit et verse le produit réactionnel dans l'eau. On extrait avec de l'acétate d'éthyle, lave la fraction organique à l'eau, sèche sur sulfate de sodium, filtre et chasse l'acétate d'éthyle avec un évaporateur rotatif, ce qui fournit un résidu huileux consistant. On purifie alors par chromatographie sur colonne de silice (éluant : dichloréthane/éthanol 9/1).

De cette manière, on obtient le [(di-n-butylamino-3 propoxy)-4 benzoyl] -2 n-butyl-3 nitro-5 benzothiophène.

d) Oxalate d'amino-5 [(di-n-butylamino-3 propoxy)-4 benzoyl]-2 n-butyl-3 benzothiophène

Ce composé a été obtenu selon le mode opératoire décrit aux Exemples 1 f et 2.

P.F. : 135°C (isopropanol)

**EXEMPLE 14**

Préparation du [(di-n-butylamino-3 propoxy)-4 benzoyl] -3 n-butyl-2 nitro-5 indole (SR 34127)

a) n-Butyl-2 nitro-5 indole

On refroidit, à 0°c à l'aide d'un bain réfrigérant glace/chlorure de sodium, une solution de 11 g (0,064 mole) de n-butyl-2 indole dans 50 ml d'acide sulfurique concentré. En maintenant cette température, on introduit, goutte à goutte, une solution de 5,4 g de nitrate de sodium dans 50 ml d'acide sulfurique concentré (durée : 1,5 h). On agite durant 0,25 h et on verse sur 400 g de glace broyée. On filtre le précipité jaune et on lave, à l'eau froide, le produit sur filtre.

De cette manière, on obtient 13,5 g de n-butyl-2 nitro-5 indole.

Rendement : 96,7 %
P.F. 105°c (isopropanol/eau 1/1)
Pureté (CLHP) : 99,1 %

De la même manière que précédemment, on a préparé le composé suivant : Nitro-5 phényl-2 indole.

Rendement : 95 %
P.F. : 193°C (isopropanol/eau)

b) (Méthoxy-4 benzoyl)-3 n-butyl-2 nitro-5 indole

Sous agitation, on chauffe à 150°C pendant 0,25 h (fin de dégagement d'acide chlorhydrique), un mélange de 20 g (0,091 mole) de n-butyl-2 nitro-5 indole et 16,2 g (0,091 mole) de chlorure de méthoxy-4 benzoyle. On reprend le mélange réactionnel dans du dichloréthane, on lave à l'eau et on chasse le solvant à l'évaporateur rotatif (résidu solide brun). On purifie alors par chromatographie sur colonne de silice (éluant : heptane/acétate d'éthyle 1/1).

De cette manière, on obtient 23 g de (méthoxy-4 benzoyl)-3 n butyl-2 méthoxy-5 indole.

Rendement : 72 %
P.F. : 170°C (heptane/isopropanol 8/2)
Pureté (CLHP) : 98,7 %
De la même manière que précédemment, on a préparé :
Méthoxy-4 benzoyl-2-n-butyl-3 nitro-5 indole
Rendement : 62,9 %
P.F. : 142°C (heptane/acétate d'éthyle 7/3)
(Méthoxy-4 benzoyl)-2n-butyl-3 méthyl-1 nitro-5 indole
Rendement : 64,5 %
P.F. : 102°C (éthanol)

c) (Hydroxy-4 benzoyl)-3 n-butyl-2 nitro-5 indole

On mélange 12,3 g (0,035 mole) de (méthoxy-4 benzoyl)-3 n-butyl-2 nitro-5 indole et 40,4 g (0,35 mole) de chlorhydrate de pyridine puis on chauffe à 180°C pendant 1,5 heure. On reprend dans l'eau, acidifie avec de l'acide chlorhydrique et filtre le précipité brun. On lave à l'eau, sur filtre, et on traite ensuite le produit brut avec une solution diluée d'hydroxyde de sodium et 5 g de charbon actif. On filtre sur plaque frittée et acidifie le filtrat avec de l'acide chlorhydrique. On filtre le précipité et lave,avec de l'eau,le produit sur filtre. On sèche alors sous vide à 60°C.

De cette manière, on obtient 9,4 g d'(hydroxy-4 benzoyl)-3 n-butyl-2 nitro-5 indole.

Rendement : 79,6 %
P.F. : 230°C (eau/isopropanol 6/4)
Pureté (CLHP) : 99,6 %

De manière analogue à celle décrite précédemment, on a préparé les composés suivants :

(Hydroxy-4 benzoyl)-2 n- butyl-3 nitro-5 indole
Rendement : 77,7 %
P.F. : 181°C (eau/isopropanol 8/2)
(Hydroxy-4 benzoyl)-2 n- butyl-3 méthyl-1 nitro-5 indole
Rendement : 84,7 %
P.F. : 172°C (isopropanol/eau 6/4)
(Hydroxy-4 benzoyl)-3 éthyl-2 méthyl-1 nitro-4 indole
P.F. : 191°C (eau/acide acétique 7/3)

d) [(Di-n-butylamino-3 propoxy)-4 benzoyl]-3 n-butyl-2 nitro-5 indole

On dissout 5 g (0,015 mole) d' (hydroxy-4 benzoyl)-3 n-butyl-2 nitro-5 indole dans 100 ml de N,N-diméthylformamide, on agite et on ajoute 12 g de carbonate de potassium anhydre broyé et 3 g (0,015 mole) de chlorure de di-n-butylaminopropyle. On chauffe à 100°C pendant 0,5 heure, on refroidit et on verse le produit réactionnel dans l'eau. On extrait à l'éther éthylique et on lave la fraction éthérée à l'eau. On sèche sur sulfate de sodium, filtre et chasse l'éther à l'évaporateur rotatif, ce qui fournit 5,1 g d'un résidu huileux que l'on purifie par chromatographie sur colonne de silice (éluant : dichloréthane + 5 % d'éthanol).

De cette manière, on obtient 2,9 g de [(di-n-butylamino-3 propoxy)-4 benzoyl] -3 n-butil-2 nitro-5 indole.
Rendement : 38,1 %
P.F. : 116°C

**EXEMPLE 15**

Préparation du dioxalate d'amino-5 [(di-n-butylamino-3 propoxy)-4 benzoyl]-3 n-butyl-2 indole (SR 34158 A)

A température ambiante et sous une pression de 7 kg/cm$^2$ (6,867 x 10$^5$ Pa), on hydrogène 4 g (0,0078 mole) de [(di-n-butylamino-3 propoxy)-4 benzoyl] -3 n-butyl-2 nitro-5 indole en solution dans 100 ml d'éthanol et en présence de 0,150 g d'oxyde de platine. On filtre et évapore l'éthanol, ce qui fournit 3,5 g d'une huile consistante [ rendement brut : 94,5 %; pureté (CLHP) : 95 %]. On purifie alors par chromatographie sur colonne de silice (éluant : heptane/éthanol 9/1), le produit basique ainsi obtenu et on le transforme en dioxalate en mileu éther diéthylique.

De cette manière, on obtient le dioxalate d'amino-5 [(di-n-butylamino-3 propoxy)-4 benzoyl]-3 n-butyl-2 indole.

**EXEMPLE 16**

Préparation du chlorhydrate de [(di-n-butylamino-3 propoxy)-4 benzoyl] -3 n-butyl-2 méthylsulfonamido-5 indole (SR 34138 A)

A la température ambiante et sous agitation, on ajoute, goutte à goutte, une solution de 5 g (0,04 mole) de chlorure de méthanesulfonyle dans 20 ml de dichloréthane, à une solution de 1,7 g (0,0035 mole) d'amino-5 [(di-n - butylamino-3 propoxy)-4 benzoyl] -3 n-butyl-2 indole, et de 0,6 g de triéthylamine dans 20 ml de dichloréthane. On poursuit l'agitation pendant 15 heures et on lave deux fois le produit de la réaction avec de l'eau. On sèche sur sulfate de sodium, filtre et évapore le dichloréthane, ce qui fournit 2,1 g d'un résidu huileux que l'on purifie par chromatographie sur colonne de silice (éluant : heptane/acétate d'éthyle 1/1). On recueille ainsi 1 g de [(di-n-butylamino-3 propoxy) - 4 benzoyl] -3 n butyl-2 méthyl-sufonamido-5 indole (rendement : 52,6 %; produit amorphe) que l'on reprend dans l'éther éthylique sec. On forme alors le chlorhydrate par ajout d'éther chlorhydrique.

De cette manière, on obtient 0,9 g de chlorhydrate de [(di-n-butylamino-3 propoxy)-4 benzoyl] -3 n-butyl-2 méthylsulfonamido-5 indole.
P.F. : 112°C
Pureté (CLHP) : 99,1 %.

**EXEMPLE 17**

Préparation du chlorhydrate de [(di-n-butylamino-3 propoxy)-4 benzoyl] - 3 n-butyl-2 méthyl-1 nitro-5 indole (SR 34147 A)

a) (Méthoxy-4 benzoyl) -3 n butyl-2 méthyl-1 nitro-5 indole

Sous agitation, on introduit, dans 300 ml de benzène, 0,8 g de 1,4,7,10,13,16-hexaoxacyclooctadécane (0,00303

mole) et 3,9 g (0,0351 mole) de tertiobutoxyde de potassium. Après 0,25 heure, on ajoute une solution de 10,7 g (0,0303 mole) de (méthoxy-4 benzoyl) - 3 n- butyl-2 nitro-5 indole dans 60 ml de benzène. On agite durant 0,25 heure puis on ajoute, goutte à goutte, 5 g (0,0303 mole) d'iodure de méthyle. On poursuit l'agitation à la température ambiante pendant 18 heures, puis on verse dans l'eau. On sépare la fraction benzénique, lave la fraction aqueuse au benzène et sèche sur sulfate de sodium. On filtre et on chasse le benzène à l'évaporateur rotatif, ce qui fournit un résidu solide brun. On cristallise alors dans un mélange acétate d'éthyle/heptane 1/1.

De cette manière, on obtient 10,3 g de (méthoxy-4 benzoyl) - 3 n-butyl-2 méthyl-1 nitro-5 indole.
P.F. : 168°C
Pureté (CLHP) : 98 %
De la même manière que précédemment, on a préparé le composé suivant : Méthyl-1 nitro-5 phényl-2 indole à partir de nitro-5 phényl-2 indole
Rendement : 92 %
P.F. : 185°C (isopropanol/heptane)

b) Hydroxy-4 benzoyl) -3 n- butyl-2 méthyl-1 nitro-5 indole

On mélange 10,3 g (0,028 mole) de (méthoxy-4 benzoyl) -3 n -butyl-2 méthyl-1 nitro-5 indole et 32,4 g (0,28 mole) de chlorhydrate de pyridine puis on chauffe à 180°C pendant 2 heures. On reprend dans l'eau, on acidifie avec de l'acide chlorhydrique et on filtre le précipité brun. On lave le produit brut à l'eau sur filtre puis on traite avec une solution diluée d'hydroxyde de sodium et 4 g de charbon actif. On filtre sur verre fritté et on acidifie le filtrat avec de l'acide chlorhydrique. On filtre le précipité formé, on lave le produit à l'eau sur filtre et on sèche sous vide à 60°C.

De cette manière, on obtient 9,3 g d'(hydroxy-4 benzoyl)-3 n butyl-2 méthyl-1 nitro-5 indole.
Rendement : 88 %
P.F. : 198°C (isopropanol/eau 6/4)
Pureté (CLHP) : 98, 8 %

c) Chlorhydrate de [(di-n-butylamino-3 propoxy)-4 benzoyl] - 3 n- butyl-2 méthyl-1 nitro-5 indole

Ce composé a été préparé selon le mode opératoire décrit à l'Exemple 14 paragraphe d au départ de 7 g (0,02 mole) d' (hydroxy-4 benzoyl) - 3 n - butyl-2 méthyl-1 nitro-5 indole, 150 ml de N,N-diméthylformamide, 15 g de carbonate de potassium anhydre broyé et 4,1 g (0,02 mole) de chlorure de di-n-butylaminopropyle, pour donner 9,1 g de composé sous forme basique (rendement : 87,5 %).
Cette base sous forme d'une huile cristallise au repos. On la purifie par chromatographie sur colonne de silice (éluant : heptane/éthanol 85/15)
P.F. (base) : 84°C (heptane)
P.F. : (chlorhydrate) : 140°C

**EXEMPLE 18**

Préparation du dioxalate d'amino-5 [(di-n-butylamino-3 propoxy) -4 benzoyl]- 3 n- butyl-2 méthyl-1 indole (SR 34156 A)

Ce composé a été préparé selon le mode opératoire de l'Exemple 15 au départ de 6,5 g (0,0124 mole) de [(di-n-butylamino-3 propoxy)-4 benzoyl] -3 n-butyl-2 méthyl-1 nitro-5 indole, 150 ml d'éthanol, 0,240 g d'oxyde de platine et au départ d'hydrogène.
La base obtenue sous forme brute se présente sous forme d'une huile consistante (rendement : 63,3 %). On la purifie par chromatographie sur colonne de silice (éluant : heptane/éthanol 95/5) puis on la transforme en dioxalate par traitement au moyen d'une solution d'acide oxalique dans l'éther diéthylique et on recristallise dans l'éthanol.

De cette manière, on obtient le dioxalate d'amino-5 [(di-n-butylamino-3 propoxy)-4 benzoyl] - 3 n-butyl-2 méthyl-1 indole.
P.F. : 168°C
Pureté (CLHP) : 100 %.

**EXEMPLE 19**

Préparation du chlorhydrate de [(di-n-butylamino-3 propoxy)-4 benzoyl]-3 n-butyl-2 méthylsulfonamido-5 méthyl-1 indole (SR 34152 A)

Ce composé a été obtenu selon le mode opératoire décrit à l'Exemple 16 au départ de 3,4 g (0,007 mole) d'amino-5 [(di-n-butylamino-3 propoxy)-4 benzoyl] -3 n- butyl-2 méthyl-1 indole, 1,2 g de triéthylamine, 40 ml de dichloréthane et 1,2 g (0,01 mole) de chlorure de méthanesulfonyle dans 40 ml de dichloréthane.
On purifie, la base obtenue sous forme brute, par chromatographie sur colonne de silice (éluant : heptane/éthanol 85/15). Cette base se présente sous forme amorphe (rendement : 50 %; P.F. : environ 80°C).
On forme alors le chlorhydrate en milieu éther éthylique par ajout d'éther chlorhydrique.

De cette manière, on obtient 1,6 g de chlorhydrate de [(di-n-butylamino-3 propoxy)-4 benzoyl] -3 n butyl-2 méthylsulfonamido-5 méthyl-1 indole.
P.F. : environ 100°C
Pureté (CLHP) : 98,14 %

**EXEMPLE 20**

Préparation du {[N-méthyl N-(diméthoxy-3,4 β -phénéthylamino-3 propoxy] - 4 benzoyl}-3 n- butyl-2 nitro-5 indole (SR 35175 A)

a) [(Chloro-3 propoxy) -4 benzoyl] - 3 n- butyl-2 nitro-5 indole

Sous agitation, on chauffe à 150°C pendant 0,25 h (fin de dégagement d'acide chlorhydrique), un mélange de 2,18 g (0,01 mole) de n-butyl-2 nitro-5 indole et 2,33 g (0,01 mole) de chlorure de (chloro-3-propoxy) -4 benzoyle. On reprend le mélange réactionnel dans du dichloréthane, on lave à l'eau et on chasse le solvant à l'évaporateur rotatif, ce qui donne 4,9 g d'un produit brut. On purifie alors par chromatographie sur colonne de silice (éluant : heptane/acétate d'éthyle 1/1).

De cette manière, on obtient 2,8 g de [(chloro-3 propoxy)-4 benzoyl] -3 n-butyl-2 nitro-5 indole après recristallisation dans un mélange heptane/acétate d'éthyle 1/1.
Rendement : 67,5 %
P.F. : 125°C
Pureté (CLHP) : 98,6 %

b) {[N-Méthyl N- (diméthoxy-3, 4β -phénéthylamino-3 propoxy]-4 benzoyl} -3 n-butyl-2 nitro-5 indole

Sous agitation, on chauffe à 60°C pendant 6h, un mélange de 6,15 g (0,015 mole) de [(chloro-3 propoxy) -4 benzoyl]-3 n-butyl-2 nitro-5 indole, 4,8 g (0,02 mole) de chlorhydrate de N-méthyl diméthoxy-3,4β- phénéthylamine, 9 g de carbonate de potassium anhydre, 1 g de 1,4,7,10,13,16-hexaoxacyclooctadécane et 75 ml d'acétonitrile. On verse le mélange dans l'eau, on extrait avec de l'éther éthylique et on lave, deux fois à l'eau, la fraction éthérée. On sèche sur sulfate de sodium, filtre et chasse l'éther à l'évaporateur rotatif. On purifie alors par chromatographie sur colonne de silice (éluant : éthanol).

De cette manière, on obtient 3,1 g de {[N-méthyl N-(diméthoxy-3,4 β -phénéthyl)amino-3 propoxy] -4 benzoyl} - 3 n- butyl-2 nitro-5 indole
Rendement : 36,9 %
P.F. (oxalate) : 211° C

**EXEMPLE 21**

Préparation du dioxalate d'amino-5 {[N-méthyl N-(diméthoxy-3,4 β-phénéthyl) amino-3 propxy]-4 benzoyl} -3 n-butyl-2 indole (SR 34176 A)

Ce composé a été préparé selon le mode opératoire décrit à l'Exemple 15 à partir de 2,1 g (0,0054 mole) de {[N-méthyl N-(diméthoxy-3,4β -phénéthyl) amino-3 propoxyl]-4 benzoyl} -3 n-butyl-2 nitro-5 indole, 75 ml d'éthanol, 0,106 g d'oxyde de platine ainsi que d'hydrogène, ce qui fournit 2,8 g d'un composé basique sous forme d'un produit gommeux (rendement : 96,5 %). On forme alors l'oxalate en mileu tétrahydrofuranne/éther éthylique 1/1.

De cette manière, on obtient le dioxalate d'amino-5 {[N-méthyl N-(diméthoxy-3,4β-phénéthyl) amino-3 propoxy]-4 benzoyl} - 3 n- butyl-2 indole
P.F. : 138°C (éthanol)

**EXEMPLE 22**

Préparation du chlorhydrate de {[N-méthyl N-(diméthoxy-3,4β -phénéthyl) amino-3 propoxy]-4 benzoyl}-3 n butyl-2 méthylsulfonamido-5 indole (SR 34196 A)

Ce composé a été préparé selon le mode opératoire décrit à l'Exemple 16 à partir de 2,1 g (0,00386 mole) d'amino-5 {[N-méthyl N-(diméthoxy 3,4β -phénéthyl) amino-3 propoxy]-4 benzoyl} - 3 butyl-2 indole, 0,6 g de triéthylamine, 30 ml de dichloréthane et 0,48 g (0,0042 mole) de chlorure de méthanesulfonyle dans 20 ml de dichloréthane.
P.F. : 125°C (isopropanol/éther)
Pureté (CLHP) : 99,8 %

**EXEMPLE 23**

Préparation de l'oxalate de {[N-méthyl N-(diméthoxy-3,4, β-phénéthyl) amino-3 propoxy]-4 benzoyl}-3 n-butyl-2 méthyl-1 nitro-5 indole **(SR 34230A)**

a) n-Butyl-2 méthyl-1 nitro-5 indole

On agite, pendant 0,25 heure, 8,3g (0,074 mole) de tertiobutoxyde de potassium et 1,7g de 1,4,7,10,13,16-hexaoxacyclooctadécane dans 600 ml de benzène. On ajoute ensuite une solution de 14g (0,064 mole) de n-butyl-2 nitro-5 indole dans 500ml de benzène. On agite pendant 0,5 heure à la température ambiante et on ajoute une solution de 9,1g (0,064 mole) d'iodure de méthyle dans 100ml de benzène. On poursuit l'agitation pendant 3 heures, lave à l'eau et sèche la solution benzénique sur sulfate de sodium. On filtre et évapore le solvant à l'évaporateur rotatif, ce qui fournit une huile qui cristallise au repos.

De cette manière, on obtient 13,6g de n-butyl-2 méthyl-1 nitro-5 indole.
Rendement : 91,5%
P.F. : 77°C (isopropanol/eau 8/2)
Pureté (CLHP) : 99,6%

b) [(Chloro-3 propoxy)-4 benzoyl]-3 n-butyl-2 méthyl-1 nitro-5 indole

Ce composé a été préparé selon le mode opératoire décrit à l'Exemple 20 paragraphe a au départ de 8,lg (0,035 mole) de n-butyl-2 méthyl-1 nitro-5 indole et de 8,2g (0,035 mole) de chlorure de (chloro-3 propoxy)-4 benzoyle. On purifie le produit obtenu, par chromatographie sur colonne de silice (éluant : dichloréthane/heptane 9/1).

De cette manière, on recueille le [(chloro-3 propoxy)-4 benzoyl]-3 n-butyl-2 méthyl-l nitro-5 indole avec un rendement de 60,1%.
P.F. : 125°C (isopropanol)
Pureté (CLHP) : 98,1%

c) Oxalate de {[N-méthyl N-(diméthoxy-3,4 β-phénéthyl)amino-3 propoxy]-4 benzoyl}-3 n-butyl-2 méthyl-1 nitro-5 indole

Ce composé a été obtenu selon le mode opératoire décrit à l'Exemple 20, paragraphe b au départ de 7,5g (0,017 mole) de [(chloro-3 propoxy)-4 benzoyl]-3 n-butyl-2 méthyl-1 nitro-5 indole, 3,93g (0,017 mole) de chlorhydrate de N-méthyl diméthoxy-3,4 β-phénéthylamine, 10g de carbonate de potassium anhydre, 1,1g de 1,4,7,10,13,16-hexaoxacyclooctadécane et 100ml d'acétonitrile.

De cette manière, on obtient 4,1 g d'oxalate de {[N-méthyl N-(diméthoxy-3,4 β-phénéthyl)amino-3 propoxy]-4 benzoyl}-3 n-butyl-2 méthyl-1 nitro-5 indole sous forme d'une huile.
Rendement : 41%
P.F. : environ 80°C (isopropanol)

**EXEMPLE 24**

Préparation du [(di-n-butylamino-3 propoxy-4 benzoyl]-3 n-butyl-2 di-n-butylaminopropyl-1 nitro-5 indole **(SR 34137A)**

On dissout 5g (0,015 mole) d'(hydroxy-4 benzoyl)-3 n-butyl-2 nitro-5 indole dans 100ml de N,N-diméthylformamide. On agite puis additionne 12g de carbonate de potassium anhydre broyé et 3g (0,015 mole) de chlorure de di-n-butylaminopropyle. On chauffe à 100°C pendant 0,5 heure puis on refroidit et verse le produit de la réaction dans l'eau. On extrait à l'éther éthylique et lave la fraction éthérée à l'eau. On sèche sur sulfate de sodium, filtre et chasse l'éther

avec un évaporateur rotatif, ce qui fournit 5,1g d'un résidu huileux. On purifie alors par chromatographie sur colonne de silice (éluant : dichloréthane + 5% d'éthanol).

De cette manière, on obtient 1g de [(di-n-butylamino-3 propoxy-4 benzoyl]-3 n-butyl-2 di-n-butylaminopropyl-1 nitro-5 indole.
P.F. : (dioxalate) : 100°C (isopropanol)

**EXEMPLE 25**

Préparation du chlorhydrate de [(di-n-butylamino-3 propoxy)-4 benzoyl]-3 n-butyl-2 méthylsulfonamido-5 méthyl-1 indole **(SR 34152A)**

On dissout 2,45g (0,005 mole) d'amino-5 [(di-n-butylamino-3 propoxy)-4 benzoyl]-3 n-butyl-2 méthyl-1 indole et 1,34g (0,0077 mole) d'anhydride de l'acide méthanesulfonique dans 100ml de dichlorométhane. On agite puis on additionne 1g de triéthylamine. On agite à température ambiante pendant 24 heures et on évapore le solvant à l'évaporateur rotatif. On reprend le résidu dans de l'acétate d'éthyle, lave avec une solution aqueuse de bicarbonate de sodium 2N puis à l'eau. On sèche sur sulfate de sodium, filtre et chasse l'acétate d'éthyle avec un évaporateur rotatif, ce qui fournit un résidu que l'on purifie par chromatographie sur colonne de silice (éluant : dichloréthane + 2,5% d'éthanol)et sèche sous vide. On forme ensuite le chlorhydrate de la base ainsi obtenue, par addition d'éther chlorhydrique.

De cette manière, on obtient le chlorhydrate de [(di-n-butylamino-3 propoxy)-4 benzoyl]-3 n-butyl-2 méthylsulfonamido-5 méthyl-1 indole.
P.F. : environ 100°C.

**EXEMPLE 26**

Préparation du [(di-n-butylamino-3 propoxy)-4 benzoyl]-3 méthyl-1 nitro-5 phényl-2 indole **(SR 34432)**

a) (Méthoxy-4 benzoyl)-3 méthyl-1 nitro-5 indole

On ajoute 10g (0,039 mole) de trifluorométhanesulfonate d'argent à une solution de 6,66g (0,039 mole) de chlorure d'anisoyle dans 100ml de dichlorométhane. On agite pendant 1h à la température ambiante et on ajoute ensuite 9,8g (0,039 mole) de méthyl-1 phényl-2 indole.
On agite à nouveau à la température ambiante pendant environ 48h puis on ajoute 40ml d'une solution d'hydroxyde de potassium 2N. On agite vigoureusement pendant 12h. On filtre, lave l'insoluble sur filtre avec un mélange de dichloréthane/isopropanol 1/1 puis on lave la fraction organique 2 fois avec de l'eau. On sèche sur sulfate de sodium anhydre, filtre et chasse le solvant à l'évaporateur rotatif. On sèche le résidu solide sous vide à la température de 60°C et recristallise dans un mélange dichloréthane/isopropanol 1/1.

De cette manière, on obtient 7g de (méthoxy-4 benzoyl)-3 méthyl-1 nitro-5 indole.
Rendement : 47%
P.F. : 229°C

b) (Hydroxy-4 benzoyl)-3 méthyl-1 nitro-5 indole

Ce composé a été obtenu selon la méthode décrite à l'Exemple 14c
Rendement : 87,36%
P.F. : 260°C (éthanol)

c) [(Di-n-butylamino-3 propoxy)-4 benzoyl]-3 méthyl-1 nitro-5 phényl-2 indole

Ce composé a été obtenu selon la méthode décrite à l'Exemple 14d
Rendement : 80%
P.F. : 82°C

**EXEMPLE 27**

Préparation de l'oxalate de [(di-n-butylamino-3 propoxy)-4 benzoyl]-3 n-butyl-2 méthylsulfonyl-1 indole **(SR 34353A)**

On ajoute 0,227g d'hydrure de sodium (suspension dans l'huile à 55%) à une solution de 2,2g (0,00475 mole) de [(di-n-butylamino-3 propoxy)-4 benzoyl]-3 n-butyl-2 indole dans 50ml de N,N-diméthylformamide et 25ml d'hexaméthylphosphoramide. On agite à température ambiante jusqu'à fin de dégagement d'hydrogène et on ajoute, ensuite, 0,544g (0,00475 mole) de chlorure de méthanesulfonyle dans 10ml de N,N-diméthylformamide. On poursuit l'agitation à la température ambiante pendant environ 15h puis on verse dans l'eau. On extrait à l'acétate d'éthyle, lave la fraction organique 2 fois à l'eau et sèche sur sulfate de sodium anhydre. On filtre et chasse le solvant à l'évaporateur rotatif ce qui fournit 1,5g de composé désiré sous forme basique (huile incolore consistante;rendement : 60%). On forme alors l'oxalate dans un mélange acétate d'éthyle/éther éthylique.

De cette manière, on obtient l'oxalate de [(di-n-butylamino-3 propoxy)-4 benzoyl]-3 n-butyl-2 méthylsulfonyl-1 indole.

P.F. : 90°C (acétate d'éthyle/éther éthylique)

**EXEMPLE 28**

Préparation de l'oxalate acide de n-butyl-2[(di-n-butlamino-3 propoxy)-4 benzoyl]-3 carbéthoxy-7 indolizine **(SR 34226A)**

a) Bromure de N-(hexanone-2 yl) méthyl-2 éthoxycarbonyl-4 pyridinium

On dissout 19,5g (0,118 mole) de méthyl-2 éthoxycarbonyl-4 pyridine dans 35g (0,196 mole) de bromo-1 hexanone-2 et on abandonne à température ambiante durant environ 15 heures. On triture la masse solide dans de l'éther diéthylique sec. On filtre, lave convenablement avec le même solvant et sèche sous vide.

De cette manière, on obtient 37,5g de bromure de N-(hexanone-2 yl) méthyl-2 éthoxycarbonyl-4 pyridinium

Rendement : 92%

P.F. : 139-140°C

De la même manière que celle décrite ci-dessus, on a préparé le composé suivant :

Bromure de N-phénylacétyl méthyl-2 éthoxycarbonyl-4 pyridinium

P.F. : 146-147°C (décomposition) (acétate d'éthyle/méthanol/diéthyléther)

b) n-Butyl-2 carbéthoxy-7 indolizine

On dissout 33g (0,101 mole) de bromure de N-(hexanone-2 yl) méthyl-2 éthoxycarbonyl-4 pyridinium dans 300ml d'éthanol absolu et on ajoute, goutte à goutte, une solution de 70ml de triéthylamine dans 300ml d'éthanol absolu chauffée à reflux. Après 2h, on évapore à sec et dissout le résidu dans l'acétate d'éthyle. On lave à l'eau, sèche la couche organique au moyen de sulfate de magnésium et évapore à sec. On purifie alors le produit obtenu sur une colonne de 800g de silice (éluant : hexane/acétate d'éthyle).

De cette manière, on obtient 17,4g de n-butyl-2 carbéthoxy-7 indolizine

Rendement : 70%

P.F. : 47-48°C

De la même manière que précédemment, on a préparé le composé suivant : Phényl-2 carbéthoxy-7 indolizine

P.F. : 143-144°C (méthanol/dichlorométhane)

c) Oxalate acide de n-butyl-2 [(di-n-butylamino-3 propoxy)-4 benzoyl]-3 carbéthoxy-7 indolizine

On chauffe à 95-100°C durant 4,5h, un mélange de 0,35g (38 mmoles) n-butyl-2 carbéthoxy-7 indolizine et 14,2g (38 mmoles) de chlorhydrate de chloro-1 (di-n-butylamino-3 propoxy)-4 benzoyle. On dissout le produit brut obtenu dans de l'acétate d'éthyle et de l'eau et on neutralise par de l'hydrogénocarbonate de sodium. On sépare la couche organique et sèche sur sulfate de magnésium. On filtre et évapore. On purifie alors sur une colonne de silice avec de l'acétate d'éthyle pour obtenir 7,6g de composé désiré sous forme basique (rendement : 97%). On forme alors le sel oxalique dans l'acétate d'éthyle et on le récupère par filtration.

De cette manière, on obtient l'oxalate acide de n-butyl-2 [(di-n-butylamino-3 propoxy)-4 benzoyl]-3 carbéthoxy-7 indolizine

P.F. : 140-142°C (acétate d'éthyle/méthanol)

**EXEMPLE 29**

Préparation du chlorhydrate de n-butyl-2 [(di-n-butylamino-3 propoxy)-4 benzoyl]-3 carboxy-7 indolizine **(SR 34227A)**

On dissout 7,60g (14,02 mmoles) de n-butyl-2[(di-n-butylamino-3 propoxy)-4 benzoyl]-3 carbéthoxy-7 indolizine dans 300ml d'éthanol contenant 60ml d'une solution aqueuse 1M en hydroxyde de sodium. On chauffe à reflux pendant

24h puis on évapore, sous vide, la plupart de l'éthanol. On ajoute 300ml d'eau et acidifie par de l'acide chlorhydrique concentré.. On extrait le précipité par trois fractions chacune de 150ml de dichlorométhane, sèche et évapore les solvants.

De cette manière, on obtient 6,13g de chlorhydrate de n-butyl-2 [(di-n-butylamino-3 propoxy)-4 benzoyl]-3 carboxy-7 indolizine
Rendement : 79%
P.F. : 190-192°C (acétate d'éthyle/méthanol)

**EXEMPLE 30**

Préparation du n-butyl-2[(di-n-butylamino-3 propoxy)-4 benzoyl]-3 benzyloxycarbonylamino-7 indolizine **(SR 34254)**

On dissout 7,10g (13,08 mmoles) de n-butyl-2[(di-n-butylamino-3 propoxy)-4 benzoyl]-3 carboxy-7 indolizine dans 100ml d'acétone sèche contenant 5,05g (50 mmoles) de triéthylamine. On refroidit dans un bain d'eau glacée et on ajoute 1,70g (15,67 mmoles) de chloroformiate d'éthyle. Après 1h, on ajoute une solution de 10g (150 mmoles) d'azide de sodium dans 70ml d'eau. Un précipité jaune se forme immédiatement. On abandonne durant 45 min. puis on verse dans de l'eau. On extrait avec de l'acétate d'éthyle puis on sèche sur sulfate de magnésium et évapore le solvant. On dissout le résidu dans 30ml d'alcool benzylique et on chauffe à 105-110°C pendant 2h. On élimine le solvant par distillation sous vide et on purifie le carbamate brut sur une colonne de silice (éluant : acétate d'éthyle).

De cette manière, on obtient 6,08g de n-butyl-2[(di-n-butylamino-3 propoxy)-4 benzoyl]-3 benzyloxycarbonylamino-7 indolizine
Rendement : 75%
P.F : 118-119C (acétate d'éthyle)

**EXEMPE 31**

Préparation de la n-butyl-2[(di-n-butylamino-3 propoxy)-4 benzoyl]-3 amino-7 indolizine (SR 34399)

On agite, sous une atmosphère d'hydrogène pendant 60h, 6,10g (10 mmoles) de n-butyl-2[(di-n-butylamino-3 propoxy)-4 benzoyl]-3 benzyloxycarbamoyl-7 indolizine et de 10% de palladium sur 0,550g de charbon actif dans 250ml d'éthanol absolu. On filtre sur verre fritté et évapore à sec pour recueillir des cristaux jaunes.

De cette manière, on obtient 4,76g de n-butyl-2[(di-n-butylamino-3 propoxy)-4 benzoyl]-3 amino-7 indolizine
P.F. : 88-89°C (hexane)

**EXEMPLE 32**

Préparation du n-butyl-2[(di-n-butylamino-3 propoxy)-4 benzoyl]-3 méthylsulfonamido-7 indolizine **(SR 34255)**

Sous agitation et à température ambiante, on ajoute 1,65g (4,04 mmoles) de chlorure de méthanesulfonyle dans du dichlorométhane sec, à une solution de 2,85g (5,975 mmoles) de n-butyl-2[(di-n-butylamino-3 propoxy)-4 benzoyl]-3 amino-7 indolizine dans du dichlorométhane contenant 10ml de triéthylamine (excès). On abandonne durant 1h, verse dans de l'eau et extrait par 3 fractions chacune de 75ml de dichlorométhane. On sèche sur sulfate de magnésium et évapore l'extrait pour obtenir 3,47g de dérivé sulfonimide brut. On dissout ensuite ce sulfonimide dans une solution d'hydroxyde de sodium (2,0g; 50 mmoles) dans 250ml d'éthanol absolu à température ambiante. Après 45 min. On dilue avec 1l d'eau et neutralise à pH = 7 par de l'acide chlorhydrique concentré, ce qui forme un précipité. On extrait avec 3 fois 150ml d'acétate d'éthyle, sèche et évapore les extraits. On purifie ensuite sur colonne de silice avec l'acétate d'éthyle comme éluant.

De cette manière, on obtient 2,59g de n-butyl-2[(di-n-butylamino-3 propoxy)-4 benzoyl]-3 méthylsulfonamido-7 indolizine
Rendement : 78%
P.F. : 87-88°C (hexane)

**EXEMPLE 33**

Préparation du n-butyl-2[(di-n-butylamino-3 propoxy)-4 benzoyl]-3 carboxamido-7 indolizine **(SR 34296)**

On chauffe dans un tube scellé durant environ 15h à 100°C, un mélange de 1g (1,87 mmole) de n-butyl-2[(di-n-butylamino-3 propoxy)-4 benzoyl]-3 carbéthoxy-7 indolizine, 5ml d'ammoniaque à 25% et 25ml de méthanol. On évapore à sec et on dissout dans du dichlorométhane. On lave à l'eau, sèche sur sulfate de magnésium et évapore. On purifie sur colonne de silice avec un mélange acétate d'éthyle/méthanol 9/1 puis on recristallise avec de l'hexane/acétate d'éthyle.

De cette manière, on obtient 0,485g de n-butyl-2[(di-n-butylamino-3 propoxy)-4 benzoyl]-3 carboxamido-7 indolizine.
Rendement : 51%
P.F. : 132°C (hexane/acétate d'éthyle)

**EXEMPLE 34**

Préparation de l'oxalate de phényl-21(di-n-butylamino-3 propoxy)-3 benzoyl]-3 carbethoxy-7 indolizine **(SR 34507A)**

a) Phényl-2 (benzyloxy-3 benzoyl)-3 carbéthoxy-7 indolizine

On chauffe à 105-110°C pendant 4h, un mélange de 4g (15,04 mmoles) de phényl-2 carbéthoxy-7 indolizine et 4g (16,22 mmoles) de chlorure de benzyloxy-3 benzoyle. On purifie le produit obtenu sur colonne de silice avec un mélange hexane/acétate d'éthyle 4/1.

De cette manière, on obtient 7,1g de phényl-2 (benzyloxy-3 benzoyl)-3 carbéthoxy-7 indolizine
Rendement : 90%
Produit huileux

De manière analogue à celle décrite précédemment, on a préparé le composé suivant :
Phényl-2 (benzyloxy-4 benzoyl)-3 carbéthoxy-7 indolizine
P.F. : 115-117°C (méthanol/dichlorométhane)

b) Phényl-2 (hydroxy-3 benzoyl)-3 carbéthoxy-7 indolizine

On dissout 6,5g (13,63 mmoles) de phényl-2 (benzyloxy-3 benzoyl)-3 carbéthoxy-7 indolizine dans 300ml d'éthanol absolu contenant 0,300g de charbon palladié à 5% et 15g de formiate d'ammonium. On chauffe à reflux pendant 24h puis on élimine le catalyseur par filtration sur verre fritté et on évapore à sec. On dissout le résidu dans l'acétate d'éthyle, filtre et lave les sels 3 fois avec de l'acétate d'éthyle. On évapore le solvant et purifie le produit brut sur colonne de silice (éluant : hexane/acétate d'éthyle 1/1).

De cette manière, on obtient 4,16g de phényl-2 (hydroxy-3 benzoyl)-3 carbéthoxy-7 indolizine
Rendement : 83%
P.F. : 193-195°C (hexane/acétate d'éthyle)

De manière analogue à celle décrite ci-dessus, on a préparé le composé suivant :
Phényl-2 (hydroxy-4 benzoyl)-3 carbéthoxy-7 indolizine
P.F. : 135°C (acétate d'éthyle/hexane)

c) Phényl-2[(di-n-butylamino-3 propoxy)-3 benzoyl]-3 carbéthoxy-7 indolizine

On dissout 0,387g (1 mmole) de phényl-2 (hydroxy-3 benzoyl)-3 carbéthoxy-7 indolizine et 0,206g (1 mmole) de di-n-butylamino-3 chloro-1 propane dans 3ml de diméthylsulfoxyde sec contenant 0,3g de carbonate de potassium et une quantité catalytique d'iodure de sodium. On maintient sous azote à température ambiante pendant 5 jours puis on verse dans de l'eau et on extrait à l'acétate d'éthyle.
On sèche sur sulfate de magnésium et on évapore les extraits. On purifie alors sur colonne de silice (éluant : acétate d'éthyle)

De cette manière, on obtient 0,503g de phényl-2[(di-n-butylamino-3 propoxy)-3 benzoyl]-3 carbéthoxy-7 indolizine
Rendement : 91%
L'oxalate de ce produit s'est révélé amorphe.

**EXEMPLE 35**

Préparation du {[(di-n-butylamino-3 propoxy)-5 thénoyl]-2}-3 bisméthyl-sulfonamido-5 n-butyl-2 benzofuranne

a) (Bisméthylsulfonamido)-5 n-butyl-2 benzofuranne

A une solution de 14,6g (0,077 mole) d'amino-5 n-butyl-2 benzofuranne dans 500ml de tétrachlorure de carbone contenant 77,9g (0,77 mole) de triéthylamine, on ajoute, goutte à goutte en 1,5h, une solution de 26,45g (0,231 mole)

de chlorure de méthanesulfonyle dans 175ml de tétrachlorure de carbone. La température monte à 30°C. On chauffe alors au reflux pendant 6h. On laisse refroidir, verse sur un mélange d'eau et de glace, évapore la phase organique et extrait la phase aqueuse avec 3 fois 150ml de chlorure de méthylène. On lave les phases organiques 3 fois avec 250ml d'eau, sèche, filtre et évapore à sec sous vide.

De cette manière, on obtient 20,1g de (bisméthylsulfonamido)-5 n-butyl-2 benzofuranne après recristallisation dans l'isopropanol.
Rendement : 75,5%
P.F : 126°C
Pureté (CHLP) : 99,5%

b) [(Bisméthylsulfonamido)-5 (méthoxy-5 thénoyl)-2]-3 n-butyl-2 benzofuranne

A une solution de 6,9g (0,02 mole) de bisméthylsulfonamido)-5 n-butyl-2-benzofuranne dans 40ml de chloroforme, on ajoute en 10 min. et à la température de 24°C, une solution de 7,3g (0,028 mole) de tétrachlorure d'étain dans 10 ml de chloroforme. On ajoute ensuite en 3,5h, une solution de 1,77g (0,01 mole) de chlorure de méthoxy-5 thénoyle dans 15ml de chloroforme. On agite le milieu réactionnel pendant 48h et on verse ensuite dans 60ml d'acide chlorhydrique 2N. On décante et on extrait la phase aqueuse avec 2 fois 50ml d'éther éthylique. On lave les phases organiques avec 40ml d'eau, 40ml d'hydroxyde de sodium 2N, 35ml d'eau puis on sèche sur sulfate de sodium. On filtre et évapore à sec sous vide. On purifie par chromatographie d'élution sur silice avec du chloroforme comme éluant.

De cette manière, on obtient 4,2g de [(bisméthylsulfonamido)-5 (méthoxy-5 thénoyl)-2]-3 N-butyl-2 benzofuranne après recristallisation dans l'éthanol.
Rendement : 43,2%
P.F. : 164-166°C
Pureté (CLHP) : 99,5%

c) [(Bisméthylsulfonamido)-5 (hydroxy-5 thénoyl)-2]-3 n-butyl-2 benzofuranne

A une température comprise entre 15 et 20°C, on ajoute en 10 min. une solution de 17ml (0,017 mole) de tribromure de bore (solution 1M dans le chlorure de méthylène) à une solution de 2,45g (0,005 mole) de [(bisméthylsulfonamido)-5 (méthoxy-5 thénoyl)-2]-3 n-butyl-2 benzofuranne dans 20ml de chloroforme.

On chauffe à reflux pendant 4h puis on verse sur un mélange d'eau et de glace auquel on ajoute ensuite 50ml d'acide chlorhydrique 2N. On filtre le solide formé et on extrait la phase aqueuse avec 2 fois 40ml de chlorure de méthylène. On lave à l'eau, sèche sur sulfate de sodium, filtre et évapore à sec.

De cette manière, on obtient 2g de [(bisméthylsulfonamido)-5 (hydroxy-5 thénoyl)-2]-3 n-butyl-2 benzofuranne sous forme solide.
Rendement : 84,8%
P.F. : 202-204°C
Pureté (CLHP) : 99,6%

d) {[(Di-n-butylamino-3 propoxy)-5 thénoyl]-2]-3 bisméthylsulfonamido-5 n-butyl-2 benzofuranne

Ce composé a été obtenu selon la méthode décrite à l'Exemple 1e.

En utilisant les méthodes exemplifiées précédemment, on a préparé les composés suivants:
Amino-5 [(di-n-butylamino-3 propoxy)-4 benzoyl]-3 isopropyl-2 benzofuranne (Ex. 36)
Huile
Pureté (CLHP) : 97,84%

Hémioxalate acide d'amino-5 [(di-n-butylamino-3 propoxy)-4 benzoyl]-3 phényl-2 benzofuranne (Ex. 37)
P.F. : 104°C (méthanol)
Pureté (CLHP) : 98,2%

Amino-5 n-butyl-2 [(n-butylamino-3 propoxy)-4 benzoyl]-3 benzofuranne (Ex. 38)
Huile
Pureté (CLHP) : 97,83%

Chlorhydrate d'amino-5 [(di-n-butylamino-3 propoxy)-4 diméthyl-3,5 benzoyl]-3 n-butyl-2 benzofuranne (SR 33750 A) (Ex.39)
P.F. : 160-161°C (acétate d'éthyle)

Dioxalate d'amino-5 [(tertiobutylamino-3 propoxy)-4 benzoyl]-3 n-butyl-2 benzofuranne (SR 33667 A) (Ex. 40)
P.F. : 146°C (méthanol)

Dioxalate d'amino-5 {[N-méthyl N-(diméthoxy-3,4 β-phénéthyl) amino-3 propoxy]-4 benzoyl}-3 n-butyl-2 benzofuranne (SR 33665 A) (Ex.41 )
P.F. : 163°C (méthanol)
n-Butylsulfonamido-5 [(di-n-butylamino-3 propoxy)-4 benzoyl]-3 n-butyl-2 benzofuranne (SR 34193) (Ex.42 )
P.F. : 78°C
Fumarate acide de [(di-n-butylamino-3 propoxy)-4 benzoyl]-3 n-butyl-2 tolylsulfonamido-5 benzofuranne (SR 34088 A) (Ex.43 )
P.F. : 110°C (isopropanol)
n-Butyl-2 [(n-butylamino-3 propoxy)-4 benzoyl]-3 méthylsulfonamido-5 benzofuranne (SR 34174) (Ex.44 )
P.F. : 86°C
Oxalate de n-butyl-2 [(di-n-butylamino-3 propoxy)-4 benzoyl]-3 méthylsulfonamido-5 benzofuranne (SR 33589 A) (Ex. 45)
P.F. : 75°C (acétone/hexane)
Chlorhydrate de n-butyl-2 [(di-n-butylamino-3 propoxy)-4 benzoyl]-3 trifluorométhylsulfonamido-5 benzofuranne (SR 34099 A) (Ex.46 )
P.F. : 60°C (éther de pétrole 40-60°C)
Chlorhydrate de n-butyl-2{[N-méthyl N-(diméthoxy-3,4 β-phénéthyl) amino-3 propoxy]-4 benzoyl}-3 méthylsulfonamido-5 benzofuranne (SR 33666 A) (Ex. 47)
P.F. : 120°C (dichlorométhane/éther)
Hémioxalate de n-butyl-2 [(di-n-butylamino-3 propoxy)-4 diméthyl-3,5 benzoyl]-3 méthylsulfonamido-5 benzofuranne (SR 33751 A) (Ex. 48)
P.F . 71°C
Phényl-2 [(di-n-butylamino-3 propoxy)-4 benzoyl]-3 méthylsulfonamido-5 benzofuranne (SR 34150) (Ex. 49)
P.F. : 40°C
p-Toluènesulfonate d'isopropyl-2 [(di-n-butylamino-3 propoxy)-4 benzoyl]-3 méthylsulfonamido-5 benzofuranne (SR 34153 A) (Ex.50 )
P.F. : 128°C (isopropanol)
p-Toluènesulfonate d'isopropyl-2 [(di-n-butylamino-3 propoxy)-4 benzoyl]-3 N,N-bis (méthylsulfonyl) amino-5 benzofuranne (Ex. 51)
P.F. : 142°C (isopropanol)
Dioxalate d'amino-5 [(di-n-butylamino-3 propoxy)-4 benzoyl]-3 méthyl-2 benzofuranne (SR 34287 A) (Ex. 52)
P.F. : 136°C (méthanol)
Pureté (CLHP) : 99,3%
Amino-5 [(di-n-butylamino-3 propoxy)-4 benzoyl]-3 isopropyl-2 benzofuranne (Ex. 53)
Huile
Pureté (CLPH) : 97,84%
Amino-5 [(di-n-butylamino-3 propoxy)-4 benzoyl]-3 phényl-2 benzofuranne (Ex. 54)
Huile
Pureté (CLHP) : 98,2%
Amino-5 [(di-n-butylamino-3 propoxy)-4 benzoyl]-3 n-propyl-2 benzofuranne (Ex. 55)
Dioxalate d'amino-5 [(di-n-butylamino-3 propoxy)-4 benzoyl]-3 éthyl-2 benzofuranne (SR 34521 A) (Ex.56 )
P.F. : 172°C (méthanol)
Pureté (CLHP) : 99,25%
Dichlorhydrate d'amino-5 n-butyl-2 [(diéthylamino-3 propoxy)-4 benzoyl]-3 benzofuranne (SR 34520 A) (Ex.57 )
P.F. : 121,5°C (éthanol)
Pureté (CLHP) : 98,7%
Dichlorhydrate d'amino-7 [(di-n-butylamino-3 propoxy)-4 benzoyl]-3 méthyl-2 benzofuranne (SR 34405 A) (Ex. 58)
P.F. : 160°C
Pureté (CLHP) : 97,5%
Dichlorhydrate d'amino-5 [(di-n-butylamino-3 propoxy)-4 benzoyl] -2 méthyl-3 benzofuranne (Ex. 59) (SR 34433 A)
P.F. : 178°C (isopropanol)
Pureté (CLHP) : 99,2%
Dioxalate d'amino-5 n-butyl-2 [(di-n-butylamino-5 pentoxy)-4 benzoyl]-3 benzofuranne (SR 34486 A) (Ex. 60)
Pureté (CLHP) : 98,91%
P.F. : 95°C (isopropanol)
Amino-5 n-butyl-2 [(n-butylamino-3 propoxy)-4 benzoyl]-3 benzofuranne (Ex. 61)
Huile
Pureté (CLHP) : 97,83%
[(Di-n-butylamino-3 propoxy)-4 benzoyl]-3 méthylsulfonamido-5 phényl-2 benzofuranne (SR 34150) (Ex. 62)

P.F. : 40°C
Pureté (CLHP) : 98,29%

Chlorhydrate de [(di-n-butylamino-3 propoxy)-4 benzoyl]-3 méthyl-2 méthylsulfonamido-5 benzofuranne (SR 34144 A) (Ex. 63)
P.F. : 163°C (éthanol)
Pureté (CLHP) : 99,75%

Toluènesulfonate de [(di-n-butylamino-3 propoxy)-4 benzoyl]-3 isopropyl-2 méthylsulfonamido-5 benzofuranne (SR 34153 A) (Ex. 64)
P.F. : 128°C (isopropanol)
Pureté (CLHP) : 98%

[(Di-n-butylamino-3 propoxy)-4 benzoyl]-3 n-butyl-2 n-butylsulfonamido-5 benzofuranne (SR 34193) (Ex. 65)
P.F. : 78°C
Pureté (CLHP) : 99,82%

Chlorhydrate de n-butyl-2 [(di-n-butylamino-3 propoxy)-4 benzoyl]-3 trifluorométhylsulfonamido-5 benzofuranne (SR 34099 A) (Ex. 66)
P.F. : 50-55°C
Pureté (CLHP) : 99,4%

Fumarate acide de n-butyl-2 [(di-n-butylamino-3 propoxy)-4 benzoyl]-3 tosylamino-5 benzofuranne (SR 34088 A) (Ex. 67)
P.F.: 110°C (isopropanol)
Pureté (CLHP) : 98,24%

n-Butyl-2 [(n-butylamino-3 propoxy)-4 benzoyl]-3 méthylsulfonamido-5 benzofuranne (SR 34174) (Ex. 68)
P.F. : 86,4°C
Pureté (CLHP) : 99,57%.

Oxalate acide de [(di-n-butylamino-3 propoxy)-4 benzoyl]-3 méthyl-2 méthylsulfonamido-7 benzofuranne (SR 34436 A) (Ex. 69)
P.F. : 135°C (méthyl éthyl cétone)
Pureté (CLHP) : 99,1%

[(Di-n-butylamino-3 propoxy)-4 benzoyl]-3 bisméthylsulfonamido-5 n-butyl-2 benzofuranne (SR 34477) (Ex. 70)
P.F. : 56°C (hexane)
Pureté (CLHP) : 99,7%

[(Di-n-butylamino-3 propoxy)-4 benzoyl]-3 bisméthylsulfonamido-5 n-propyl-2 benzofuranne (Ex. 71) (SR 34555)
P.F. : 57°C (pentane). Pureté (CLHP) : 99,8%

[(Diéthylamino-3 propoxy)-4 benzoyl]-3 n-butyl-2 bisméthylsulfonamido-5 benzofuranne (SR 34505) (Ex. 72)
P.F. : 83°C
pureté (CLHP) : 98,9%

p-Toluènesulfonate de [(di-n-butylamino-2 éthoxy)-4 benzoyl]-3 bisméthylsulfonamido-5 n-butyl-2 benzofuranne (Ex. 73)
P.F. : 142°C (acétate d'éthyle)
Pureté (CLHP) : 98,42%

[(Di-n-butylamino-5 pentoxy)-4 benzoyl]-3 bisméthylsulfonamido-5 n-butyl-2 benzofuranne (SR 34343) (Ex. 74)
P.F. : 84°C
Pureté (CLHP) : 97,1%

[(Di-n-butylamino-3 propoxy)-4 benzoyl]-3 bisméthylsulfonamido-7 méthyl-2 benzofuranne (SR 34403) (Ex. 75)
P.F. : 108°C
Pureté (CLHP) : 96,7%

[(Di-n-butylamino-3 propoxy)-4 benzoyl]-2 bisméthylsulfonamido-5 méthyl-3 benzofuranne (SR 33602) (Ex. 76)
P.F. : 125°C (méthanol)
Pureté (CLHP) : 99%

p-Toluènesulfonate de [(di-n-butylamino-3 propoxy)-4 benzoyl] -3 bisméthylsulfonamido-5 isopropyl-2 benzofuranne (SR 34154 A) (Ex. 77)
P.F. : 142°C (isopropanol)
Pureté (CLHP) : 98%

[(Di-n-butylamino-5 pentoxy)-4 benzoyl]-3 n-butyl-2 méthylsulfonamido-5 benzofuranne (SR 34492) (Ex. 78)
P.F. : 55°C
Pureté (CLHP) : 96,21%

Dioxalate de [(di-n-butylamino-3 propoxy)-4 benzoyl]-3 éthyl-2 méthylsulfonamido-5 benzofuranne (SR 34536 A) (Ex. 79)

P.F. : 111°C (acétate d'éthyle)

[(Di-n-butylamino-3 propoxy)-4 benzoyl]-3 n-propyl-2 méthylsulfonamido-5 benzofuranne, oxalate acide (Ex. 80)
P.F. :145 - 147°C

[(Diéthylamino-3 propoxy)-4 benzoyl]-3 n-butyl-2 méthylsulfonamido-5 benzofuranne (SR 34523 A) (Ex. 81)
P.F. : 65°C
Pureté (CLHP) : 96,33%

Chlorhydrate de [(di-n-butylamino-3 propoxy)-4 benzoyl]-2 méthyl -3 méthylsulfonamido-5 benzofuranne (SR 34474 A) (Ex. 82)
P.F. : 90°C
Pureté (CLHP) : 99,6%

p-Toluènesulfonate de [(di-n-butylamino-3 propoxy-4) benzoyl] -3 isopropyl-2 méthylsulfonamido-5 benzofuranne (Ex. 83)
P.F. : 128°C (isopropanol)
Pureté (CLHP) : 99%

Dichlorhydrate d'amino-5 {[N-méthyl N-(diméthoxy-3,4 β -phénéthyl) amino-3 propoxy)-4 benzoyl]}-3 éthyl-2 benzothiophène (SR 34453 A) (Ex. 84)
Rendement : 81,2%
P.F. : 138°C (éther diéthylique)

Chlorhydrate de [(di-n-butylamino-3 propoxy)-4 benzoyl]-3 éthyl-2 méthylsulfonamido-5 benzothiophène (SR 34413 A) (Ex. 85)
P.F. : 94°C

Chlorhydrate de {[N-méthyl N-(diméthoxy-3,4 β -phénéthyl) amino-3 propoxy]-4 benzoyl}-3 ethyl-2 méthylsulfonamido-5 benzothiophène (SR 34463 A) (Ex. 86)
P.F. : environ 110°C (éther diéthylique/tétrahydrofuranne)

Chlorhydrate de [(di-n-butylamino-3 propoxy)-4 benzoyl]-3 n-butyl-2 méthylsulfonamido-5 benzothiophène (SR 34489 A) (Ex. 87)
P.F. : environ 82°C (éther diéthylique)

Sesquioxalate d'amino-5 {[N-méthyl N-(diméthoxy-3,4 β -phénéthyl)-amino-3 propoxy]-4 benzoyl}-2 n-butyl-3 benzothiophène (SR 34340 A) (Ex. 88)
P.F. : environ 120°C (acétate d'éthyle/éthanol 7/3)

Chlorhydrate de [(di-n-butylamino-3 propoxy)-4 benzoyl]-2 n-butyl -3 méthylsulfonamido-5 benzothiophène (SR 34232 A) (Ex. 89)
P.F. : environ 90°C (éther diéthylique)

Oxalate de {[N-méthyl N-(diméthoxy-3,4 β-phénéthyl) amino-3 propoxy]-4 benzoyl}-2 n-butyl-3 méthylsulfonamido-5 benzothiophène (SR 34371 A) (Ex. 90)
P.F. : 136°C (acétate d'éthyle/isopropanol 9/1)

Oxalate d'amino-5 {[N-méthyl N-(diméthoxy-3,4 β-phénéthyl) amino -3 propoxy]-4 benzoyl}-3 n-butyl-2 méthyl-1 indole (SR 34225 A) (Ex. 91)
P.F. : 188°C (éthanol)

Fumarate de {[N-méthyl N-(diméthoxy-3,4 β-phénéthyl) amino-3 propoxy]-4 benzoyl}-3 n-butyl-2 méthyl-1 méthylsulfonamido-5 indole (SR 34291 A) (Ex. 92)
P.F. : 166°C (acétate d'éthyle)

Méthanesulfonate d'amino-5 [(di-n-butylamino-3 propoxy)-4 benzoyl] -3 n-butyl-2 di-n-butylaminopropyl-1 indole (SR 34294 A) (Ex. 93)
Hygroscopigue
P.F. : environ 85°C (éther diéthylique)

[(Di-n-butylamino-3 propoxy)-4 benzoyl]-2 n-butyl-3 nitro-5 indole (SR 34358) (Ex. 94)
P.F. : 80°C (heptane)

Dioxalate de [(di-n-butylamino-3 propoxy)-4 benzoyl]-2 n-butyl-3 di-n-butylaminopropyl-1 nitro-5 indole (SR 34359 A) (Ex. 95)
P.F. : 79°C (isopropanol/éther diéthylique)

Méthanesulfanate de [(di-n-butylamino-3 propoxy)-4 benzoyl]-2 n-butyl-3 méthyl-1 nitro-5 indole (SR 34305 A) (Ex. 96)
P.F. : 115°C (acétate d'éthyle)

Oxalate de [(di-n-butylamino-3 propoxy)-4 benzoyl]-3 éthyl-2 méthyl-1 nitro-4 indole (SR 34532 A) (Ex. 97)
P.F. : environ 84°C.

Oxalate de {[N-méthyl N-(diméthoxy-3.4 β-phénéthyl) amino-3 propoxy]-4 benzoyl}-3 n-butyl-2 méthyl-1 nitro-5 indole (SR 34230 A) (Ex. 98)

P.F. : environ 80°C (isopropanol)

[(Di-n-butylamino-3 propoxy)-4 benzoyl]-3 n-butyl-2 di-n-butylaminopropyl-1 méthanesulfonamido-5 indole (SR 34304 A) (Ex. 99)

P.F. : 81°C (hexane)

[(Di-n-butylamino-3 propoxy)-4 benzoyl]-3 n-butyl-2 di-n-butylaminopropyl-1 trifluorométhylsulfonamido-5 indole (SR 34334) (Ex.100)

P.F. : 142°C (heptane/acétate d'éthyle)

[(Di-n-butylamino-3 propoxy)-4 benzoyl]-3 méthyl-1 phényl-2 méthylsulfonamido-5 indole (SR 34451) (Ex. 101)

P.F. : 116°C (éther diéthylique)

Oxalate d'amino-5 {[N-méthyl N-(diméthoxy-3,4 β-phénéthyl) amino-3 propoxy]-4 benzoyle}-3 n-butyl-2 méthyl-l indole (SR 34225 A) (Ex. 102)

P.F. : 188°C (éthanol)

Dichlorhydrate d'amino-5 [(di-n-butylamino-3 propoxy)-4 benzoyl] -3 méthyl-1 phényl-2 indole (SR 34443 A) (Ex. 103)

P.F. : 154°C (éther diéthylique)

Oxalate d'amino-5 [(di-n-butylamino-3 propoxy)-4 benzoyl]-2 n-butyl-3 méthyl-1 indole (SR 34333 A) (Ex. 104)

P.F. : 110°C (isopropanol)

Trichlorhydrate d'amino-5 5(di-n-butylamino-3 propoxy)-4 benzoyl] -2 n-butyl-3 di-n-butylaminopropyl-1 indole (SR 34449 A) (Ex. 105)

P.F. : 130°C (éther diéthylique)

Fumarate de [(di-n-butylamino-3 propoxy)-4 benzoyl]-2 n-butyl-3 méthyl-l méthylsulfonamido-5 indole (SR 34227 A) (Ex. 106)

P.F. : environ 85°C (éthanol/éther diéthylique)

Oxalate de [(di-n-butylamino-3 propoxy)-4 benzoyl]-3 n-butyl-2 (méthyl-4 benzènesulfonyl)-1 indole (SR 34376 A) (Ex. 107)

P.F. : 98°C (éther éthylique)

Amino-4 [(di-n-butylamino-3 propoxy)-4 benzoyl)-3 éthyl-2 méthyl-1 indole. Oxalate : P.F. : 86°C (éther diéthylique) (Ex. 108).

[(Di-n-butylamino-3 propoxy)-4 benzoyl]-3 éthyl-2 méthyl-1 méthylsulfonamido-4 indole. P.F. : 150°C (acétate d'éthylelisopropanol (Ex. 109)

Oxalate acide de phényl-2 [(di-n-butylamino-3 propoxy)-4 benzoyl] -3 carbéthoxy-7 indolizine (SR 34400 A) (Ex. 110)

P.F.: 148-151°C (acétate d'éthyle/méthanol)

Chlorhydrate de phényl-2 [(di-n-butylamino-3 propoxy-4 benzoyl]-3 carboxy-7 indolizine (SR 34401 A) (Ex. 111)

P.F. : 222-224°C (acétate d'éthyle/méthanol)

Oxalate de phényl-2 [(di-n-butylamino-3 propoxy)-4 benzoyl]-3 benzyloxycarbonylamino-7 indolizine (SR 34402 A) (Ex. 112)

P.F. : 177-178°C (acétate d'éthyle/méthanol/éther diéthylique)

Phényl-2 [(di-n-butylamino-3 propoxy)-4 benzoyl]-3 amino-7 indolizine (SR 34408) (Ex. 113)

P.F. : 102-103°C (hexane)

Chlorhydrate de phényl-2 [(di-n-butylamino-3 propoxy)-4 benzoyl]-3 méthylsulfonamido-7 indolizine (SR 34417 A) (Ex. 114)

P.F. : 190-191 (acétate d'éthyle/méthanol)

n-Butyl-2 [(di-n-butylamino-3 propoxy)-4 benzoyl-3 N-méthylcarbox-amido-7 indolizine (SR 34330) (Ex. 115)

P.F. : 94°C (hexane/dichlorométhane)

Oxalate acide de n-butyl-2 [(di-n-butylamino-3 propoxy)-4 benzoyl] -3 pyrrolidinocarbonyl-7 indolizine (SR 34329 A) (Ex. 116)

P.F.: 143-144°C (acétate d'éthyle/dichlorométhane)

Oxalate de phényl-2 {[N-méthyl N-(diméthoxy-3,4 β-phénéthyl) amino-3 propoxy]-4 benzoyl}-3 carbéthoxy-7 indolizine (SR 34484 A) (Ex. 117)

P.F. : 162°C (méthanol)

Phényl-2 [(di-n-butylamino-3 propoxy)-3 benzoyl]-3 carboxy-7 indolizine (Ex. 118) (SR 34515 A)

P.F. (chlorhydrate) : 210-215°C (acétate d'éthyle/méthanol)

Chlorhydrate de phényl-2 {5N-méthyl N-(diméthoxy-3,4 β-phénéthyl)amino-3 propoxy]-4 benzoyl}-3 carboxy-7 indolizine (Ex. 119) (SR 34506 A)

P.F. 128-130°C (acétate d'éthyle/méthanol)

oxalate de n-butyl-2 [(di-n-butylamino-3 propionamido)-4 benzoyl]-3 méthyl-sulfonamido-5 benzofuranne (Ex. 120) (SR 34335 A)

P.F. : 162°C (méthanol/éther diéthylique)

Oxalate de n-butyl-2 [(di-n-butylamino-3 propoxy)-4 benzoyl]-3 méthylsulfonamido-7 benzofuranne (Ex. 121)

P.F : 81°C (isopropanol/éther diéthylique)

Pureté (CLHP) : 96,1%

Oxalate de n-butyl-2 (di-n-butylamino-3 propoxy)-4 di-tertiobutyl-3,5 benzoyl]-3 méthylsulfonamido-5 benzofuranne (Ex. 122)

Oxalate de phényl-2 (di-n-butylamino-3 propoxy-4 benzoyl]-3 méthylsulfonamido-6 indolizine (Ex. 123) (SR 34203 A)

Poudre jaune amorphe

Chlorhydrate de phényl-2 {[N-méthyl N-(diméthoxy-3,4 β-phénéthyl)amino-3 propoxy]-4 benzoyl}-3 méthylsulfonamido-7 indolizine (Ex. 124) (SR 34552 A)

P.F. : 110°C (méthanol/eau)

Oxalate de phényl-2 [(di-n-butylamino-3 propoxy-4 benzoyl]-3 carbéthoxy-7 indolizine (Ex. 125) (SR 34547 A)

Poudre jaune amorphe

Amino-7 [(di-n-butylamino-3 propoxy-4 benzoyl]-3 n-butyl-2 indolizine (Ex. 126) (SR 34399)

P.F. : 88-89°C (n-hexane)

[(Di-n-butylamino-3 propoxy)-4 benzoyl]-3 n-butyl-2 tolylsulfonamido-7 indolizine (Ex. 127) (SR 34428)

Poudre jaune amorphe

Amino-7 [(N-méthyl N-(diméthoxy-3,4 β-phénétyl)amino-3 propoxy]-4 benzoyl}-3 phényl-2 indolizine (Ex. 128) (SR 34548)

P.F. : 115-117°C (dichlorométhane/méthanol)

Oxalate de n-butyl-2 {[N-méthyl N-diméthoxy-3,4 β-phénéthyl) amino-3 propoxy]-4 benzoyl-3 bisméthylsulfonamido-7 indolizine (Ex. 129) (SR 34509 A)

Poudre amorphe

Sesquioxalate de n-butyl-2 {N-méthyl N-(diméthoxy-3,4 β-phénéthyl)amino-3 propoxy]-4 benzoyl}-3 méthylsulfonamido-7 indolizine (Ex. 130) (SR 34534 A)

Poudre jaune amorphe

Chlorhydrate de phényl-2 [(di-n-butylamino-3 propoxy)-2 benzoyl]-3 carbéthoxy-7 indolizine (Ex. 131) (SR 34550 A)

P.F. : 216-218°C (acétate d'éthyle/méthanol)

Oxalate de phényl-2 [(di-n-butylamino-3 propoxy)-3 benzoyle-3 carbéthoxy-7 indolizine (Ex. 132) (SR 34507 A)

Poudre jaune amorphe

EXEMPLE 133

Selon des techniques pharmaceutiques connues, on a préparé une capsule contenant les ingrédients suivants :

| Ingrédient | mg |
|---|---|
| Composé de l'invention | 100,0 |
| Amidons | 99,5 |
| Silice colloïdale | 0,5 |
| | 200,0 |

## Revendications

**1.** Dérivés de benzofuranne, benzothiophène, indole ou indolizine de formule générale :

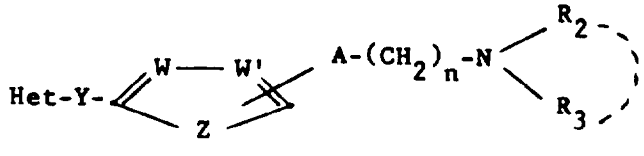

I

dans laquelle :

Het représente l'un des groupements

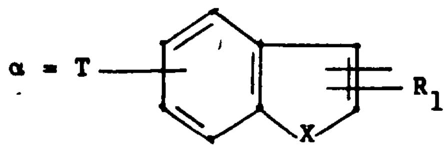

ou

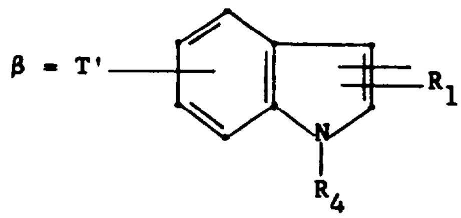

ou

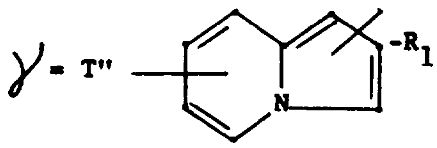

dans lesquels :

T représente un groupement :

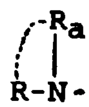

dans lequel :
R et $R_a$, identiques ou différents, représentent :

- l'hydrogène
- un radical alkyle en $C_1$-$C_4$
- un radical -$SO_2R'$ dans lequel R′ représente un radical alkyle, linéaire ou ramifié, en $C_1$-$C_6$, un radical trifluorométhyle, un radical phényle éventuellement substitué par un radical alkyle, en $C_1$-$C_4$, un radical benzyle éventuellement substitué par un radical alkyle en $C_1$-$C_4$ ou un radical benzoyle éventuellement substitué par un radical alkyle en $C_1$-$C_4$, $R_a$ et R′ pouvant former avec l'atome d'azote du groupement sulfonamide, un cycle comportant de 3 à 6 atomes de carbone,

T' représente :

- le groupement nitro
- un groupement

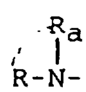

tel que défini précédemment,

T" représente :

- un groupement benzyloxycarbonylamino
- un groupement :

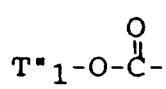

dans lequel T"$_1$ représente l'hydrogène ou un groupement alkyle en $C_1$-$C_4$ - un groupement :

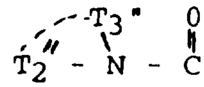

dans lequel T"$_2$ et T"$_3$, identiques ou différents, représentent l'hydrogène ou un groupement alkyle en $C_1$-$C_4$ ou T"2 et T"$_3$ forment avec l'atome d'azote auquel ils sont attachés, un cycle ayant de 4 à 6 atomes de carbone

- un groupement

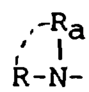

tel que défini précédemment

X représente -O- ou -S-
Y représente un radical

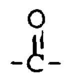, 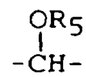

ou $-CH_2-$ dans lequel $R_5$ représente l'hydrogène, un radical alkyle en $C_1$-$C_4$ ou un radical acyle de formule

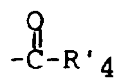

dans lequel $R'_4$ représente un radical alkyle en $C_1$-$C_4$
$R_1$ représente un radical alkyle en $C_1$-$C_6$, phényle éventuellement substitué par un radical alkyle en $C_1$-$C_4$ ou halogénophényle
$R_2$ représente :

- l'hydrogène
- un radical alkyle linéaire ou ramifié en $C_1$-$C_6$

$R_3$ représente :

- un radical alkyle, linéaire ou ramifié en $C_1$-$C_6$
- un radical de formule :

$$Alk\text{-}R_6$$

dans lequel Alk représente une liaison simple ou un radical alkylène, linéaire ou ramifié en $C_1$-$C_5$ et $R_6$ représente un radical pyridyle, phényle, phénoxy, méthylènedioxy-3,4 phényle ou un groupement phényle ou phénoxy substitué par un ou plusieurs substituants, identiques ou différents, sélectionnés parmi des halogènes, des groupements alkyles en $C_1$-$C_4$ ou alkoxy en $C_1$-$C_4$,

$R_2$ et $R_3$, lorsqu'ils sont pris ensemble représentent un radical alkylène ou alkénylène en $C_3$-$C_6$ éventuellement substitué par un radical phényle ou éventuellement interrompu par -0- -NH-, -N= ou $>$N-$R_7$, $R_7$ représentant un radical alkyle en $C_1$-$C_4$ ou phényle,
$R_4$ représente :

- l'hydrogène
- un radical alkyle en $C_1$-$C_4$

- un radical $-SO_2R'_1$ dans lequel $R'_1$ représente un radical alkyle en $C_1$-$C_4$, un radical phényle éventuellement substitué par un radical alkyle en $C_1$-$C_4$ ou un radical benzyle éventuellement substitué par un radical alkyle en $C_1$-$C_4$,
- un radical

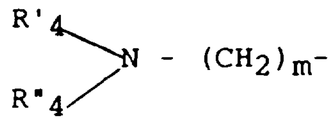

dans lequel $R'_4$ et $R''_4$, identiques ou différents, représentent un radical alkyle en $C_1$-$C_4$ et m représente un nombre de 1 à 3

A représente -0-, -S- ou

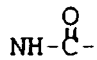

W, W′ et Z sont tels que :

- lorsque W et W′ identiques représentent

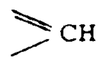

ou N, Z représente -O-
- lorsque W représente

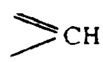

et W′ représente

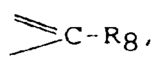

Z représente $-CH=C'-R'_8$
$R_8$ et $R'_8$ étant identiques ou différents et représentant l'hydrogène, un atome d'halogène ou un radical alkyle en $C_1$-$C_4$ ou alkoxy en $C_1$-$C_4$

n représente un nombre de 1 à 5 à condition que lorsque $R_4$ représente un radical $-SO_2R'_1$, T' représente un groupement nitro ou un groupement

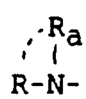

cyclisé, ainsi que leurs sels pharmaceutiquement acceptables.

2. Dérivés selon la Revendication 1 dans laquelle R représente un groupement $-SO_2R'$ et $R_a$ représente l'hydrogène.

3. Dérivés selon la Revendication 1 ou 2 dans laquelle y représente un radical

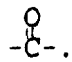

**4.** Dérivés selon une des Revendications 1 à 3 dans laquelle l'entité

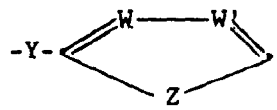

représente un radical benzoyle

**5.** Dérivés selon une des Revendications 1 à 4 dans laquelle l'entité

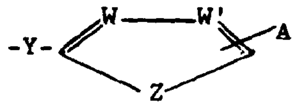

représente un radical benzoyl oxy-4

**6.** Dérivés selon une des Revendications 1 à 5 dans laquelle $R_1$, $R_2$ et $R_3$ représentent un radical n-butyle et n représente 3.

**7.** Dérivés selon une des Revendications 1 à 6 dans laquelle X représente -O-.

**8.** Dérivés selon une des Revendications 1 à 7 dans laquelle la chaine

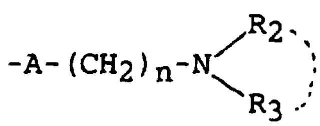

se trouve en position 4.

**9.** Dérivés selon une des Revendications 1 à 8 dans laquelle le sel pharmaceutiquement acceptable est l'oxalate, le fumarate, le chlorhydrate ou le p-toluènesulfonate.

**10.** Dérivés selon la Revendication 1 choisis parmi :

le n-butyl-2[(di-n-butylamino-3 propoxy)-4 benzoyl]-3 méthylsulfonamido-5 benzofuranne,
le n-butyl-2 {[N-méthyl N-(diméthoxy-3,4β -phénéthyl)amino-3 propoxy]- 4 benzoyl}-3 méthylsulfonamido-5 benzofuranne,
l'isopropyl-2[(di-n-butylamino-3 propoxy)-4 benzoyl]-3 méthylsulfonamido-5 benzofuranne,
l'amino-5 [(di-n-butylamino-3 propoxy)-4 benzoyle-3 n-butyl -2 méthyl-1 indole,

et leurs sels pharmaceutiquement acceptables.

**11.** Procédé de préparation de dérivés selon la Revendication 1 dans laquelle Y représente un groupement :

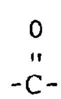

et T ou T' représente un groupement amino, caractérisé en ce que l'on hydrogène un dérivé nitré de formule générale :

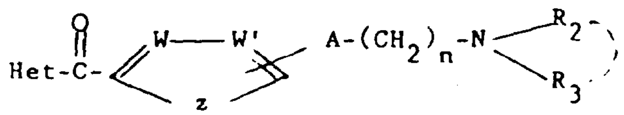

dans laquelle Het représente un groupement

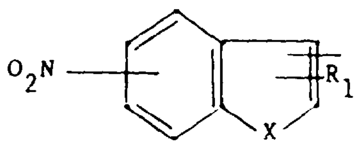

ou

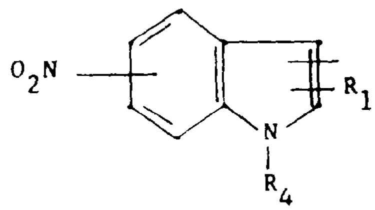

dans lequel $R_1$, $R_2$, $R_3$, $R_4$, A, W, W', X, Y, Z et n ont la même signification que dans la Revendication 1, la réaction ayant lieu en présence d'un catalyseur approprié et dans un solvant polaire ce qui fournit, sous forme de base libre, les composés désirés que l'on fait réagir, si on le désire, avec un acide organique ou inorganique pour former un sel pharmaceutiquement acceptable de ces dérivés.

**12.** Procédé de préparation de dérivés selon la Revendication 1 dans laquelle Y représente un groupement

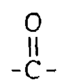

et T, T' ou T" représente un groupement

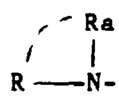

dans lequel R représente un groupement $-SO_2R'$ et $R_a$ représente l'hydrogène ou un groupement $-SO_2R'$, caractérisé en ce que l'on fait réagir un composé de formule I selon la Revendication 1 dans laquelle R et Ra représentent chacun l'hydrogène, avec un ou deux équivalents d'un halogénure de formule générale :

$$Hal\text{-}SO_2\text{-}R'$$

ou d'un anhydride de formule générale :

$$(R'SO_2)_2O$$

dans laquelle R′ a la même signification que dans la Revendication 1 et Hal représente un atome d'halogène, la réaction ayant lieu en présence d'un accepteur d'acide et dans un solvant organique approprié, ce qui fournit, sous forme de base libre, les composés désirés que l'on fait réagir, si on le désire, avec un acide organique ou inorganique pour former un sel pharmaceutiquement acceptable de ces dérivés.

**13.** Procédé de préparation de dérivés selon la Revendication 1 dans laquelle Y représente un groupement

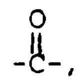

T, T′ ou T″ représente un groupement

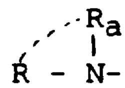

dans lequel R a la valeur indiquée et Ra représente un radical alkyle, caractérisé en ce que l'on fait réagir, en milieu alcalin, un composé de formule I selon la Revendication 1 dans laquelle R a la valeur indiquée et $R_a$ représente l'hydrogène, avec un ou deux équivalents d'un halogénure de formule générale :

$$R'_a\text{-Hal}$$

dans laquelle Hal représente un atome d'halogène et $R'_a$ représente un radical alkyle en $C_1$-$C_4$, ce qui fournit, sous forme de base libre, les composés désirés que l'on fait réagir, si on le désire, avec un acide organique ou inorganique, pour former un sel pharmaceutiquement acceptable de ces dérivés.

**14.** Procédé de préparation de dérivés selon la Revendication 1 dans laquelle Y représente un groupement

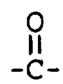

et soit T′ représente un groupement nitro, soit T, T′ ou T″ représente un groupement

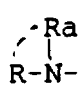

cyclisé, caractérisé en ce que l'on fait réagir, dans un solvant polaire ou apolaire, un composé de formule générale :

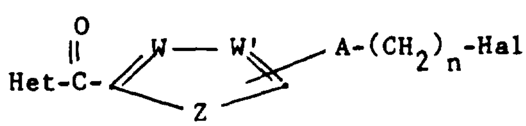

dans laquelle A, W, W′,Z, Hal et n ont la même signification que dans la Revendication 1 et Het représente un groupement de formule générale :

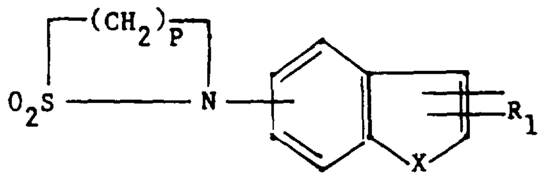

ou

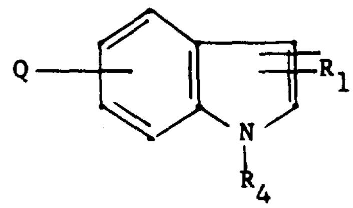

ou

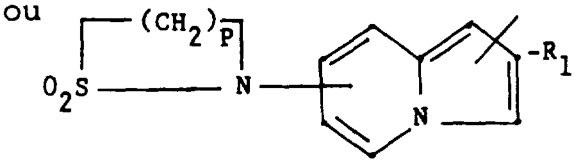

dans laquelle $R_1$, $R_4$ et X ont la même signification que dans la Revendication 1, Q représente un groupement nitro ou

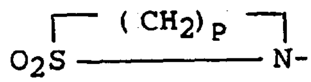

et P représente un nombre de 1 à 4, avec un composé azoté de formule générale :

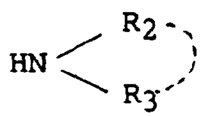

laquelle $R_2$ et $R_3$ ont la même signification que dans la Revendication 1, la réaction ayant lieu en présence d'un agent basique, ce qui fournit, sous forme de base libre, les composés désirés que l'on fait réagir, si on le désire, avec un acide organique ou inorganique pour former un sel pharmaceutiquement acceptable de ces dérivés.

**15.** Procédé de préparation de
Dérivés selon la Revendication 1 dans laquelle T″ représente un groupement carbalkoxy et Y représente un groupement

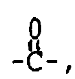

caractérisé en ce que l'on traite à une température de 80 à 110°C, un dérivé d'indolizine de formule générale :

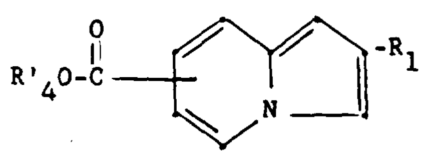

dans laquelle $R_1$ et $R'_4$ ont la même signification que dans la Revendication 1, avec un halogénure de formule générale :

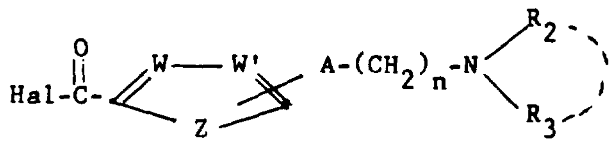

dans laquelle A, $R_2$, $R_3$, W, W′, Z et n ont la même signification que précédemment et Hal représente un atome d'halogène, ce qui fournit, sous forme de base libre, les composés désirés que l'on fait réagir, si on le désire, avec un acide organique ou inorganique pour former un sel pharmaceutiquement acceptable de ces dérivés.

**16.** Procédé de préparation de dérivés selon la Revendication 1 dans laquelle T″ représente un groupement carboxy et Y représente un groupement

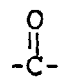 ,

caractérisé en ce que l'on saponifie, un dérivé de carbalkoxy indolizine de formule I selon la Revendication 1 dans laquelle Het représente un groupement de formule générale :

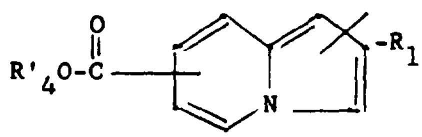

dans laquelle $R_1$ et $R'_4$ ont la même signification que dans la Revendication 1 et ce, au moyen d'un hydroxyde de métal alcalin dans un solvant approprié et à la température de reflux du milieu, ce qui fournit, sous forme de base libre, les composés désirés que l'on fait réagir, si on le désire, avec un acide organique ou inorganique pour former un sel pharmaceutiquement acceptable de ces dérivés.

**17.** Procédé de préparation de dérivés selon la Revendication 1 dans laquelle T″ représente un groupement benzyloxycarbamoyle et Y représente un groupement

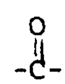

caractérisé en ce que l'on fait réagir dans un solvant approprié et à une température de 0 à +10°C, un dérivé de carboxy-indolizine de formule I selon la Revendication 1 dans laquelle Het représente un groupement de formule générale :

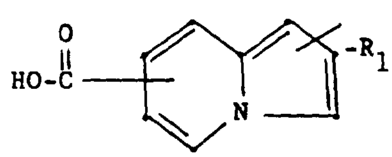

dans laquelle $R_1$ a la même signification que dans la Revendication 1, avec le chloroformiate d'éthyle en présence d'un accepteur d'acide, ensuite avec un azide de métal alcalin et finalement avec l'alcool benzylique, ce qui fournit, sous forme de base libre, les composés désirés que l'on fait réagir, si on le désire, avec un acide organique ou inorganique pour former un sel pharmaceutiquement acceptable de ces dérivés.

**18.** Procédé de préparation de dérivés selon la Revendication 1 dans laquelle T″ représente un groupement amino

et Y représente un groupement

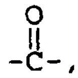

caractérisé en ce que l'on hydrogène dans un solvant approprié et en présence d'un catalyseur approprié, un dérivé de benzyloxycarbamoyl-indolizine de formule I selon la Revendication 1 dans laquelle Het représente un groupement de formule :

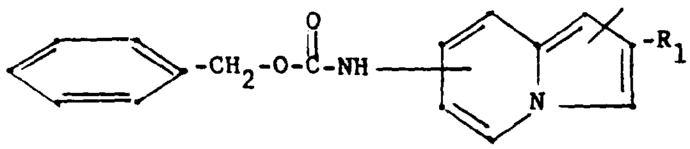

dans laquelle $R_1$ a la même signification que dans la Revendication 1, ce qui fournit, sous forme de base libre, les composés désirés que l'on fait réagir, si on le désire, avec acide organique ou inorganique pour former un sel pharmaceutiquement acceptable de ces dérivés.

**19.** Procédé de préparation de dérivés selon la Revendication 1 dans laquelle Y représente un groupement

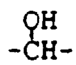

caractérisé en ce que l'on réduit au moyen d'un borohydrure de métal alcalin et dans un solvant approprié, un composé de formule I selon la Revendication 1 dans laquelle Y représente un groupement

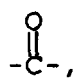

ce qui fournit, sous forme de base libre, les composés désirés que l'on fait réagir, si on le désire, avec un acide organique ou inorganique, pour former un sel pharmaceutiquement acceptable de ces dérivés.

**20.** Procédé de préparation de dérivés selon la Revendication 1 dans laquelle Y représente un groupement

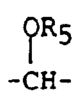

dans laquelle $R_5$ représente un radical $R'_4$ ou un radical acyle de formule

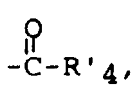

caractérisé en ce que l'on fait réagir un composé de formule I selon la Revendication 1 dans laquelle Y représente un groupement

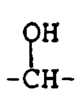 :

soit avec un alcoolate de métal alcalin, puis avec un halogénure de formule générale :

$R'_4$-Hal

dans laquelle $R'_4$ a la même valeur que dans la Revendication 1 et Hal représente un atome d'halogène, soit avec un halogénure de formule générale :

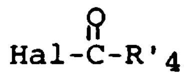

dans laquelle Hal et $R'_4$ ont la même signification que précédemment, la réaction ayant lieu en présence d'un accepteur d'acide ce qui fournit les composés désirés, sous forme de bases libres, que l'on fait réagir, si on le désire, avec un acide organique ou inorganique pour former un sel pharmaceutiquement acceptable de ces dérivés.

**21.** Procédé de préparation de dérivés selon la Revendication 1, dans laquelle Y représente un groupement $-CH_2-$, caractérisé en ce que l'on réduit au moyen d'un borohydrure de métal alcalin, en présence d'acide trifluoroacétique et dans un solvant approprié, un composé de formule I selon la Revendication 1 dans laquelle Y représente un groupement

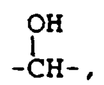

ce qui fournit, sous forme de base libre les composés désirés que l'on fait réagir, si on le désire, avec un acide organique ou inorganique, pour former un sel pharmaceutiquement acceptable de ces dérivés.

**22.** Procédé de préparation de dérivés selon la Revendication 1 dans laquelle T représente un groupement nitro et Y représente un groupement

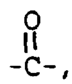

caractérisé en ce que l'on condense, en présence d'un acide de Lewis, un halogénure d'acyle de formule générale :

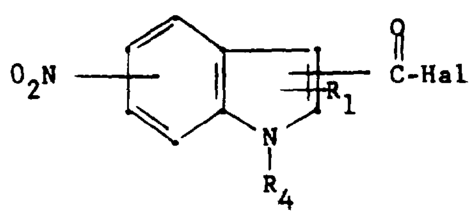

dans laquelle $R_1$ et $R_4$ ont la même signification que dans la Revendication 1 et Hal représente un atome d'halogène avec une amine de formule générale :

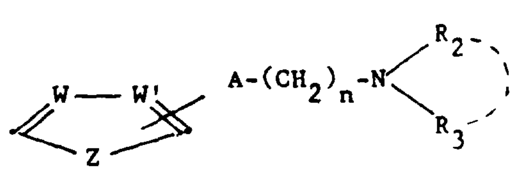

dans laquelle A, $R_2$, $R_3$, W, W′, Z et n ont la même signification que dans la Revendication 1, ce qui fournit sous forme de base libre, les composés désirés que l'on fait réagir, si on le désire, avec un acide organique ou inorganique pour former un sel pharmaceutiquement acceptable.

**23.** Procédé de préparation de dérivés selon la Revendication 1 dans laquelle T, T′ ou T″ représente un groupement

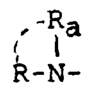

cyclisé, Y représente un groupement

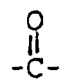

et A représente -0-, caractérisé en ce que l'on condense à une température de 90 à 110°C et dans un solvant approprié, une cétone de formule générale :

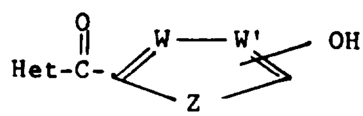

dans laquelle W, W′ et Z ont la même signification que dans la Revendication 1 et Het représente un groupement de formule générale :

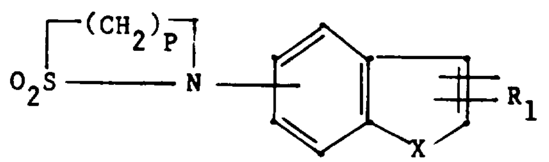

ou

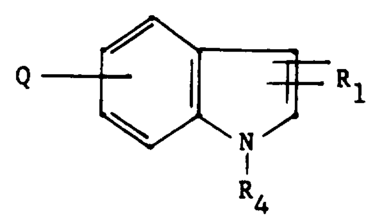

ou

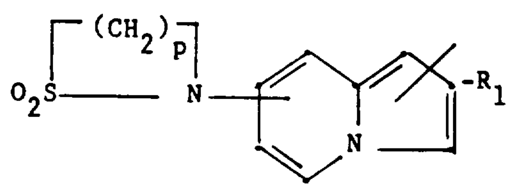

dans laquelle Q, $R_1$, $R_4$, X et p ont la même signification que dans la Revendication 1, avec un composé de formule générale :

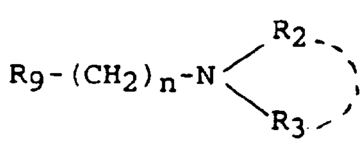

dans laquelle $R_2$, $R_3$ et n ont la même signification que précédemment et $R_9$ représente un atome d'halogène, un radical alkylsulfonyloxy en $C_1$-$C_4$ ou arylsulfonyloxy en $C_6$-$C_{10}$, ce qui fournit, sous forme de base libre, les composés désirés que l'on fait réagir, si on le désire, avec un acide organique ou inorganique pour former un sel pharmaceutiquement acceptable.

**24.** Procédé de préparation de dérivés selon la Revendication 1 dans laquelle T' représente l'hydrogène, un groupement nitro ou un groupement

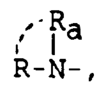

$R_4$ représente un groupement $-SO_2R'$ et Y représente un groupement

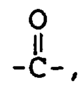

caractérisé en ce que l'on traite, dans un solvant approprié et en présence d' un agent basique, un dérivé d'indole de formule générale :

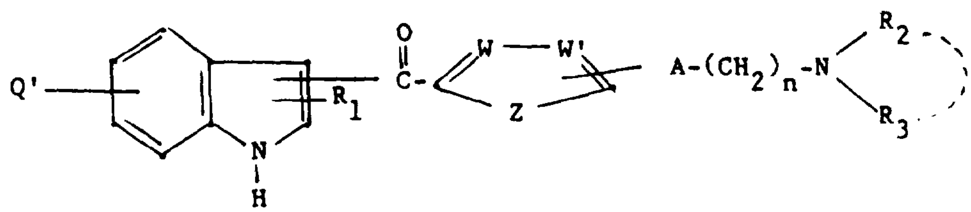

dans laquelle A, $R_1$, $R_2$, $R_3$, W, W′, Z et n ont la même signification que dans la Revendication 1 et Q′ représente l'hydrogène, un groupement nitro ou un groupement

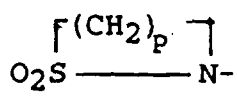

dans lequel p représente un nombre de 1 à 4, avec un halogénure de formule générale :

$$Hal\text{-}SO_2R'$$

ou un anhydride de formule générale :

$$(R'SO_2)_2O$$

dans laquelle R a la même signification que dans la Revendication 1 et Hal représente un atome d'halogène, ce qui fournit sous forme de base libre les composés désirés que l'on fait réagir, si on le désire, avec un acide organique ou inorganique pour former un sel pharmaceutiquement acceptable.

**25.** Procédé de préparation de dérivés selon la Revendication 1 dans laquelle T′ représente un groupement nitro, Y représente un groupement

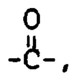

A représente -O-, $R_2$, $R_3$, $R_4$ et $R'_4$ sont identiques et m et n sont identiques, caractérisé en ce que l'on fait réagir, en présence d'un agent basique, un dérivé cétonique de formule générale :

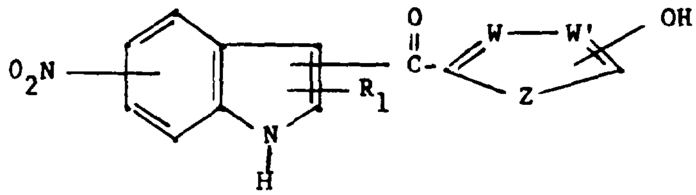

dans laquelle $R_1$, W, W′ et Z ont la même signification que dans la Revendication 1, avec un composé de formule générale :

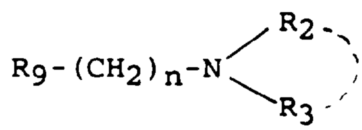

dans laquelle $R_2$, $R_3$ et n ont la même signification que dans la Revendication 1 et $R_9$ représente un atome d'halogène, un radical alkylsulfonyloxy en $C_1$-$C_4$ ou arylsulfonyloxy en $C_6$-$C_{10}$ ce qui fournit, sous forme de base libre, les composés désirés que l'on fait réagir, si on le désire, avec un acide organique ou inorganique pour former un sel pharmaceutiquement acceptable.

**26.** Procédé de préparation de dérivés selon la Revendication 1 dans laquelle T, T' ou T″ représente un groupement -$NHSO_2R'$, caractérisé en ce que l'on traite, dans un solvant approprié, un composé de formule générale :

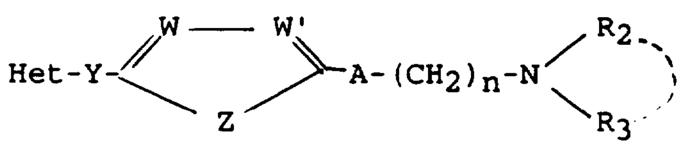

dans laquelle $R_2$, $R_3$, W, W′, Y, Z, A et n ont la même signification que précédemment et Het représente un groupement de formule générale

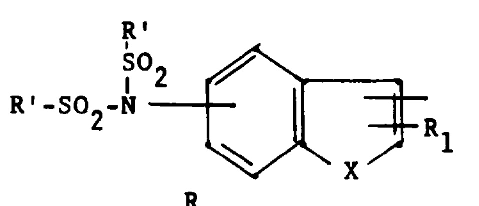

ou

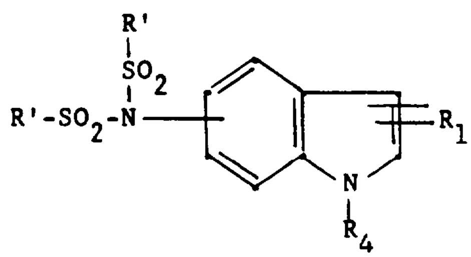

ou

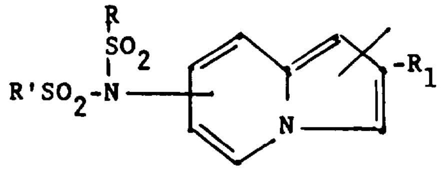

dans laquelle R', $R_1$, $R_4$ et X ont la même signification que dans la Revendication 1, avec un hydroxyde de métal alcalin, ce qui fournit les composés désirés que l'on fait réagir, si on le désire, avec un acide organique ou inorganique pour former un sel pharmaceutiquement acceptable.

**27.** Procédé de préparation de dérivés selon la Revendication 1 dans laquelle T ou T' représente un groupement

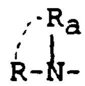

dans lequel R et $R_a$ sont identiques et représentent chacun un groupement $-SO_2R'$, Y représente un groupement

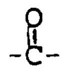

et A représente un groupement -O-, caractérisé en ce que l'on fait réagir, en présence d'un agent basique et à la température de reflux du milieu, un composé de formule générale :

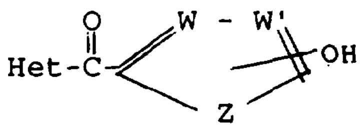

dans laquelle W, W′ et Z ont la même signification que dans la Revendication 1 et Het représente un groupement de formule générale :

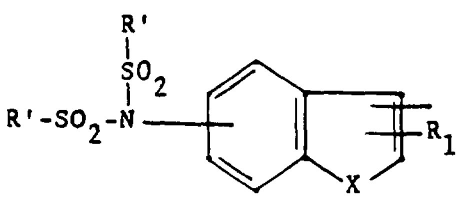

ou

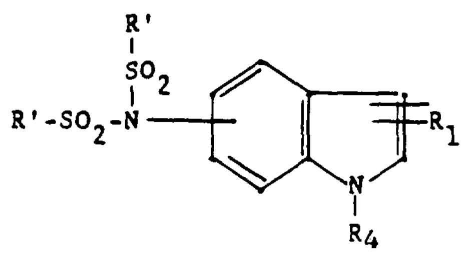

dans laquelle R', $R_1$, $R_4$ et X ont la même signification que dans la Revendication 1, avec un composé de formule générale :

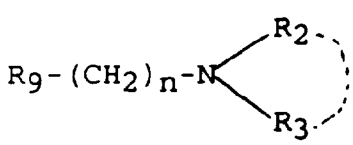

dans laquelle $R_2$, $R_3$ et n ont la même signification que dans la Revendication 1 et $R_9$ représente un atome d'halogène, un radical alkylsulfonyloxy en $C_1$-$C_4$ ou arylsulfonyloxy en $C_6$-$C_{10}$ ce qui fournit sous forme de base libre, les composés désirés que l'on fait réagir, si on le désire, avec un acide organique ou inorganique pour former un sel pharmaceutiquement acceptable.

**28.** Compositions pharmaceutiques ou vétérinaires contenant comme principe actif, au moins un dérivé de benzofuranne, benzothiophène, indole ou indolizine selon l'une des Revendications 1 à 9, en association avec un véhicule pharmaceutique ou un excipient approprié.

**29.** Compositions pharmaceutiques ou vétérinaires contenant comme principe actif au moins un dérivé selon la Revendication 10, en association avec un véhicule pharmaceutique ou un excipient approprié.

**30.** Compositions pharmaceutiques ou vétérinaires selon l'une des Revendications 28 ou 29 pour le traitement de syndromes pathologiques du système cardiovasculaire contenant de 50 à 500 mg de principe actif.

**31.** Utilisation d'au moins un dérivé de benzofuranne, benzothiophène, indole ou indolizine selon l'une des Revendications 1 à 10 pour la fabrication d'un médicament destiné au traitement de syndromes pathologiques du système cardiovasculaire.

## Patentansprüche

**1.** Benzofuran-, Benzothiophen-, Indol- oder Indolizin-Derivate der allgemeinen Formel:

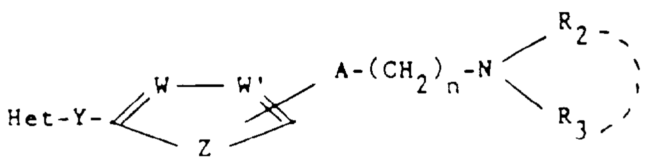

I

in der:
Het eine der Gruppen

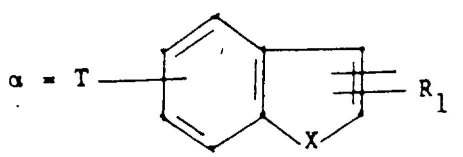

oder

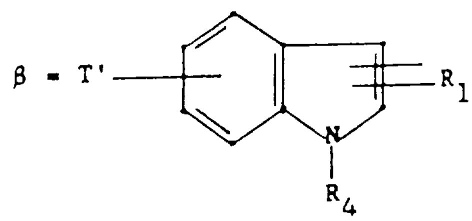

oder

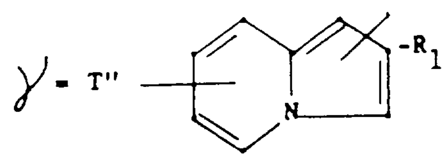

darstellt, in denen:

T eine Gruppe:

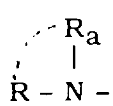

darstellt,
in der:
R und $R_a$, die gleichartig oder verschieden sind:

- Wasserstoff,
- eine $C_1$-$C_4$-Alkylgruppe,
- eine -$SO_2R'$-Gruppe, worin R' eine geradkettige oder verzweigte $C_1$-$C_6$-Alkylgruppe, eine Trifluormethylgruppe, eine gegebenenfalls durch eine $C_1$-$C_4$-Alkylgruppe substituierte Phenylgruppe, eine gegebenenfalls durch eine $C_1$-$C_4$-Alkylgruppe substituierte Benzylgruppe oder eine gegebenenfalls durch eine $C_1$-$C_4$-Alkylgruppe substituierte Benzoylgruppe darstellt, bedeuten, wobei $R_a$ und R' zusammen mit dem

Stickstoffatom der Sulfonamidgruppe einen Ring mit 3 bis 6 Kohlenstoffatomen bilden können,

T':

- die Nitrogruppe,
- eine Gruppe

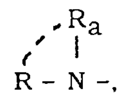

wie sie oben definiert ist, darstellt,

T":

- eine Benzyloxycarbonylaminogruppe,
- eine Gruppe:

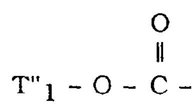

in der $T"_1$ Wasserstoff oder eine $C_1$-$C_4$-Alkylgruppe darstellt,
- eine Gruppe:

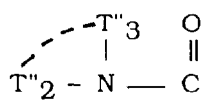

in der $T"_2$ und $T"_3$, die gleichartig oder verschieden sind, Wasserstoff oder eine $C_1$-$C_4$-Alkylgruppe darstellen oder $T"_2$ und $T"_3$ mit dem Stickstoffatom, an das sie gebunden sind, einen Ring mit 4 bis 6 Kohlenstoffatomen bilden,
- eine Gruppe

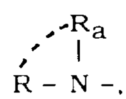

wie sie oben definiert ist, bedeutet,

X -O- oder -S- darstellte,
Y eine Gruppe

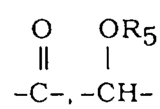

oder $-CH_2-$ darstellt, in der $R_5$ Wasserstoff, eine $C_1$-$C_4$-Alkylgruppe oder eine Acylgruppe der Formel

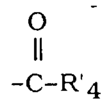

darstellt, in der $R'_4$ eine $C_1$-$C_4$-Alkylgruppe bedeutet,
$R_1$ eine $C_1$-$C_6$-Alkylgruppe, eine gegebenenfalls durch eine $C_1$-$C_4$-Gruppe substituierte Phenylgruppe oder eine Halogenphenylgruppe darstellt,
$R_2$:

- Wasserstoff,
- eine geradkettige oder verzweigte $C_1$-$C_6$-Alkylgruppe darstellt,

$R_3$:

- eine geradkettige oder verzweigte $C_1$-$C_6$-Alkylgruppe,
- eine Gruppe der Formel: Alk-$R_6$ darstellt,
  in der Alk eine Einfachbindung oder eine geradkettige oder verzweigte $C_1$-$C_5$-Alkylengruppe und $R_6$ eine Pyridyl-, Phenyl-, Phenoxy-, 3,4-Methylendioxy-phenyl-gruppe oder eine Phenyl- oder Phenoxy-gruppe, die durch einen oder mehrere gleichartige oder verschiedene Substituenten ausgewählt aus Halogenatomen, $C_1$-$C_4$-Alkylgruppen oder $C_1$-$C_4$-Alkoxygruppen substituiert ist, bedeuten,

$R_2$ und $R_3$, wenn sie gemeinsam eine Gruppe bilden, eine $C_3$-$C_6$-Alkylen- oder -Alkenylen-gruppe darstellen, die gegebenenfalls durch eine Phenylgruppe substituiert oder durch -O-, -NH-, -N= oder >N-$R_7$, worin $R_7$ eine $C_1$-$C_4$-Alkylgruppe oder eine Phenylgruppe darstellt, unterbrochen ist,
$R_4$:

- Wasserstoff
- eine $C_1$-$C_4$-Alkylgruppe,
- eine Gruppe -$SO_2R'_1$, worin $R'_1$ eine $C_1$-$C_4$-Alkylgrupper eine gegebenenfalls durch eine $C_1$-$C_4$-Alkylgruppe substituierte Phenylgruppe oder eine gegebenenfalls durch eine $C_1$-$C_4$-Alkylgruppe substituierte Benzylgruppe darstellt,
- eine Gruppe

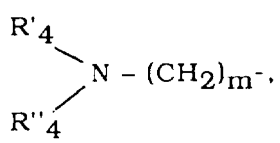

  in der $R'_4$ und $R''_4$, die gleichartig oder verschieden sind, eine $C_1$-$C_4$-Alkylgruppe und m eine Zahl mit einem Wert von 1 bis 3 darstellen, bedeutet,

A -O-, -S- oder

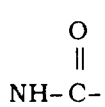

bedeutet,
W, W' und Z solche Bedeutungen besitzen, daß:

- wenn W und W' identisch sind und eine Gruppe

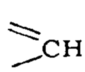

oder N darstellen, Z -O- bedeutet,

- wenn W

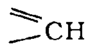 und W' 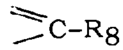

bedeuten, Z -CH=C'-R'$_8$ darstellt, worin $R_8$ und R'$_8$ gleichartig oder verschieden sind und Wasserstoff, ein Halogenatom oder eine $C_1$-$C_4$-Alkylgruppe oder eine $C_1$-$C_4$-Alkoxygruppe darstellen,

n eine Zahl mit einem Wert von 1 bis 5 mit der Maßgabe bedeutet, daß wenn $R_4$ eine Gruppe -$SO_2$R'$_1$ darstellt, T' eine Nitrogruppe oder eine cyclisierte Gruppe

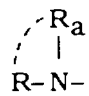

darstellt, sowie deren pharmazeutisch annehmbare Salze.

**2.** Derivate nach Anspruch 1, worin R eine Gruppe -$SO_2$R' und $R_a$ Wasserstoff bedeuten.

**3.** Derivate nach Anspruch 1 oder 2, worin Y eine Gruppe

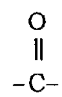

darstellt.

**4.** Derivate nach einem der Ansprüche 1 bis 3, worin die Einheit

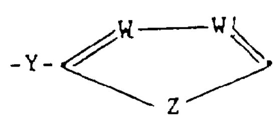

eine Benzoylgruppe bedeutet.

**5.** Derivate nach einem der Ansprüche 1 bis 4, worin die Einheit

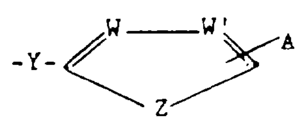

eine Benzoyl-4-oxy-gruppe derstellt.

**6.** Derivate nach einem der Ansprüche 1 bis 5, worin $R_1$, $R_2$ und $R_3$ eine n-Butylgruppe und n 3 bedeuten.

**7.** Derivate nach einem der Ansprüche 1 bis 6, worin X -O- bedeutet.

**8.** Derivate nach einem der Ansprüche 1 bis 7, worin die Kette

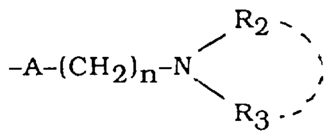

in der 4-Stellung steht.

**9.** Derivate nach einem der Ansprüche 1 bis 8, worin das pharmazeutisch annehmbare Salz das Oxalat, das Fumarat, das Hydrochlorid oder das p-Toluolsulfonat ist.

**10.** Derivate nach Anspruch 1, ausgewählt aus:

2-n-Butyl-3-[4-(3-di-n-butylamino-propoxy]-benzoyl]-5-methylsulfonamidobenzofuran,
2-n-Butyl-3-{4-[3-N-methyl-N-(3,4-dimethoxy-β-phenethyl)-amino-propoxy]-benzoyl}-5-methylsulfonamido-benzofuran,
2-Isopropyl-3-[4-(3-di-n-butylamino-propoxy)-benzoyl]-5-methylsulfonamidobenzofuran,
5-Amino-3-[4-(3-di-n-butylamino-propoxy)-benzoyl]-2-n-butyl-1-methyl-indol und deren pharmazeutisch annehmbare Salze.

**11.** Verfahren zur Herstellung der Derivate nach Anspruch 1, worin Y eine Gruppe

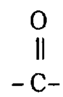

und T oder T' eine Aminogruppe bedeuten, **dadurch gekennzeichnet**, daß man ein Nitroderivat der allgemeinen Formel:

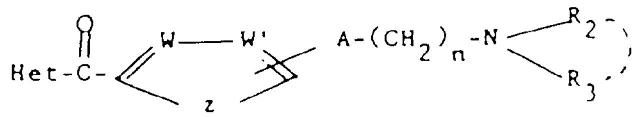

in der Het eine Gruppe:

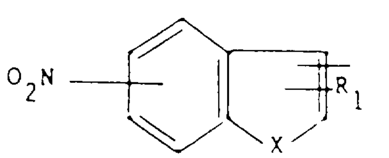

oder

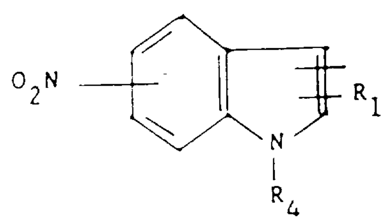

darstellt, in denen $R_1$, $R_2$, $R_3$, $R_4$, A, W, W', X, Y, Z und n die in Anspruch 1 angegebenen Bedeutungen besitzen, hydriert, wobei man die Reaktion in Gegenwart eines geeigneten Katalysators und in einem polaren Lösungsmittel durchgeführt wird, was die gewünschten Verbindungen in Form der freien Base liefert, welche man gewünschtenfalls mit einer organischen oder anorganischen Säure umsetzen kann zur Bildung eines pharmazeutisch annehmbaren Salzes dieser Derivate.

**12.** Verfahren zur Herstellung der Derivate nach Anspruch 1, in denen Y eine Gruppe

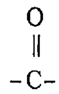

und T, T' oder T " eine Gruppe

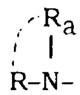

darstellen, worin R eine Gruppe -$SO_2R'$ und $R_a$ Wasserstoff oder eine Gruppe -$SO_2R'$ bedeuten, **dadurch gekennzeichnet,** daß man eine Verbindung der Formel I nach Anspruch 1, worin R und $R_a$ jeweils Wasserstoff bedeuten, mit ein oder zwei Äquivalenten eines Halogenids der allgemeinen Formel:

$$Hal\text{-}SO_2\text{-}R'$$

oder einem Anhydrid der allgemeinen Formel:

$$(R'SO_2)_2O$$

in denen R' die in Anspruch 1 angegebenen Bedeutungen besitzt und Hal ein Halogenatom darstellt, umsetzt, wobei die Reaktion in Gegenwart eines Säureakzeptors und eines geeigneten organischen Lösungsmittels durchgeführt wird, was die gewünschten Verbindungen in Form der freien Base liefert, welche man gewünschtenfalls mit einer organischen oder anorganischen Säure umsetzt zur Bildung eines pharmazeutisch annehmbaren Salzes dieser Derivate.

**13.** Verfahren zur Herstellung der Derivate nach Anspruch 1, bei denen Y eine Gruppe

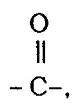

T, T' oder T" eine Gruppe

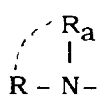

bedeuten, worin R die angegebene Bedeutung besitzt und $R_a$ eine Alkylgruppe darstellt, **dadurch gekennzeichnet,** daß man eine Verbindung der Formel I nach Anspruch 1, in der R die angegebenen Bedeutungen besitzt und $R_a$ Wasserstoff bedeutet, in alkalischem Medium mit ein oder zwei Äquivalenten eines Halogenids der allgemeinen Formel:

$$R'_a\text{-}Hal$$

in der Hal ein Halogenatom und $R'_a$ eine $C_1$-$C_4$-Alkylgruppe bedeuten, umsetzt, was die gewünschten Verbindungen in Form der freien Base liefert, welche man gewünschtenfalls mit einer organischen oder anorganischen Säure zur Bildung eines pharmazeutisch annehmbaren Salzes dieser Derivate umsetzt.

**14.** Verfahren zur Herstellung der Derivate nach Anspruch 1, in denen Y eine Gruppe

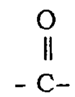

und entweder T' eine Nitrogruppe oder T, T' oder T" eine cyclisierte Gruppe

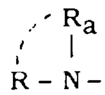

bedeuten, **dadurch gekennzeichnet,** daß man eine Verbindung der allgemeinen Formel:

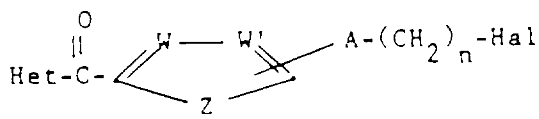

in der A, W, W', Z, Hal und n die in Anspruch 1 angegebenen Bedeutungen besitzen und Het eine Gruppe der allgemeinen Formel:

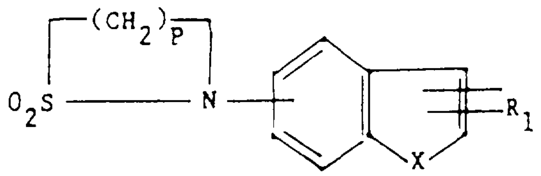

oder

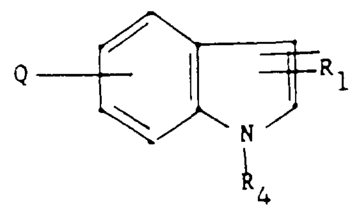

oder

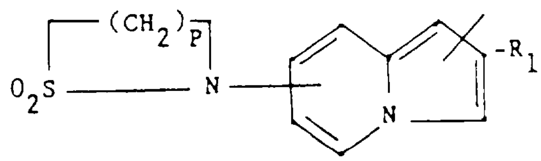

darstellt, worin $R_1$, $R_4$ und X die in Anspruch 1 angegebenen Bedeutungen besitzen, Q eine Nitrogruppe oder eine Gruppe

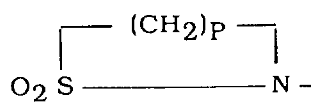

und P eine Zahl mit einem Wert von 1 bis 4 darstellen, in einem polaren oder apolaren Lösungsmittel mit einer Stickstoffverbindung der allgemeinen Formel:

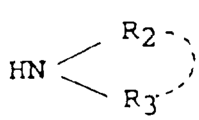

in der $R_2$ und $R_3$ die in Anspruch 1 angegebenen Bedeutungen besitzen, umsetzt, wobei die Reaktion in Gegenwart eines basischen Mittels durchgeführt wird, was die gewünschten Verbindungen in Form der freien Base liefert, welche man gewünschtenfalls mit einer organischen oder anorganischen Säure zur Bildung eines pharmazeutisch annehmbaren Salzes dieser Derivate umsetzen kann.

**15.** Verfahren zur Herstellung der Derivate nach Anspruch 1, in denen T" eine Carbalkoxygruppe und Y eine Gruppe

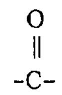

bedeuten, **dadurch gekennzeichnet,** daß man ein Indolizinderivat der allgemeinen Formel:

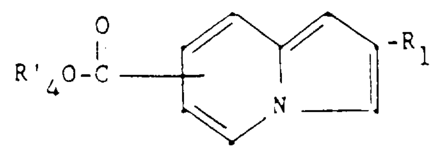

in der $R_1$ und $R'_4$ die in Anspruch 1 angegebenen Bedeutungen besitzen, bei einer Temperatur von 80 bis 110°C mit einem Halogenid der allgemeinen Formel:

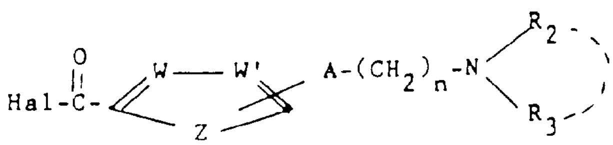

in der A, $R_2$, $R_3$, W, W', Z und n die oben angegebenen Bedeutungen besitzen und Hal ein Halogenatom darstellt, umsetzt, was die gewünschten Verbindungen in Form der freien Base liefert, die man gewünschtenfalls mit einer organischen oder anorganischen Säure zur Bildung eines pharmazeutisch annehmbaren Salzes dieser Derivate umsetzt.

**16.** Verfahren zur Herstellung der Derivate nach Anspruch 1, in denen T " eine Carboxygruppe und Y eine Gruppe

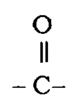

bedeuten, **dadurch gekennzeichnet,** daß man ein Carbalkoxy-indolizin-Derivat der Formel I nach Anspruch 1, in der Het eine Gruppe der allgemeinen Formel darstellt:

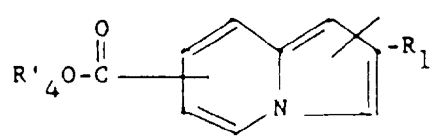

in der $R_1$ und $R'_4$ die in Anspruch 1 angegebenen Bedeutungen besitzen, mit einem Alkalimetallhydroxid in einem geeigneten Lösungsmittel und bei der Rückflußtemperatur des Mediums verseift, so daß man die gewünschten Verbindungen in Form der freien Base erhält, die man gewünschtenfalls mit einer organischen oder anorganischen Säure umsetzt zur Bildung eines pharmazeutisch annehmbaren Salzes dieser Derivate.

**17.** Verfahren zur Herstellung der Derivate nach Anspruch 1, worin T " eine Benzyloxycarbamoylgruppe und Y eine Gruppe

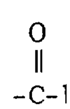

bedeuten, **dadurch gekennzeichnet,** daß man ein Carboxy-indolizin-Derivat der Formel I nach Anspruch 1, in der Het eine Gruppe der allgemeinen Formel darstellt:

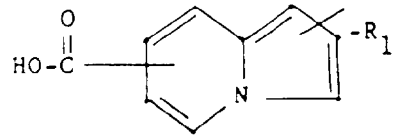

in der $R_1$ die in Anspruch 1 angegebenen Bedeutungen besitzt, in einem geeigneten Lösungsmittel und bei einer Temperatur von 0 bis +10°C mit Chlorameisensäureethylester in Gegenwart eines Säureakzeptors umsetzt und anschließend mit einem Alkalimetallazid und schließlich mit Benzylalkohol, so daß man die gewünschten Verbindungen in Form der freien Base erhält, welche man gewünschtenfalls mit einer organischen oder anorganischen Säure umsetzen kann zur Bildung eines pharmazeutisch annehmbaren Salzes dieser Derivate.

**18.** Verfahren zur Herstellung der Derivate nach Anspruch 1, in denen T " eine Aminogruppe und Y eine Gruppe

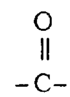

bedeuten, **dadurch gekennzeichnet,** daß man ein Benzyloxycarbamoyl-indolizin-Derivat der Formel I nach Anspruch 1, in der Het eine Gruppe der Formel darstellt:

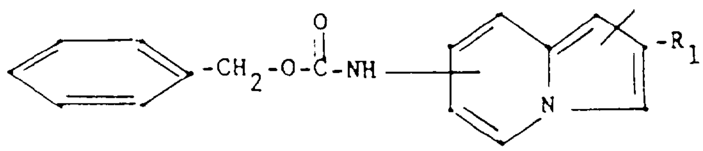

in der $R_1$ die in Anspruch 1 angegebenen Bedeutungen besitzt, in einem geeigneten Lösungsmittel und in Gegenwart eines geeigneten Katalysators hydriert, so daß man die gewünschten Verbindungen in Form der freien Base erhält, welche man gewünschtenfalls mit einer organischen oder anorganischen Säure umsetzen kann zur Bildung eines pharmazeutisch annehmbaren Salzes dieser Derivate.

**19.** Verfahren zur Herstellung der Derivate nach Anspruch 1, worin Y eine Gruppe

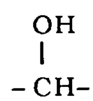

darstellt, **dadurch gekennzeichnet,** daß man eine Verbindung der Formel I nach Anspruch 1, in der Y eine Gruppe

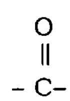

darstellt, in einem geeigneten Lösungsmittel mit einem Alkalimetallborhydrid reduziert, so daß man die gewünschten Verbindungen in Form der freien Base erhält, welche man gewünschtenfalls mit einer organischen oder anorganischen Säure umsetzen kann zur Bildung eines pharmazeutisch annehmbaren Salzes dieser Derivate.

**20.** Verfahren zur Herstellung der Derivate nach Anspruch 1, in denen Y eine Gruppe.

$$\begin{array}{c} OR_5 \\ | \\ -CH-. \end{array}$$

in der $R_5$ eine Gruppe $R'_4$ darstellt, oder eine Acylgruppe der Formel

$$\begin{array}{c} O \\ \| \\ -C-R'_4 \end{array}$$

bedeutet, **dadurch gekennzeichnet**, daß man eine Verbindung der Formel I nach Anspruch 1, in der Y eine Gruppe

$$\begin{array}{c} OH \\ | \\ -CH- \end{array}$$

darstellt:

entweder mit einem Alkalimetallalkoholat und dann mit einem Halogenid der allgemeinen Formel:

$$R'_4\text{-Hal}$$

in der $R'_4$ die in Anspruch 1 angegebenen Bedeutungen besitzt und Hal ein Halogenatom darstellt,
oder mit einem Halogenid der allgemeinen Formel:

$$\begin{array}{c} O \\ \| \\ Hal-C-R'_4 \end{array}$$

in der Hal und $R'_4$ die oben angegebenen Bedeutungen besitzen, umsetzt, wobei die Reaktion in Gegenwart eines Säureakzeptors durchgeführt wird und die gewünschten Verbindungen in Form der freien Basen liefert, welche man gewünschtenfalls mit einer organischen oder anorganischen Säure umsetzt zur Bildung eines pharmazeutisch annehmbaren Salzes diese Derivate.

**21.** Verfahren zur Herstellung der Derivate nach Anspruch 1, in denen Y eine Gruppe $-CH_2-$ darstellt, **dadurch gekennzeichnet,** daß man eine Verbindung der Formel I nach Anspruch 1, in der Y eine Gruppe

$$\begin{array}{c} OH \\ | \\ -CH- \end{array}$$

darstellt, in einem geeigneten Lösungsmittel in Gegenwart von Trifluoressigsäure mit einem Alkalimetallborhydrid reduziert, so daß man die gewünschten Verbindungen in Form der freien Base erhält, welche man gewünschtenfalls mit einer organischen oder anorganischen Säure umsetzt zur Bildung eines pharmazeutisch annehmbaren Salzes dieser Derivate.

**22.** Verfahren zur Herstellung der Derivate nach Anspruch 1, in denen T eine Nitrogruppe und Y eine Gruppe

$$\begin{array}{c} O \\ \| \\ -C- \end{array}$$

bedeuten, **dadurch gekennzeichnet**, daß man ein Acylhalogenid der allgemeinen Formel:

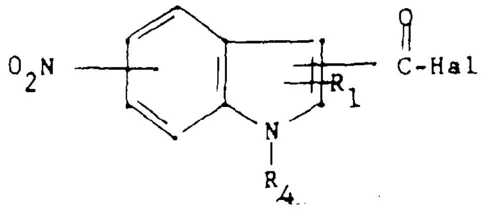

in der $R_1$ und $R_4$ die in Anspruch 1 angegebenen Bedeutungen besitzen und Hal ein Halogenatom darstellt, in Gegenwart einer Lewis-Säure mit einem Amin der allgemeinen Formel:

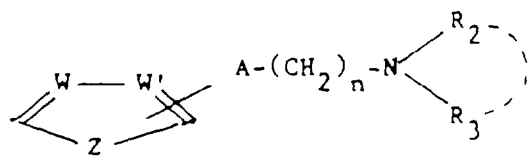

in der A, $R_2$, $R_3$, W, W', Z und n die in Anspruch 1 angegebenen Bedeutungen besitzen, kondensiert, so daß man die gewünschten Verbindungen in Form der freien Base erhält, welche man gewünschtenfalls mit einer organischen oder anorganischen Säure zur Bildung eines pharmazeutisch annehmbaren Salzes umsetzt.

**23.** Verfahren zur Herstellung der Derivate nach Anspruch 1, in denen T, T ' oder T" eine cyclisierte Gruppe

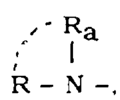

Y eine Gruppe

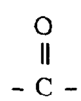

- und A-O- bedeuten, **dadurch gekennzeichnet**, daß man ein Keton der allgemeinen Formel:

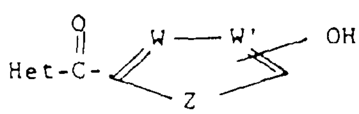

in der W, W' und Z die in Anspruch 1 angegebenen Bedeutungen besitzen und Het eine Gruppe der allgemeinen Formel bedeutet:

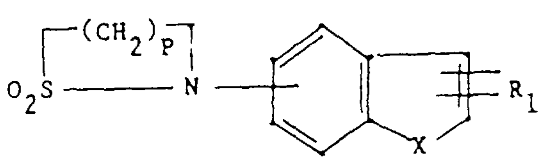

oder

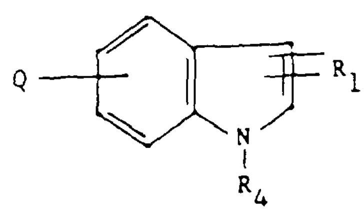

oder

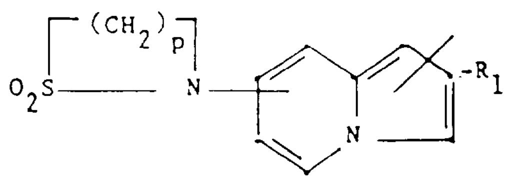

in denen Q, $R_1$, $R_4$, X und p die in Anspruch 1 angegebenen Bedeutungen besitzen, in einem geeigneten Lösungsmittel bei einer Temperatur von 90 bis 110°C mit einer Verbindung der allgemeinen Formel:

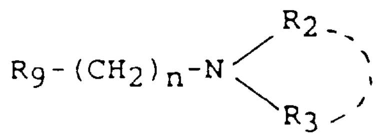

in der $R_2$, $R_3$ und n die oben angegebenen Bedeutungen besitzen und $R_9$ ein Halogenatom, eine $C_1$-$C_4$-Alkylsulfonyloxygruppe oder eine $C_6$-$C_{10}$-Arylsulfonyloxygruppe bedeutet, kondensiert, so daß man die gewünschten Verbindungen in Form der freien Base erhält, welche man gewünschtenfalls mit einer organischen oder anorganischen Säure umsetzt zur Bildung eines pharmazeutisch annehmbaren Salzes.

**24.** Verfahren zur Herstellung der Derivate nach Anspruch 1, in denen T' Wasserstoff, eine Nitrogruppe oder eine Gruppe

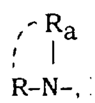

$R_4$ eine Gruppe -$SO_2$R' und Y eine Gruppe

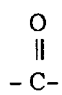

bedeuten, **dadurch gekennzeichnet**, daß man ein Indolderivat der allgemeinen Formel:

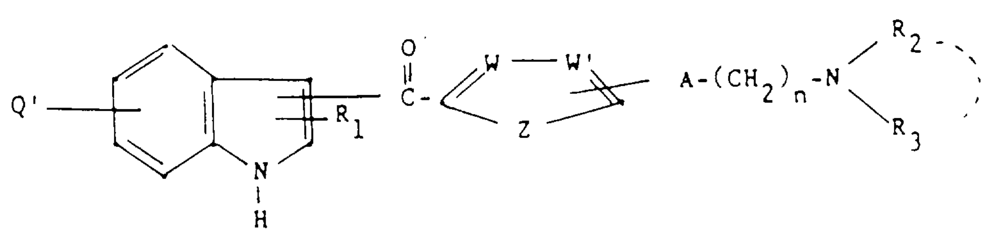

in der A, $R_1$, $R_2$, $R_3$, W, W', Z und n die in Anspruch 1 angegebenen Bedeutungen besitzen und Q' Wasserstoff, eine Nitrogruppe oder eine Gruppe

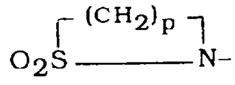

bedeutet, worin p eine Zahl mit einem Wert von 1 bis 4 darstellt, in einem geeigneten Lösungsmittel und in Gegenwart eines basischen Mittels mit einem Halogenid der allgemeinen Formel:

$$Hal\text{-}SO_2R'$$

oder einem Anhydrid der allgemeinen Formel:

$$(R'SO_2)_2O$$

in denen R die in Anspruch 1 angegebenen Bedeutungen besitzt und Hal ein Halogenatom darstellt, behandelt, so daß man die gewünschten Verbindungen in Form der freien Base erhält, welche man gewünschtenfalls mit einer organischen oder anorganischen Säure zu einem pharmazeutisch annehmbaren Salz umsetzt.

**25.** Verfahren zur Herstellung der Derivate nach Anspruch 1, in denen T' eine Nitrogruppe, Y eine Gruppe

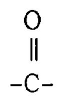

und A-O- bedeuten, $R_2$, $R_3$, $R_4$ und $R'_4$ identisch sind und m und n identisch sind, **dadurch gekennzeichnet,** daß man ein Ketonderivat der allgemeinen Formel:

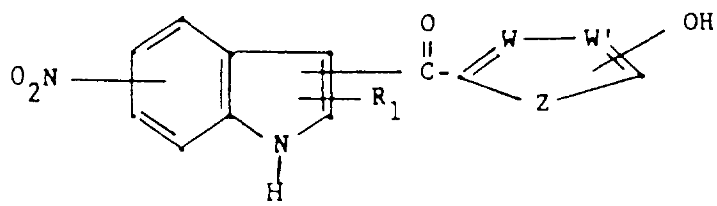

in der $R_1$, W, W' und Z die in Anspruch 1 angegebenen Bedeutungen besitzen, in Gegenwart eines basischen Mittels mit einer Verbindung der allgemeinen Formel:

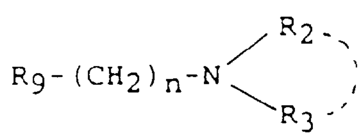

in der $R_2$, $R_3$ und n die in Anspruch 1 angegebenen Bedeutungen besitzen und $R_9$ ein Halogenatom, eine $C_1$-$C_4$-Alkylsulfonyloxygruppe oder eine $C_6$-$C_{10}$-Arylsulfonyloxygruppe darstellt, umsetzt, so daß man die gewünschten Verbindungen in Form der freien Base erhält, die man gewünschtenfalls mit einer organischen oder anorganischen Säure zur Bildung eines pharmazeutisch annehmbaren Salzes umsetzt.

**26.** Verfahren zur Herstellung der Derivate nach Anspruch 1, in denen T, T ' oder T" eine Gruppe $-NHSO_2R'$ bedeuten, **dadurch gekennzeichnet,** daß man eine Verbindung der allgemeinen Formel:

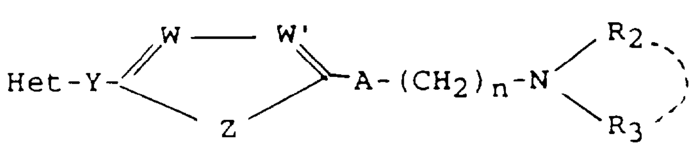

in der $R_2$, $R_3$, W, W', Y, Z, A und n die oben angegebenen Bedeutungen besitzen und Het eine Gruppe der allgemeinen Formel darstellt:

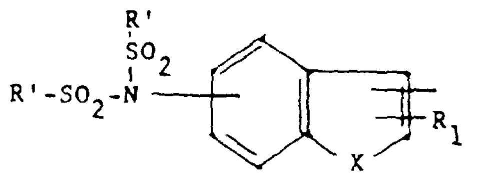

oder

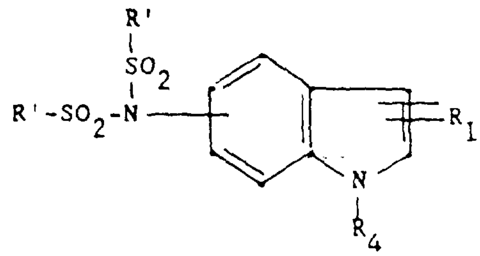

oder

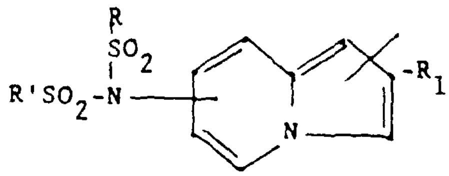

in denen R', $R_1$, $R_4$ und X die in Anspruch 1 angegebenen Bedeutungen besitzen, in einem geeigneten Lösungsmittel mit einem Alkalimetallhydroxid behandelt, so daß man die gewünschten Verbindungen erhält, welche man gewünschtenfalls mit einer organischen oder anorganischen Säure zur Bildung eines pharmazeutisch annehmbaren Salzes umsetzt.

**27.** Verfahren zur Herstellung der Derivate nach Anspruch 1, in denen T oder T' eine Gruppe

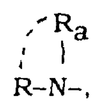

worin R und $R_a$ identisch sind und jeweils eine Gruppe $-SO_2R'$ darstellen, Y eine Gruppe

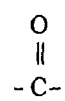

und A eine Gruppe -O- bedeuten, **dadurch gekennzeichnet,** daß man eine Verbindung der allgemeinen Formel:

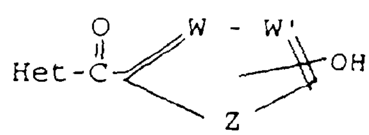

in der W, W' und Z die in Anspruch 1 angegebenen Bedeutungen besitzen und Het eine Gruppe der allgemeinen Formel darstellt:

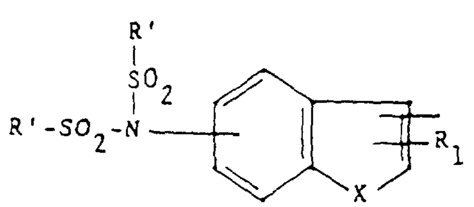

oder

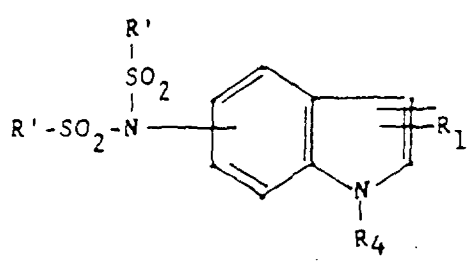

in denen R', $R_1$, $R_4$ und X die in Anspruch 1 angegebenen Bedeutungen besitzen, in Gegenwart eines basischen

Mittels bei der Rückflußtemperatur des Mediums mit einer Verbindung der allgemeinen Formel:

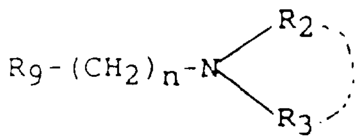

in der $R_2$, $R_3$ und n die in Anspruch 1 angegebenen Bedeutungen besitzen und $R_9$ ein Halogenatom, eine $C_1$-$C_4$-Alkylsulfonyloxygruppe oder eine $C_6$-$C_{10}$-Arylsulfonyloxygruppe darstellt, umsetzt, so daß man die gewünschten Verbindungen in Form der freien Base erhält, die man gewünschtenfalls mit einer organischen oder anorganischen Säure zur Bildung eines pharmazeutisch annehmbaren Salzes umsetzt.

**28.** Pharmazeutische oder veterinärmedizinische Zubereitungen, enthaltend als Wirkstoffmindestens ein Benzofuran-, Benzothiophen-, indol- oder IndolizinDerivat nach einem der Ansprüche 1 bis 9 in Kombination mit einem pharmazeutischen Trägermaterial oder einem geeigneten Bindemittel.

**29.** Pharmazeutische oder veterinärmedizinische Zubereitungen, enthaltend als Wirkstoffmindestens ein Derivat nach Anspruch 10 in Kombination mit einem pharmazeutischen Trägermaterial oder einem geeigneten Bindemittel.

**30.** Pharmazeutische oder veterinärmedizinische Zubereitungen nach einem der Ansprüche 28 oder 29 zur Behandlung von pathologischen Syndromen des kardiovaskulären Systems, enthaltend 50 bis 500 mg des Wirkstoffs.

**31.** Verwendung mindestens eines Benzofuran-, Benzothiophen-, Indol- oder Indolizin-Derivats nach einem der Ansprüche 1 bis 10 zur Herstellung eines Arzneimittels zur Behandlung von pathologischen Syndromen des kardiovaskulären Systems.

## Claims

**1.** Benzofuran, benzothiophene, indole or indolizine derivatives of general formula:

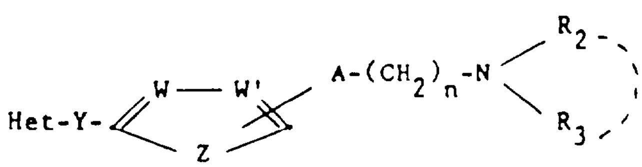

I

wherein
Het represents one of the groups

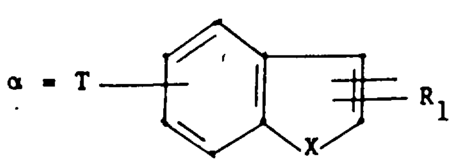

or

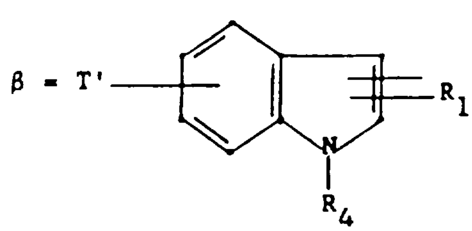

or

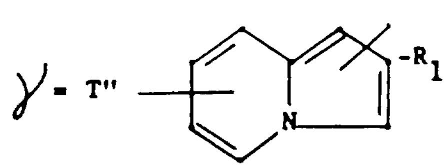

wherein

T represents a group:

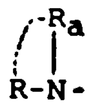

wherein
R and $R_a$, which may be identical or different, denote:

- hydrogen
- a $C_{1-4}$-alkyl radical
- an $-SO_2R'$ radical wherein R' represents a straight-chained or branched $C_{1-6}$-alkyl radical, a trifluoromethyl radical, a phenyl radical optionally substituted by a $C_{1-4}$-alkyl radical, a benzyl radical optionally substituted by a $C_{1-4}$-alkyl radical or a benzoyl radical optionally substituted by a $C_{1-4}$-alkyl radical, wherein $R_a$ and R' may form a cyclic group having 3 to 6 carbon atoms together with the nitrogen atom of the sulphonamide group,

T' represents:

- a nitro group
- a group

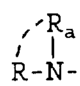

as hereinbefore defined,

T" denotes:

- a benzyloxycarbonylamino group
- a group

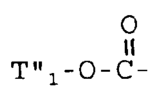

wherein $T"_1$ denotes hydrogen or a $C_{1-4}$-alkyl group
- a group:

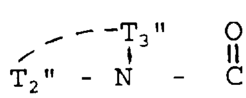

wherein $T"_2$ and $T"_3$, which may be identical or different, represent hydrogen or a $C_{1-4}$-alkyl group or $T"_2$ and $T"_3$ form, together with the nitrogen atom to which they attached, a cyclic group having 4 to 6 carbon atoms
- a group

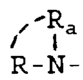

as hereinbefore defined

X denotes -O- or -S-
Y denotes a radical

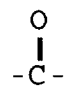, 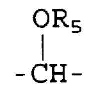

or $-CH_2-$ wherein $R_5$ denotes hydrogen, a $C_{1-4}$-alkyl radical or an acyl radical of formula

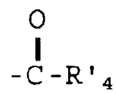

wherein $R'_4$ denotes a $C_{1-4}$-alkyl radical
$R_1$ denotes a $C_{1-6}$-alkyl radical, phenyl optionally substituted by a $C_{1-4}$-alkyl radical or halophenyl
$R_2$ represents:

- hydrogen
- a straight-chained or branched $C_{1-6}$-alkyl radical

$R_3$ represents:

- a straight-chained or branched $C_{1-6}$-alkyl radical
- a radical or formula: Alk-$R_6$
  wherein Alk denotes a single bond or a straight-chained or branched $C_{1-5}$-alkylene radical and $R_6$ denotes a pyridyl, phenyl, phenoxy, 3,4-methylenedioxyphenyl radical or a phenyl or phenoxy group substituted by one or more substituents which may be identical or different, selected from the halogens, the $C_{1-4}$-alkyl groups or $C_{1-4}$-alkoxy groups,

$R_2$ and $R_3$, when taken together, denote a $C_{3-6}$-alkylene or alkenylene radical optionally substituted by a phenyl radical or optionally interrupted by -O- -NH-, -N= or $\rangle$N-$R_7$, wherein $R_7$ denotes a $C_{1-4}$-alkyl or phenyl radical,
$R_4$ denotes:

- hydrogen
- a $C_{1-4}$-alkyl radical
- an $-SO_2$ $R'_1$ radical wheein $R'_1$ represents a $C_{1-4}$-alkyl radical, a phenyl radical optionally substituted by a $C_{1-4}$-alkyl radical or a benzyl radical optionally substituted by a $C_{1-4}$-alkyl radical,
- a radical

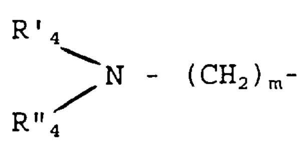

wherein $R'_4$ and $R''_4$, which may be identical or different, represent a $C_{1-4}$-alkyl radical and m denotes a number from 1 to 3

A represents -O-, -S- or

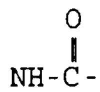

W, W' and Z are such that:

- when W and W' are identical and represent

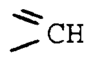

  or N, Z represents -O
- when W represents

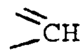

  and W' represents

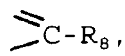

  Z represents -CH=C'-R'$_8$
  in which $R_8$ and $R'_8$ are identical or different and represent hydrogen, a halogen atom or a $C_{1-4}$-alkyl or $C_{1-4}$-alkoxy radical

n denotes a number from 1 to 5, with the proviso that if $R_4$ represents an $-SO_2R'_1$ radical, T' represents a nitro group or a cyclised

$R_a$
R-N- group, and the pharmaceutically acceptable salts thereof.

**2.** Derivatives according to claim 1, wherein R denotes an $-SO_2R'$ group and $R_a$ represents hydrogen.

**3.** Derivatives according to claim 1 or 2 wherein y represents a radical

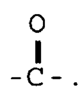

**4.** Derivatives according to one of claims 1 to 3, wherein the entity

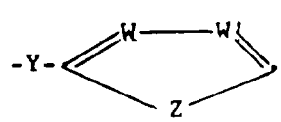

represents a benzoyl radical.

**5.** Derivatives according to one of claims 1 to 4 wherein the entity

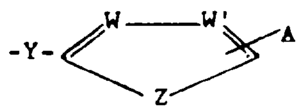

represents a benzoyl-4-oxy radical.

**6.** Derivatives according to one of claims 1 to 5, wherein $R_1$, $R_2$ and $R_3$ denote an n-butyl radical and n represents 3.

**7.** Derivatives according to one of claims 1 to 6, wherein X represents -O-.

**8.** Derivatives according to one of claims 1 to 7, wherein the chain

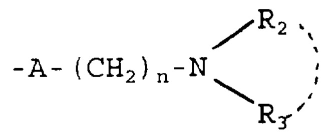

is in the 4 position.

**9.** Derivatives according to one of claims 1 to 8, wherein the pharmaceutically acceptable salt is the oxalate, fumarate, hydrochloride or p-toluenesulphonate.

**10.** Derivatives according to claim 1 selected from:

n-butyl-2[(di-n-butylamino-3-propoxy)-4-benzoyl]-3-methylsulphonamide-5-benzofuran,

n-butyl-2-{[N-methyl-N-(dimethoxy-3,4β-phenethyl)amino-3-propoxy]-4-benzoyl}-3-methylsulphonamido-5-benzofuran,

isopropyl-2[(di-n-butylamino-3-propoxy)-4-benzoyl]-3-methylsulphonamido-5-benzofuran,

amino-5-[(di-n-butylamino-3-propoxy)-4-benzoyl]-3-n-butyl-2-methyl-1-indole,

and the pharmaceutically acceptable salts thereof.

**11.** Process for preparing derivatives according to claim 1, wherein Y represents a

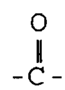

group and T or T' reprresents an amino group, characterised in that a nitro derivative of general formula:

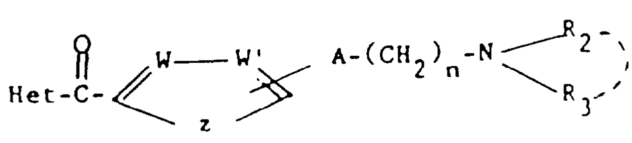

wherein Het represents a group:

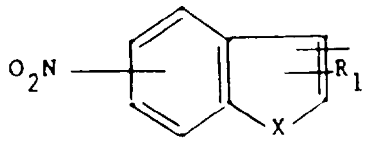 or 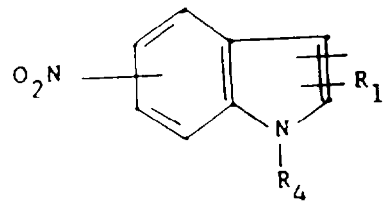

wherein $R_1$, $R_2$, $R_3$, $R_4$, A, W, W', X, Y, Z and n are defined as in claim 1, is hydrogenated, the reaction being carried out in the presence of a suitable catalyst and in a polar solvent, to yield, in the form of the free base, the desired compounds which are reacted, if desired, with an organic or inorganic acid to form a pharmaceutically acceptable salt of these derivatives.

**12.** Process for preparing derivatives according to claim 1 wherein Y represents a group

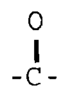

and T, T' or T" denotes an

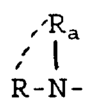

group wherein R represents an $-SO_2R'$ group and $R_a$ represents hydrogen or an $-SO_2R'$ group, characterised in that a compound of formula I according to claim 1 wherein R and $R_a$ each denote hydrogen is reacted with one or two equivalents of a halide of general formula:

$$Hal\text{-}SO_2\text{-}R'$$

or an anhydride of general formula:

$$(R'SO_2)_2O$$

wherein R' is defined as in claim 1 and Hal denotes a halogen atom, the reaction being carried out in the presence of an acid acceptor and in a suitable organic solvent, to yield, in the form of the free base, the desired compounds which are reacted, if desired, with an organic or inorganic acid to form a pharmaceutically acceptable salt of these derivatives.

**13.** Process for preparing derivatives according to claim 1 wherein Y represents a group

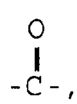

T, T' or T" represents a group

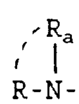

wherein R has the value indicated and $R_a$ denotes an alkyl radical, characterised in that a compound of formula I according to claim 1 wherein R has the meaning given and $R_a$ denotes hydrogen, is reacted with one or two equivalents of a halide of general formula:

$$R'_a\text{-Hal}$$

wherein Hal represents a halogen atom and $R'_a$ represents a $C_{1-4}$-alkyl radical, to yield, in the form of a free base, the desired compounds which are reacted, if desired, with an organic or inorganic acid, in order to form a pharmaceutically acceptable salt of these derivatives.

**14.** Process for preparing derivatives according to claim 1 wherein Y represents a group

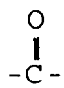

and either T' represents a nitro group or T, T' or T" represent a cyclised group

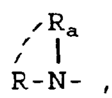

characterised in that a compound of general formula:

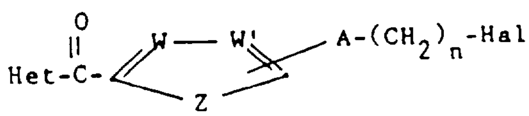

wherein A, W, W', Z, Hal and n are as defined in claim 1 and Het represents a group of general formula:

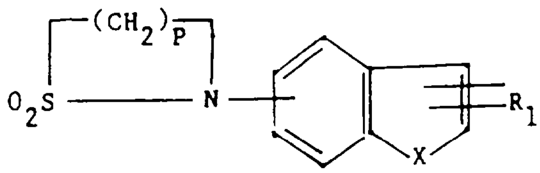

or

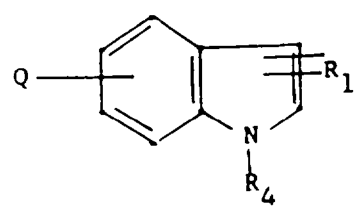

or

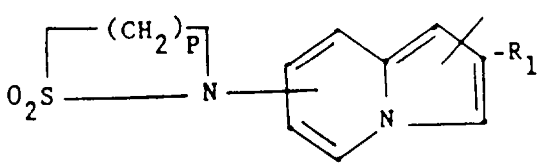

wherein $R_1$, $R_4$ and X are defined as in claim 1, Q represents a nitro group or

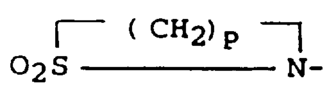

and P represents a number from 1 to 4, is reacted in a polar or non-polar solvent with a nitro compound of general formula:

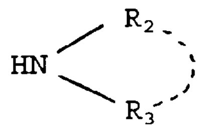

wherein $R_2$ and $R_3$ have the same meaning as in claim 1, the reaction being carried out in the presence of a basic agent in order to yield, in the form of the free base, the desired compounds which are reacted, if desired, with an organic or inorganic base to form a pharmaceutically acceptable salt of these derivatives.

**15.** Process for preparing derivatives according to claim 1, wherein T" represents a carbalkoxy group and Y represents a group

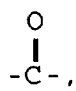

characterised in that at a temperature of 80 to 110°C, an indolizine derivative of general formula:

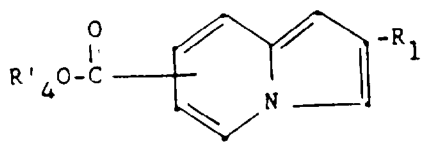

wherein $R_1$ and $R'_4$ are defined as in claim 1, is treated with a halide of general formula

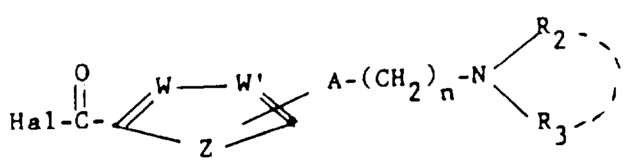

wherein A, $R_2$, $R_3$, W, W', Z and n are as hereinbefore defined and Hal represents a halogen atom, to yield the desired compounds in the form of the free base, which may be reacted if desired with an organic or inorganic acid to form a pharmaceutically acceptable salt of these derivatives.

**16.** Process for preparing derivatives according to claim 1 wherein T" represents a carboxy group and Y represents a group

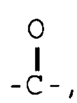

characterised in that a carbalkoxy indolizine derivative of formula I according to claim 1, wherein Het represents a group of general formula:

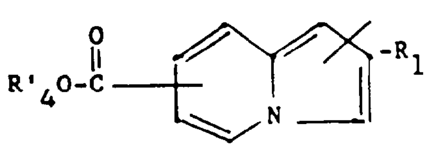

wherein $R_1$ and $R'_4$ have the same meaning as in claim 1, is saponified, using an alkali metal hydroxide in a suitable solvent and at the reflux temperature of the medium, in order to obtain, in the form of a free base, the desired compounds which are reacted, if desired, with an organic or inorganic acid to form a pharmaceutically acceptable salt of these derivatives.

**17.** Process for preparing derivatives according to claim 1, wherein T" represents a benzyloxycarbamoyl group and Y represents a group

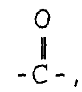

characterised in that a carboxyindolizine derivative of formula I according to claim 1, wherein Het represents a group of general formula:

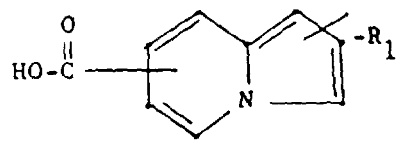

wherein $R_1$ is defined as in claim 1, is reacted in a suitable solvent and at a temperature from 0 to +10°C with ethyl chloroformate in the presence of an acid acceptor, then with an alkali metal azide and finally with benzyl alcohol, to obtain, in the form of a free base, the desired compounds which are reacted, if desired, with an organic or inorganic acid to form a pharmaceutically acceptable salt of these derivatives.

**18.** Process for preparing derivatives according to claim 1 wherein T" represents an amino group and Y represents a group

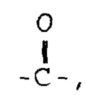

characterised in that a benzyloxycarbamoyl indolizine derivative of formula I according to claim 1, wherein Het represents a group of the formula

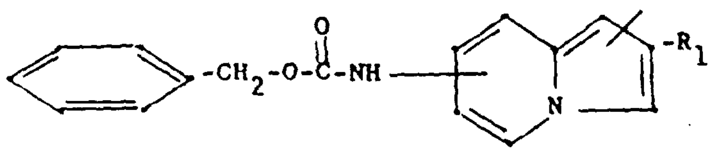

wherein $R_1$ is defined as in claim 1, is hydrogenated in a suitable solvent and in the presence of a suitable catalyst, to obtain, in the form of a free base, the desired compounds which are reacted, if desired, with an organic or inorganic acid to form a pharmaceutically acceptable salt of these derivatives.

**19.** Process for preparing derivatives according to claim 1, wherein Y represents a group

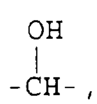

characterised in that a compound of formula I according to claim 1 wherein Y represents a group

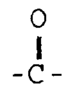

is reduced by means of an alkyl metal borohydride and in a suitable solvent, to obtain the desired compounds in the form of free bases, which are reacted if desired with an organic or inorganic acid to form a pharmaceutically acceptable salt of these derivatives.

**20.** Process for preparing derivatives according to claim 1, wherein Y represents a group

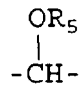

wherein $R_5$ represents a radical $R'_4$ or an acyl radical of the formula

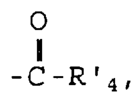

characterised in that a compound of formula I according to claim 1 wherein Y represents a group

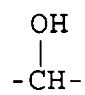

is reacted:

<u>either</u> with an alkali metal alkoxide, and then with a halide of general formula:

$$R'_4\text{-Hal}$$

wherein $R'_4$ has the same meaning as in claim 1 and Hal denotes a halogen atom,
<u>or</u> with a halide of general formula:

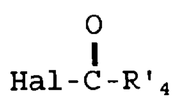

wherein Hal and $R'_4$ have the same meaning as hereinbefore, the reaction being carried out in the presence of an acid acceptor, to yield the desired compounds in the form of free bases, which may be reacted if desired with an organic or inorganic acid to form a pharmaceutically acceptable salt of these derivatives.

**21.** Process for preparing derivatives according to claim 1, wherein Y represents a group $-CH_2-$,
characterised in that a compound of formula I according to claim 1 wherein Y represents a group

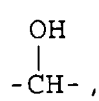

is reduced by means of an alkali metal borohydride, in the presence of trifluoroacetic acid and in a suitable solvent,

to obtain the desired compounds, in the form of free bases, which are reacted if desired with an organic or inorganic acid, to form a pharmaceutically acceptable salt of these derivatives.

**22.** Process for preparing derivatives according to claim 1, wherein T represents a nitro group and Y represents a group

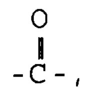

characterised in that a acyl halide of general formula

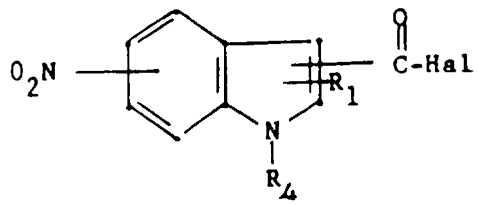

wherein $R_1$ and $R_4$ have the same meaning as in claim 1 and Hal represents a halogen atom, is condensed in the presence of a Lewis acid with an amine of general formula

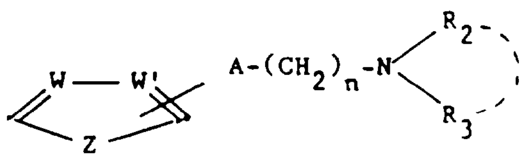

wherein A, $R_2$, $R_3$, W, W', Z and n are defined as in claim 1, to yield the desired compounds in the form of free bases which are reacted, if desired, with an organic or inorganic acid to form a pharmaceutically acceptable salt.

**23.** Process for preparing derivatives according to claim 1, wherein T, T' or T" represents a cyclised group

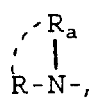

Y represents a group

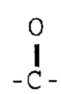

and A represents -O-, characterised in that a ketone of general formula

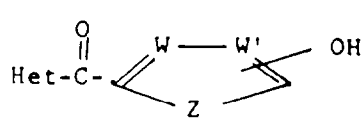

wherein W, W' and Z have the same meaning as in claim 1 and Het represents a group of general formula

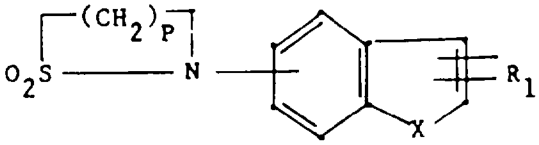

or

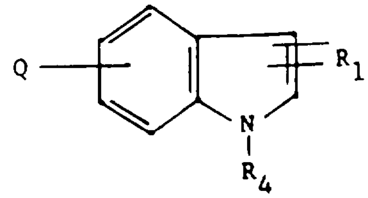

or

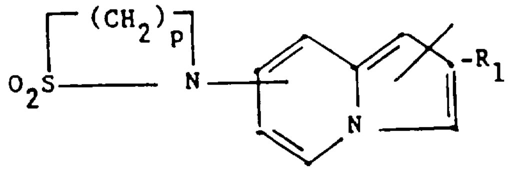

wherein Q, $R_1$, $R_4$, X and p are as defined in claim 1, is condensed at a temperature of 90 to 110°C and in a suitable solvent with a compound of general formula:

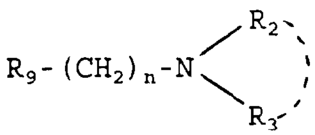

wherein $R_2$, $R_3$ and n are as hereinbefore defined and $R_9$ represents a halogen atom, a $C_{1-4}$-alkylsulphonyloxy radical or $C_{6-10}$-arylsulphonyloxy radical, to obtain the desired compounds, in the form of the free bases, which may be reacted if desired with an organic or inorganic acid to form a pharmaceutically acceptable salt.

**24.** Process for preparing derivatives according to claim 1 wherein T' represents hydrogen, a nitro group or a group

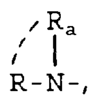

$R_4$ represents a group $-SO_2R'$ and Y represents a group

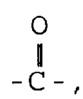

characterised in that an indole derivative of general formula

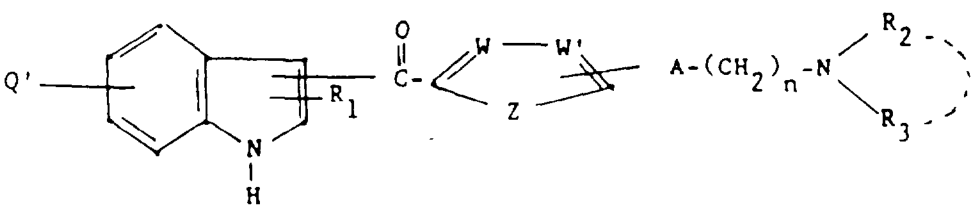

wherein A, $R_1$, $R_2$, $R_3$, W, W', Z and n have the same meaning as in claim 1 and Q' represents hydrogen, a nitro group or a group

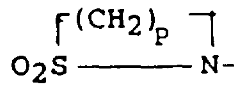

wherein p denotes a number from 1 to 4, is treated, in a suitable solvent and in the presence of a basic agent, with a halide of general formula:

$$Hal\text{-}SO_2R'$$

or an anhydride of general formula:

$$(R'SO_2)_2O$$

wherein R has the same meaning as in claim 1 and Hal denotes a halogen atom, to obtain the desired compounds in the form of free bases which are reacted, if desired, with an organic or inorganic acid to form a pharmaceutically acceptable salt.

**25.** Process for preparing derivatives according to claim 1 wherein T' represents a nitro group, Y represents a

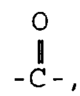

A denotes -O- , $R_2$ , $R_3$, $R_4$ and $R'_4$ are identical and m and n are identical, characterised in that a ketone derivative of general formula:

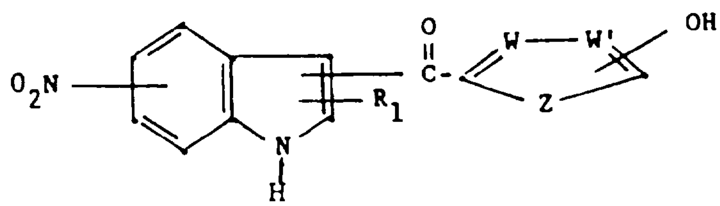

wherein $R_1$, W, W' and Z are defined as in claim 1, is reacted in the presence of a basic agent with a compound of general formula:

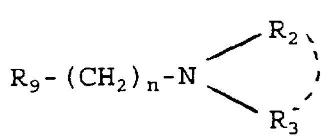

wherein $R_2$, $R_3$ and n have the same meaning as in claim 1 and $R_9$ represents a halogen atom, a $C_{1-4}$-alkylsulphonyloxy radical or $C_{6-10}$-arylsulphonyloxy radical, to obtain the desired compounds in the form of free bases which are reacted, if desired, with an organic or inorganic acid to form a pharmaceutically acceptable salt.

**26.** Process for preparing derivatives according to claim 1, wherein T, T' or T" represents a group $-NHSO_2R'$, characterised in that a compound of general formula:

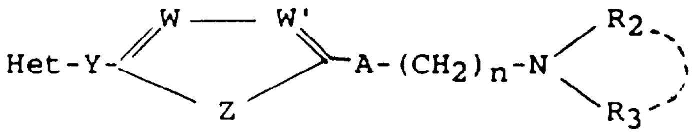

wherein $R_2$, $R_3$, W, W', Y, Z, A and n are as hereinbefore defined and Het represents a group of general formula

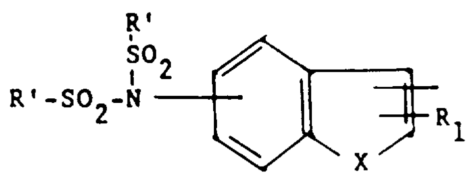

or

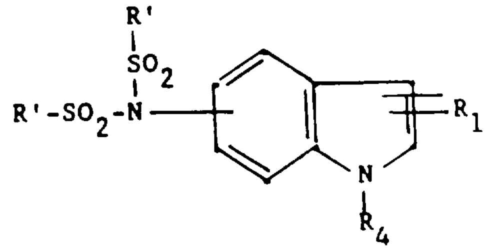

or

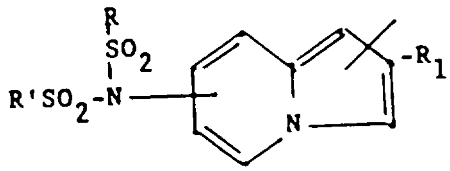

wherein R', $R_1$, $R_4$ and X are defined as in claim 1, is treated in an appropriate solvent with an alkali metal hydroxide, to obtain the desired compounds which are reacted, if desired, with an organic or inorganic acid to form a pharmaceutically acceptable salt.

**27.** Process for preparing derivatives according to claim 1, wherein T or T' represents a group

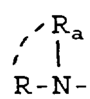

wherein R and $R_a$ are identical and each represent a group $-SO_2R'$, Y represents a group

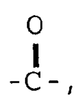

and A represents a group -O-, characterised in that a compound of general formula:

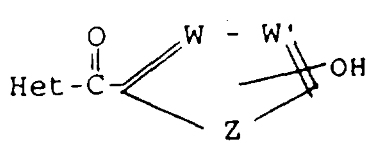

wherein W, W' and Z are defined as in claim 1 and Het represents a group of general formula

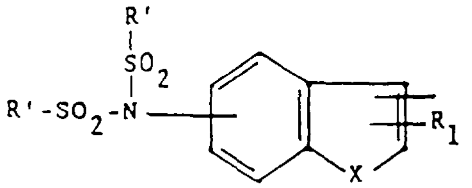 or 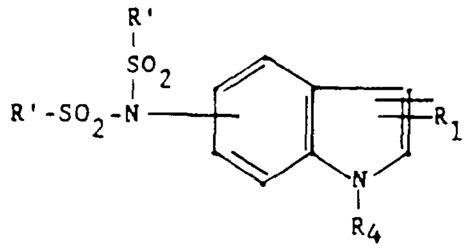

wherein R', $R_1$, $R_4$ and X are defined as in claim 1, is reacted in the presence of a basic agent and at the reflux temperature of the medium with a compound of general formula

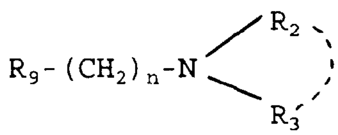

wherein $R_2$, $R_3$ and n have the same meaning as in claim 1 and $R_9$ represents a halogen atom, a $C_{1-4}$-alkylsulphonyloxy radical or $C_{6-10}$-arylsulphonyloxy radical, to obtain the desired compounds in the form of free bases which are reacted, if desired, with an organic or inorganic acid to form a pharmaceutically acceptable salt.

**28.** Pharmaceutical or veterinary compositions containing as active principle at least one benzofuran, benzothiophene, indole or indolizine derivative according to one of claims 1 to 9, in conjunction with a pharmaceutical carrier or a suitable excipient.

**29.** Pharmaceutical or veterinary compositions containing as active principle at least one derivative according to claim 10, in conjunction with a pharmaceutical carrier or a suitable excipient.

**30.** Pharmaceutical or veterinary compositions according to one of claims 28 or 29 for treating pathological syndromes of the cardiovascular system, containing 50 to 500 mg of active principle.

**31.** Use of at least one benzofuran, benzothiophene, indole or indolizine derivative according to one of claims 1 to 10 for the production of a pharmaceutical composition intended for the treatment of pathological syndromes of the cardiovascular system.